# EUROPEAN PATENT SPECIFICATION

(11) **EP 1 611 104 B1**
(45) Date of publication and mention of the grant of the patent: **01.07.2009**
(21) Application number: 04722563.6
(22) Date of filing: 23.03.2004
(51) Int. Cl.: C07D 217/02, C07D 217/18, C07D 217/20, C07D 405/06, C07D 217/04, C07D 217/16, A61P 3/04, A61P 25/20, A61K 31/472

(54) **TETRAHYDROISOQUINOLYL ACETAMIDE DERIVATIVES FOR USE AS OREXIN RECEPTOR ANTAGONISTS**
TETRAHYDROISOCHINOLYLACETAMIDDERIVATE ZUR VERWENDUNG ALS OREXINREZEPTORANTAGONISTEN
DERIVES DE TETRAHYDRO-ISOQUINOLYL-ACETAMIDE DESTINES A SERVIR D'ANTAGONISTES DES RECEPTEURS D'OREXINE

(30) Priority: 26.03.2003 WO PCT/EP03/03143
(43) Date of publication of application: 04.01.2006
(73) Proprietor: Actelion Pharmaceuticals Ltd., 4123 Allschwil (CH)
(72) Inventor: AISSAOUI, Hamed, F-68840 Pulversheim (FR); CLOZEL, Martine, CH-4102 Binningen (CH); WELLER, Thomas, CH-4102 Binningen (CH); KOBERSTEIN, Ralf, 79539 Lörrach (DE); SIFFERLEN, Thierry, F-68116 Guewenheim (FR); FISCHLI, Walter, CH-4123 Allschwil (CH)
(74) Representative: Schager, Frank
(86) International application number: PCT/EP2004/003057
(87) International publication number: WO 2004/085403

(56) References cited:
- WO-A-01/68609

## Description

The present invention relates to novel acetamide derivatives of formula (I) and their use as pharmaceuticals. The invention also concerns related aspects including processes for the preparation of the compounds, pharmaceutical compositions containing one or more compounds of formula (I), and especially their use as orexin receptor antagonists.
The orexins (hypocretins) comprise two neuropeptides produced in the hypothalamus: the orexin A (OX-A) (a 33 aminoacid peptide) and the orexin B (OX-B) (a 28 aminoacid peptide) (Sakurai T. et al., Cell, 1998, 92, 573-585). Orexins are found to stimulate food consumption in rats suggesting a physiological role for these peptides as mediators in the central feedback mechanism that regulates feeding behaviour (Sakurai T. et al., Cell, 1998, 92, 573-585). On the other hand, it was also proposed that orexins regulate states of sleep and wakefulness opening potentially novel therapeutic approaches for narcoleptic patients (Chemelli R.M. et al., Cell, 1999, 98, 437-451). Two orexin receptors have been cloned and characterised in mammals which belong to the G-protein coupled receptor superfamily (Sakurai T. et al., Cell, 1998, 92, 573-585), the orexin-1 receptor (OX₁) which is selective for OX-A and the orexin-2 receptor (OX₂) which is capable to bind OX-A as well as OX-B.

Orexin receptors are found in the mammalian brain and may have numerous implications in pathologies such as depression; anxiety; addictions, obsessive compulsive disorder; affective neurosis; depressive neurosis; anxiety neurosis; dysthymic disorder; mood disorder; sexual dysfunction; psychosexual dysfunction; sex disorder; schizophrenia; manic depression; delirium; dementia; severe mental retardation and dyskinesias such as Huntington's disease and Tourette syndrome; eating disorders; sleep disorders; cardiovascular diseases, diabetes; appetite/taste disorders; vomiting/nausea; asthma; Parkinson's disease; Cushing's syndrome/disease; basophil adenoma; prolactinoma; hyperprolactinemia; hypopituitarism; hypophysis tumour/adenoma; hypothalamic diseases; inflammatory bowel disease; gastric dyskinesia; gastric ulcers; Froehlich's syndrome; hypophysis diseases, hypothalamic hypogonadism; Kallman's syndrome (anosmia, hyposmia); functional or psychogenic amenorrhea; hypothalamic hypothyroidism; hypothalamic-adrenal dysfunction; idiopathic hyperprolactinemia; hypothalamic disorders of growth hormone deficiency; idiopathic growth deficiency; dwarfism; gigantism; acromegaly; disturbed biological and circadian rhythms; sleep disturbances associated with diseases such as neurological disorders, neuropathic pain and restless leg syndrome; heart and lung diseases, acute and congestive heart failure; hypotension; hypertension; urinary retention; osteoporosis; angina pectoris; myocardial infarction; ischemic or haemorrhagic stroke; subarachnoid haemorrhage; ulcers; allergies; benign prostatic hypertrophy; chronic renal failure; renal disease; impaired glucose tolerance; migraine; hyperalgesia; pain; enhanced or exaggerated sensitivity to pain such as hyperalgesia, causalgia, and allodynia; acute pain; burn pain; atypical facial pain; neuropathic pain; back pain; complex regional pain syndrome I and II; arthritic pain; sports injury pain; pain related to infection e.g. HIV, post-chemotherapy pain; post-stroke pain; post-operative pain; neuralgia; conditions associated with visceral pain such as irritable bowel syndrome, migraine and angina; urinary bladder incontinence e.g. urge incontinence; tolerance to narcotics or withdrawal from narcotics; sleep apnea; narcolepsy; insomnia; parasomnia; and neurodegenerative disorders including nosological entities such as disinhibition-dementia-parkinsonism-amyotrophy complex; pallido-ponto-nigral degeneration epilepsy; seizure disorders and other diseases related to general orexin system dysfunction.

Up to now some low molecular weight compounds are known which have a potential to antagonise either specifically OX₁ or OX₂, or both receptors at the same time. In WO 99/09024, WO 99/58533, WO 00/47576, WO 00/47577 and WO 00/47580 formerly SmithKline Beecham reported phenylurea, phenylthiourea and cinnamide derivatives as

OX₁ selective antagonists. More recently WO 01/85693 from Banyu Pharmaceuticals has been published wherein N-acyltetrahydroisoquinoline derivatives are disclosed. 2-Aminomethylpiperidine derivatives (WO 01/96302), 3-aminomethyl-morpholine derivatives (WO 02/44172) and N-aroyl cyclic amines (WO 02/89800, WO 02/90355, WO 03/51368 and WO 03/51871) have been suggested by formerly SmithKline Beecham as orexin receptor antagonists. Related compounds are disclosed in WO 03/02559, WO 03/02561, WO 03/32991, WO 03/41711, WO 03/51872 and WO 03/51873. In WO 03/37847 formerly SmithKline Beecham reported benzamide derivatives as orexin receptor antagonists. International patent applications WO 01/68609 and WO 02/51838 disclose 1,2,3,4-tetrahydroisoquinoline and novel benzazepine derivatives as orexin receptor antagonists. The novel compounds of the present invention belong to a different class of low molecular weight compounds as compared to all prior art orexin receptor antagonists so far published.

The present invention comprises acetamide derivatives, which are non-peptide antagonists of the human orexin receptors. These compounds, therefore, are of potential use in the treatment of disturbed homeostasis and eating disorders (e.g. bulimia, obesity, food abuse, compulsive eating or irritable bowel syndrome), as well as disturbed sleep/wake schedule, sleep disorders (e.g. insomnias, apneas, dystonias) or stress-related diseases (e.g. anxiety, mood and blood pressure disorders) or any other disease related to the orexin dysfunction.

The present invention relates to novel acetamide derivatives of the formula (I). 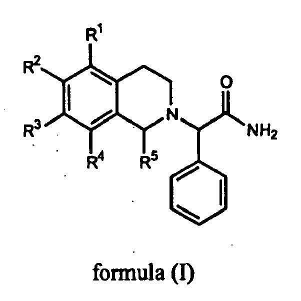 wherein
R¹, R², R³, R⁴ independently represent hydrogen; cyano; halogen; hydroxyl; C1-C4 alkyl; C2-C4 alkenyl; C1-C4 alkoxy; vinyloxy; allyloxy; trifluoromethoxy; C3-C6 cycloalkyloxy; or R¹ and R² together as well as R² and R³ together or R³ and R⁴ together may form with the phenyl ring, to which they are attached, a five, six or seven-membered ring containing one or two oxygen atoms; and
R⁵ represents a mono-, di- or trisubstituted phenyl-ethyl group, substituted independently with C1-C4 alkyl, C1-C4 alkoxy, C2-C4 alkenyl, trifluoromethyl, triflourmethoxy, difluoromethoxy or halogen;
and pharmaceutically acceptable salts thereof,
and pure enantiomers, mixtures of enantiomers, pure diastereoisomers, mixtures of diastereoisomers, diastereoisomeric racemates, mixtures of diastereoisomeric racemates and the meso-form and pharmaceutically acceptable salts, solvent complexes, and morphological forms, thereof.

In the present description the term "lower alkyl", alone or in combination, means a straight-chain or branched-chain alkyl group with 1 to 8 carbon atoms, preferably a straight or branched-chain alkyl group with 1-4 carbon atoms. Examples of straight-chain and branched C₁-C₈ alkyl groups are methyl, ethyl, propyl, isopropyl, butyl, pentyl, hexyl, heptyl, octyl, isobutyl, sec.-butyl, tert.-butyl, the isomeric pentyls, the isomeric hexyls, the isomeric heptyls and the isomeric octyls, preferably methyl, ethyl, propyl, isopropyl, butyl, sec.-butyl, tert.-butyl, isobutyl and pentyl.

The term "lower alkenyl", alone or in combination, means a straight-chain or branched-chain alkenyl group with 2 to 5 carbon atoms, preferably 2to 4 carbon atoms, preferably allyl and vinyl.

The term "lower alkoxy", alone or in combination, means a group of the formula alkyl-O- in which the term "alkyl" has the previously given significance, such as methoxy, ethoxy, n-propoxy, isopropoxy, n-butoxy, isobutoxy, sec.-butoxy and tert.-butoxy, preferably methoxy and ethoxy.

Lower alkenyloxy groups are preferably vinyloxy and allyloxy.

The term "cycloalkyl", alone or in combination, means a cycloalkyl ring with 3 to 8 carbon atoms and preferably a cycloalkyl ring with 3 to 6 carbon atoms. Examples of C₃-C₈ cycloalkyl are cyclopropyl, cyclobutyl, cyclopentyl, cyclohexyl, cycloheptyl and cyclooctyl, preferably cyclopropyl, cyclohexyl and particularly cyclohexyl or lower alkyl substituted cycloalkyl which may preferably be substituted with lower alkyl such as methylcyclopropyl, dimethyl-cyclopropyl, methyl-cyclobutyl, methyl-cyclopentyl, methylcyclohexyl, dimethyl-cyclohexyl.

The term "aryl", alone or in combination, means a phenyl or naphthyl group which optionally carries one or more substituents, preferably one or two substituents, each independently selected from cyano, halogen, hydroxy, lower alkyl, lower alkenyl, lower alkoxy, lower alkenyloxy, nitro, trifluoromethyl, difluoromethoxy, trifluoromethoxy, NH₂CO-, CH₃NHCO-, (CH₃)₂NCO-, H₂N-, CH₃NH-, (CH₃)₂N-, CH₃SO₂NH-, CH₃NHSO₂- or CH₃CO-NH-. Preferred are naphthyl, phenyl and phenyl independently substituted with lower alkyl, lower alkoxy, trifluoromethyl, difluoromethoxy, trifluoromethoxy, or halogen.

The term "aralkyl", alone or in combination, means a lower alkyl or cycloalkyl group as previously defined in which one hydrogen atom has been replaced by an aryl group as previously defined. Preferred are benzyl or 2-phenyl-ethyl groups that might be unsubstituted or independently substituted at the aryl group with lower alkyl, lower alkoxy, trifluoromethyl, difluoromethoxy, trifluoromethoxy or halogen.

The term "aralkenyl", alone or in combination, means a lower alkenyl group as previously defined in which one hydrogen atom has been replaced by an aryl group as previously defined. Preferred are 2-phenyl-ethenyl groups. Particularly preferred are 2-phenyl-ethenyl groups substituted at the phenyl group with one or two fluorine atoms.

For the term "heterocyclyl" and "heterocyclyl-lower alkyl", the heterocyclyl group is preferably a 5- to 10-membered monocyclic or bicyclic ring, which may be saturated, partially unsaturated or aromatic containing for example 1, 2 or 3 heteroatoms selected from oxygen, nitrogen and sulphur which may be the same or different. Examples of such heterocyclyl groups are pyrrolidinyl, piperidinyl, piperazinyl, morpholinyl, pyridyl, pyrimidinyl, pyrazinyl, pyridazinyl, quinolyl, isoquinolyl, thienyl, thiazolyl, isothiazolyl, furyl, imidazoyl, pyrazolyl, pyrrolyl, indazolyl, indolyl, isoindolyl, isoxazolyl, oxazolyl, quinoxalinyl, phthalazinyl, cinnolinyl, dihydropyrrolyl, pyrrolidinyl, isobenzofuranyl, tetrahydrofuranyl, dihydropyranyl. The heterocyclyl group, if aromatic, may have up to 3 optional substituents. Saturated heterocyclyl groups may have one substituent. Examples of suitable substituents include halogen, lower alkyl, amino, nitro, cyano, hydroxy, lower alkoxy, carboxy and lower alkyloxy-carbonyls.

The term "halogen" means fluorine, chlorine, bromine or iodine and preferably fluorine and chlorine.

The term "carboxy", alone or in combination, signifies a -COOH group.
Examples of preferred compounds of formula (I) are:
2-{1-[2-(3,4-Difluoro-phenyl)-ethyl]-6,7-dimethoxy-3,4-dihydro-1H-isoquinolin-2-yl}-2-phenyl-acetamide;
2-{1-[2-(2,3-Difluoro-phenyl)-ethyl]-6,7-dimethoxy-3,4-dihydro-1H-isoquinolin-2-yl}-2-phenyl-acetamide;
2-{6,7-Dimethoxy-1-[2-(4-trifluoromethyl-phenyl)-ethyl]-3,4-dihydro-1H-isoquinolin-2-yl}-2-phenyl-acetamide;
2-{6,7-Dimethoxy-1-[2-(3-methoxy-phenyl)-ethyl]-3,4-dihydro-1H-isoquinolin-2-yl}-2-phenyl-acetamide;
2-{1-[2-(2,5-Difluoro-phenyl)-ethyl]-6,7-dimethoxy-3,4-dihydro-1H-isoquinolin-2-yl}-2-phenyl-acetamide;
2-{6,7-Dimethoxy-1-[2-(2-methoxy-phenyl)-ethyl]-3,4-dihydro-1H-isoquinolin-2-yl}-2-phenyl-acetamide;
2-{1-[2-(4-Fluoro-phenyl)-ethyl]-6,7-dimethoxy-3,4-dihydro-1H-isoquinolin-2-yl}-2-phenyl-acetamide;
2-{1-[2-(3,4-Dimethoxy-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2-methyl-5-trifluoromethyl-phenyl)-ethyl]-3,4-dihydro-1H-isoquinolin-2-yl}-2-phenyl-acetamide;
(R)-2-{(S)-1-[2-(3-Chloro-2-fluoro-phenyl)-ethyl]-6,7-dimethoxy-3,4-dihydro-1H-isoquinolin-2-yl}-2-phenyl-acetamide;
(R)-2-{(S)-1-[2-(3-Fluoro-4-methyl-phenyl)-ethyl]-6,7-dimethoxy-3,4-dihydro-1H-isoquinolin-2-yl}-2-phenyl-acetamide;
(R)-2-{(S)-1-[2-(4-Fluoro-2-trifluoromethyl-phenyl)-ethyl]-6,7-dimethoxy-3,4-dihydro-1H-isoquinolin-2-yl}-2-phenyl-acetamide;
(R)-2-{(S)-1-[2-(5-Fluoro-2-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Fluoro-5-trifluoromethyl-phenyl)-ethyl]-6,7-dimethoxy-3,4-dihydro-1H-isoquinolin-2-yl}-2-phenyl-acetamide;
(R)-2-{(S)-1-[2-(3-Fluoro-4-trifluoromethyl-phenyl)-ethyl]-6,7-dimethoxy-3,4-dihydro-1H-isoquinolin-2-yl}-2-phenyl-acetamide;
(R)-2-{(S)-1-[2-(4-Fluoro-3-methyl-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2,3,5-trifluoro-phenyl)-ethyl]-3,4-dihydro-1H-isoquinolin-2-yl}-2-phenyl-acetamide;
(R)-2-{(S)-1-[2-(4-Fluoro-3-trifluoromethyl-phenyl)-ethyl]-6,7-dimethoxy-3,4-dihydro-1H-isoquinolin-2-yl}-2-phenyl-acetamide;
(R)-2-{(S)-1-[2-(3-Fluoro-5-trifluoromethyl-phenyl)-ethyl]-6,7-dimethoxy-3,4-dihydro-1H-isoquinolin-2-yl}-2-phenyl-acetamide;
(R)-2-{(S)-1-[2-(5-Chloro-2-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Fluoro-3-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Fluoro-4-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Difluoromethoxy-phenyl)-ethyl]-6,7-dimethoxy-3,4-dihydro-1H-isoquinolin-2-yl}-2-phenyl-acetamide;
(R)-2-{(S)-1-[2-(3-Fluoro-2-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Chloro-3-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Fluoro-6-trifluoromethyl-phenyl)-ethyl]-6,7-dimethoxy-3,4-dihydro-1H-isoquinolin-2-yl}-2-phenyl-acetamide;
(R)-2-{(S)-1-[2-(4-Chloro-3-trifluoromethyl-phenyl)-ethyl]-6,7-dimethoxy-3,4-dihydro-1H-isoquinolin-2-yl}-2-phenyl-acetamide;
(R)-2-{(S)-1-[2-(2,3-Difluoro-4-methyl-phenyl)-ethyl]-6,7-dimethoxy-3,4-dihydro-1H-isoquinolin-2-yl}-2-phenyl-acetamide;
(R)-2-{(S)-1-[2-(4-Difluoromethoxy-phenyl)-ethyl]-6,7-dimethoxy-3,4-dihydro-1H-isoquinolin-2-yl}-2-phenyl-acetamide;
(R)-2-{(S)-1-[2-(3,4-Dimethyl-phenyl)-ethyl]-6,7-dimethoxy-3,4-dihydro-1H-isoquinolin-2-yl}-2-phenyl-acetamide;
(R)-2-{(S)-1-[2-(3-Chloro-4-methyl-phenyl)-ethyl]-6,7-dimethoxy-3,4-dihydro-1H-isoquinolin-2-yl}-2-phenyl-acetamide;
(R)-2-{(S)-1-[2-(2-Chloro-6-fluoro-phenyl)-ethyl]-6,7-dimethoxy-3,4-dihydro-1H-isoquinolin-2-yl}-2-phenyl-acetamide;
(R)-2-[(S)-6,7-Dimethoxy-1-(2-o-tolyl-ethyl)-3,4-dihydro-1H-isoquinolin-2-yl]-2-phenyl-acetamide;
(R)-2-[(S)-6,7-Dimethoxy-1-(2-m-tolyl-ethyl)-3,4-dihydro-1H-isoquinolin-2-yl]-2-phenyl-acetamide;
(R)-2-[(S)-6,7-Dimethoxy-1-(2-p-tolyl-ethyl)-3,4-dihydro-1H-isoquinolin-2-yl]-2-phenyl-acetamide;
(R)-2-{(S)-1-[2-(2-Fluoro-phenyl)-ethyl]-6,7-dimethoxy-3,4-dihydro-1H-isoquinolin-2-yl}-2-phenyl-acetamide;
(R)-2-{(S)-1-[2-(3-Fluoro-phenyl)-ethyl]-6,7-dimethoxy-3,4-dihydro-1H-isoquinolin-2-yl}-2-phenyl-acetamide;
(R)-2-{(S)-1-[2-(3,4-Dichloro-phenyl)-ethyl]-6,7-dimethoxy-3,4-dihydro-1H-isoquinolin-2-yl}-2-phenyl-acetamide;
(R)-2-{(S)-1-[2-(3,5-Dimethyl-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2-trifluoromethyl-phenyl)-ethyl]-3,4-dihydro-1H-isoquinolin-2-yl}-2-phenyl-acetamide;
(R)-2-{(S)-1-[2-(2,4-Difluoro-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2,3,6-trifluoro-phenyl)-ethyl]-3,4-dihydro-1H-isoquinolin-2-yl}-2-phenyl-acetamide;
(R)-2-{(S)-6,7-Dimethoxy-1-[2-(4-trifluoromethoxy-phenyl)-ethyl]-3,4-dihydro-1H-isoquinolin-2-yl}-2-phenyl-acetamide;
(R)-2-{(S)-1-[2-(2-Fluoro-3-methyl-phenyl)-ethyl]-6,7-dimethoxy-3,4-dihydro-1H-isoquinolin-2-yl}-2-phenyl-acetamide;
(R)-2-{(S)-1-[2-(4-Chloro-phenyl)-ethyl]-6,7-dimethoxy-3,4-dihydro-1H-isoquinolin-2-yl}-2-phenyl-acetamide;
(R)-2-{(S)-1-[2-(3-Chloro-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(3-trifluoromethoxy-phenyl)-ethyl]-3,4-dihydro-1H-isoquinolin-2-yl}-2-phenyl-acetamide;
(R)-2-{(S)-6,7-Dimethoxy-1-[2-(3,4,5-trifluoro-phenyl)-ethyl]-3,4-dihydro-1H-isoquinolin-2-yl}-2-phenyl-acetamide;
(R)-2-{(S)-6,7-Dimethoxy-1-[2-(3-trifluoromethyl-phenyl)-ethyl]-3,4-dihydro-1H-isoquinolin-2-yl}-2-phenyl-acetamide;
(R)-2-{(S)-6,7-Dimethoxy-1-[2-(2,3,4-trifluoro-phenyl)-ethyl]-3,4-dihydro-1H-isoquinolin-2-yl}-2-phenyl-acetamide;
(R)-2-{(S)-1-[2-(4-Bromo-2-fluoro-phenyl)-ethyl]-6,7-dimethoxy-3,4-dihydro-1H-isoquinolin-2-yl}-2-phenyl-acetamide;
(R)-2-{(S)-1-[2-(2,6-Difluoro-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2-trifluoromethoxy-phenyl)-ethyl]-3,4-dihydro-1H-isoquinolin-2-yl}-2-phenyl-acetamide;
(R)-2-{(S)-1-[2-(2,4-Dichloro-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2,4,5-trifluoro-phenyl)-ethyl]-3,4-dihydro-1H-isoquinolin-2-yl}-2-phenyl-acetamide;
(R)-2-{(S)-1-[2-(3-Bromo-phenyl)-ethyl]-6,7-dimethoxy-3,4-dihydro-1H-isoquinolin-2-yl}-2-phenyl-acetamide;
(R)-2-{(S)-1-[2-(2-Bromo-phenyl)-ethyl]-6,7-dimethoxy-3,4-dihydro-1H-isoquinolin-2-yl}-2-phenyl-acetamide;
(R)-2-{(S)-1-[2-(4-Bromo-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(4-trifluoromethyl-phenyl)-ethyl]-3,4-dihydro-1H-isoquinolin-2-yl}-2-phenyl-acetamide;
(R)-2-{(S)-1-[2-(2,6-Dichloro-phenyl)-ethyl]-6,7-dimethoxy-3,4-dihydro-1H-isoquinolin-2-yl}-2-phenyl-acetamide; and
(R)-2-{(S)-1-[2-(4-tert-Butyl-phenyl)-ethyl]-6,7-dimethoxy-3,4-dihydro-1H-isoquinolin-2-yl}-2-phenyl-acetamide.
(R)-2-{(S)-1-[2-(3-Chloro-2-fluoro-phenyl)-ethyl]-6,7-dimethoxy-3,4-dihydro-1H-isoquinolin-2-yl}-2-phenyl-acetamide;
(R)-2-{(S)-1-[2-(3-Fluoro-4-methyl-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2,3,5-trifluoro-phenyl)-ethyl]-3,4-dihydro-1H-isoquinolin-2-yl}-2-phenyl-acetamide;
(R)-2-{(S)-1-[2-(2-Difluoromethoxy-phenyl)-ethyl]-6,7-dimethoxy-3,4-dihydro-1H-isoquinolin-2-yl}-2-phenyl-acetamide;
(R)-2-{(S)-1-[2-(4-Difluoromethoxy-phenyl)-ethyl]-6,7-dimethoxy-3,4-dihydro-1H-isoquinolin-2-yl}-2-phenyl-acetamide;
(R)-2-{(S)-1-[2-(3,4-Dimethyl-phenyl)-ethyl]-6,7-dimethoxy-3,4-dihydro-1H-isoquinolin-2-yl}-2-phenyl-acetamide;
(R)-2-[(S)-6,7-Dimethoxy-1-(2-o-tolyl-ethyl)-3,4-dihydro-1H-isoquinolin-2-yl]-2-phenyl-acetamide;
(R)-2-[(S)-6,7-Dimethoxy-1-(2-m-tolyl-ethyl)-3,4-dihydro-1H-isoquinolin-2-yl]-2-phenyl-acetamide;
(R)-2-[(S)-6,7-Dimethoxy-1-(2-p-tolyl-ethyl)-3,4-dihydro-1H-isoquinolin-2-yl]-2-phenyl-acetamide;
(R)-2-{(S)-1-[2-(2-Fluoro-phenyl)-ethyl]-6,7-dimethoxy-3,4-dihydro-1H-isoquinolin-2-yl}-2-phenyl-acetamide;
(R)-2-{(S)-1-[2-(3-Fluoro-phenyl)-ethyl]-6,7-dimethoxy-3,4-dihydro-1H-isoquinolin-2-yl}-2-phenyl-acetamide;
(R)-2-{(S)-1-[2-(2,4-Difluoro-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2,3,6-trifluoro-phenyl)-ethyl]-3,4-dihydro-1H-isoquinolin-2-yl}-2-phenyl-acetamide;
(R)-2-{(S)-1-[2-(2-Fluoro-3-methyl-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2,3,4-trifluoro-phenyl)-ethyl]-3,4-dihydro-1H-isoquinolin-2-yl}-2-phenyl-acetamide;
(R)-2-{(S)-6,7-Dimethoxy-1-[2-(2,4,5-trifluoro-phenyl)-ethyl]-3,4-dihydro-1H-isoquinolin-2-yl}-2-phenyl-acetamide;
(R)-2-{(S)-1-[2-(3-Bromo-phenyl)-ethyl]-6,7-dimethoxy-3,4-dihydro-1H-isoquinolin-2-yl}-2-phenyl-acetamide.

The present invention encompasses physiologically usable or pharmaceutically acceptable salts of compounds of formula (I). This encompasses salts with physiologically compatible mineral acids such as hydrochloric acid, sulphuric or phosphoric acid; or with organic acids such as formic acid, methanesulphonic acid, acetic acid, trifluoroacetic acid, citric acid, fumaric acid, maleic acid, tartaric acid, succinic acid or salicylic acid and the like. The compounds of formula (I) which are acidic can also form salts with physiologically compatible bases. Examples of such salts are alkali metal, alkali earth metal, ammonium and alkylammonium salts such as Na, K, Ca or tetraalkylammonium salt. For a comprehensive list see "Handbook of Pharmaceutical Salts", P.H. Stahl, C.G. Wermuth Eds., Wiley-VCH, Weinheim/Zürich 2002, p. 329-350. The compounds of formula (I) can also be present in the form of a zwitterion.

The present invention encompasses different solvation complexes of compounds of general formula (I). The solvation can be effected in the course of the manufacturing process or can take place separately, e.g. as a consequence of hygroscopic properties of an initially anhydrous compound of general formula (I).

The present invention further encompasses different morphological forms, e.g. crystalline forms, of compounds of general formula (I) and their salts and solvation complexes. Particular heteromorphs may exhibit different dissolution properties, stability profiles, and the like, and are all included in the scope of the present invention.

The compounds of formula (I) might have one or several asymmetric centres and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates and the meso-forms.

Preferred compounds of formula (I) have IC₅₀ values below 100 nM, particularly preferred compounds have IC₅₀ values below 10 nM which have been determined with the FLIPR (Fluorometric Imaging Plates Reader) method described in the beginning of the experimental section.

The compounds of formula (I) and their pharmaceutically usable salts are useful in the preparation of a medicament for the prevention or treatment of diseases selected from the group consisting of depression; anxiety; addictions; obsessive compulsive disorder; affective neurosis; depressive neurosis; anxiety neurosis; dysthymic disorder; mood disorder; sexual dysfunction; psychosexual dysfunction; schizophrenia; manic depression; delirium; dementia; severe mental retardation and dyskinesias such as Huntington's disease and Tourette syndrome; diabetes; appetite/taste disorders; vomiting/nausea; asthma; Parkinson's disease; Cushing's syndrome/disease; basophil adenoma; prolactinoma; hyperprolactinemia; hypopituitarism; hypophysis tumour/adenoma; hypothalamic diseases; inflammatory bowel disease; gastric dyskinesia; gastric ulcers; Froehlich's syndrome; hypophysis diseases, hypothalamic hypogonadism; Kallman's syndrome (anosmia, hyposmia); functional or psychogenic amenorrhea; hypothalamic hypothyroidism; hypothalamic-adrenal dysfunction; idiopathic hyperprolactinemia; hypothalamic disorders of growth hormone deficiency; idiopathic growth deficiency; dwarfism; gigantism; acromegaly; disturbed biological and circadian rhythms; sleep disturbances associated with diseases such as neurological disorders, neuropathic pain and restless leg syndrome; heart and lung diseases, acute and congestive heart failure; hypotension; hypertension; urinary retention; osteoporosis; angina pectoris; myocardial infarction; ischemic or haemorrhagic stroke; subarachnoid haemorrhage; ulcers; allergies; benign prostatic hypertrophy; chronic renal failure; renal disease; impaired glucose tolerance; migraine; pain; enhanced or exaggerated sensitivity to pain such as hyperalgesia, causalgia, and allodynia; acute pain; burn pain; atypical facial pain; neuropathic pain; back pain; complex regional pain syndrome I and II; arthritic pain; sports injury pain; pain related to infection e.g. by HIV; post-chemotherapy pain; post-stroke pain; post-operative pain; neuralgia; conditions associated with visceral pain such as irritable bowel syndrome; migraine and angina; urinary bladder incontinence e.g. urge incontinence; tolerance to narcotics or withdrawal from narcotics; sleep disorders; eating disorders; cardiovascular disorders; neurodegenerative disorders; sleep apnea; narcolepsy; insomnia; parasomnia; and neurodegenerative disorders including nosological entities such as disinhibition-dementia-parkinsonism-amyotrophy complex; pallido-ponto-nigral degeneration epilepsy; seizure disorders and other diseases related to general orexin system dysfunctions.

Compounds of the general formula (I) are particularly suitable for use in the treatment of diseases or disorders selected from the group consisting of eating disorders or sleep disorders.
Eating disorders may be defined as comprising metabolic dysfunction; dysregulated appetite control; compulsive obesities; emeto-bulimia or anorexia nervosa. This pathologically modified food intake may result from disturbed appetite (attraction or aversion for food); altered energy balance (intake vs expenditure); disturbed perception of food quality (high fat or carbohydrates, high palatability); disturbed food availability (unrestricted diet or deprivation) or disrupted water balance.
Sleep disorders include insomnias, narcolepsy and other disorders of excessive sleepiness, sleep-related dystonias; restless leg syndrome; sleep apneas; jet-lag syndrome; shift-work syndrome, delayed or advanced sleep phase syndrome. Insomnias are defined as comprising sleep disorders associated with aging; intermittent treatment of chronic insomnia; situational transient insomnia (new environment, noise) or short-term insomnia due to stress; grief; pain or illness.

The compounds of formula (I) and their pharmaceutically usable salts can be used as medicament (e.g. in the form of pharmaceutical preparations). The pharmaceutical preparations can be administered enterally, such as orally (e.g. in the form of tablets, coated tablets, dragées, hard and soft gelatine capsules, solutions, emulsions or suspensions), nasally (e.g. in the form of nasal sprays) or rectally (e.g. in the form of suppositories). However, the administration can also be effected parenterally, such as intramuscularly or intravenously (e.g. in the form of injection solutions), or topically, e.g. in the form of ointments, creams or oils.

The compounds of formula (I) and their pharmaceutically usable salts can be processed with pharmaceutically inert, inorganic or organic adjuvants for the production of tablets, coated tablets, dragées, and hard gelatine capsules. Lactose, cornstarch or derivatives thereof, talc, stearic acid or its salts etc. can be used, for example, as such adjuvants for tablets, dragées, and hard gelatine capsules. Suitable adjuvants for soft gelatine capsules, are, for example, vegetable oils, waxes, fats, semi-solid substances and liquid polyols, etc. Suitable adjuvants for the production of solutions and syrups are, for example, water, polyols, saccharose, invert sugar, glucose, etc. Suitable adjuvants for injection solutions are, for example, water, alcohols, polyols, glycerol, vegetable oils, etc. Suitable adjuvants for suppositories are, for example, natural or hardened oils, waxes, fats, semi-solid or liquid polyols, etc.

Moreover, the pharmaceutical preparations can contain preservatives, solubilizers, viscosity-increasing substances, stabilisers, wetting agents, emulsifiers, sweeteners, colorants, flavorants, salts for varying the osmotic pressure, buffers, masking agents or antioxidants. The compounds of formula (I) may also be used in combination with one or more other therapeutically useful substances. Examples are anorectic drugs like fenfluramine and related substances; lipase inhibitors like orlistat and related substances; antidepressants like fluoxetine and related substances; anxiolytics like alprazolam and related substances; sleep-inducers like zopiclone and related substances; or any other therapeutically useful substance.

The dosage of compounds of formula (I) can vary within wide limits depending on the disease to be controlled, the age and the individual condition of the patient and the mode of administration, and will, of course, be fitted to the individual requirements in each particular case. For adult patients a daily dosage of about 1 mg to 1000 mg, especially about 50 mg to about 500 mg, comes into consideration.

The pharmaceutical preparations conveniently contain about 1 - 500 mg, preferably 5 - 200 mg of a compound of formula (I).

The compounds of formula (I) of the present invention are prepared according to the general sequence of reactions outlined in the schemes below, wherein R¹, R², R³, R⁴, R⁵, R⁶ are as defined in formula (I) above. As the case may be any compound obtained with one or more optically active carbon atom may be resolved into pure enantiomers or diastereoisomers, mixtures of enantiomers or diastereoisomers, diastereoisomeric racemates and the meso-forms in a manner known per se.

The compounds obtained may also be converted into a pharmaceutically acceptable salt thereof in a manner known per se.

The compounds of formula (I) may be prepared as single compounds or as libraries of compounds comprising at least 2, typically 5 to 100 compounds of formula (I).

Compound libraries are prepared by multiple parallel synthesis using solution phase chemistry.

The compounds of formula (I) can be prepared as mixtures of all possible stereoisomers by following one of three possible synthetic pathways (*Scheme 1*). The first pathway starts with the reaction of the respective 1,2,3,4-tetrahydroisoquinoline with a methyl bromoacetate derivative. After saponification of the ester the obtained acid is coupled with ammonium bromide in the presence of EDC and DMAP to give the desired amides of formula (I). 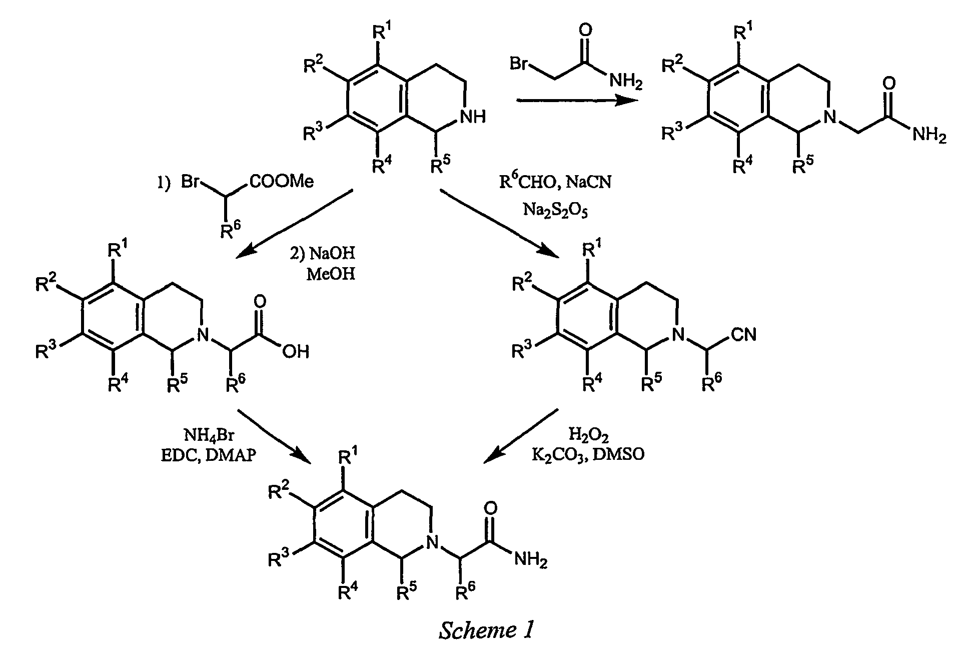 In a second pathway the 1,2,3,4-tetrahydroisoquinoline is reacted with an aldehyde and sodium cyanide to give the corresponding α-amino acetonitrile derivatives. These can be hydrolysed with hydrogen peroxide and potassium carbonate as base. In the case of the unsubstituted glycinamide analogues (R⁶ = H) the final compounds of general formula (I) can be obtained by reaction of the 1,2,3,4-tetrahydroisoquinoline with commercially available 2-bromo-acetamide. The 1,2,3,4-tetrahydroisoquinolines, if not commercially available, can be prepared as racemic mixtures from the corresponding phenylethylamines by coupling with the desired carboxylic acid followed by treatment with POCl₃ and finally NaBH₄ (see experimental part).

The stereoselective synthesis of the desired 1,2,3,4-tetrahydroisoquinoline derivatives is possible following one of the synthetic pathways shown in *scheme 2*. The key-intermediates, the 1-arylethyl-substituted 3,4-dihydroisoquinolines (R¹¹ = aryl), can be obtained either by cyclisation of *N*-arylethyl-propionamides with POCl₃ or by alkylation of 1-methyl-3,4-dihydroisoquinoline derivatives with arylmethyl bromides or chlorides. These intermediates can be reduced to enantiomerically enriched 1,2,3,4-tetrahydroisoquinoline derivatives by transfer hydrogenation in the presence of a chiral Ru(II) complex (chiral catalyst), which was originally described by R. Noyori et al. (J. Am. Chem. Soc. 1996, 118, 4916-4917 and WO 97/20789).

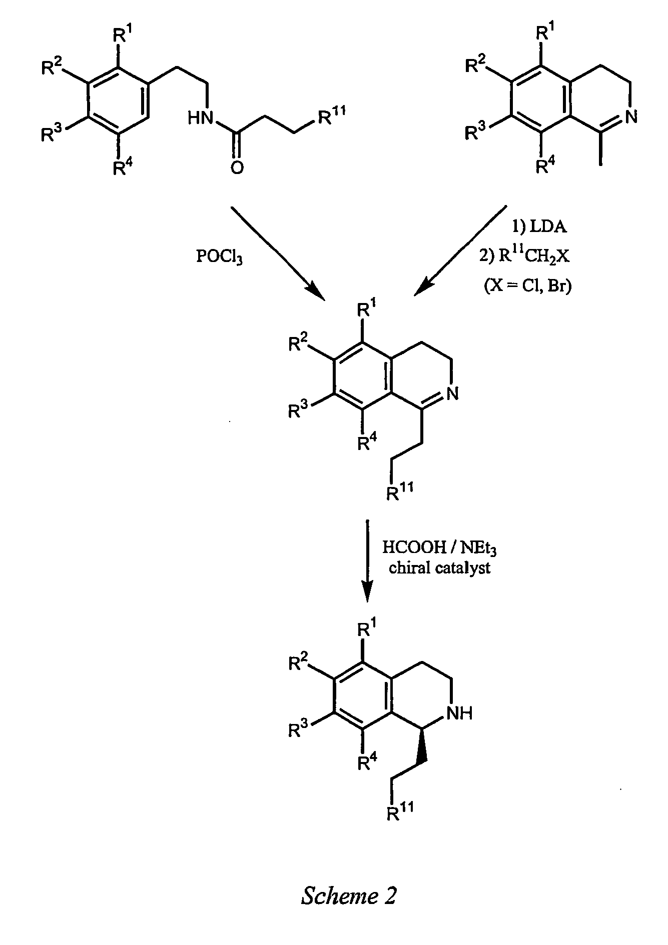

The chiral catalyst (Ru(II) complex) used is as follows:

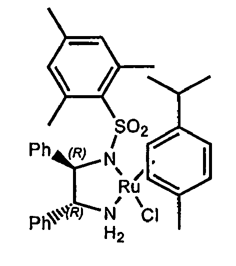

As illustrated in *scheme 3* enantiomerically enriched 1,2,3,4-tetrahydroisoquinoline intermediates can be converted to compounds of formula (I) by alkylation with tosylated α-hydroxy acetamides. With racemic tosylates mixtures of diastereoisomers of formula (I) are obtained. Optically pure tosylates lead to essentially only one diastereoisomer. These can be prepared in a two step procedure starting from a single enantiomer of a 2-substituted methyl glycolate derivative by amidation and tosylation.

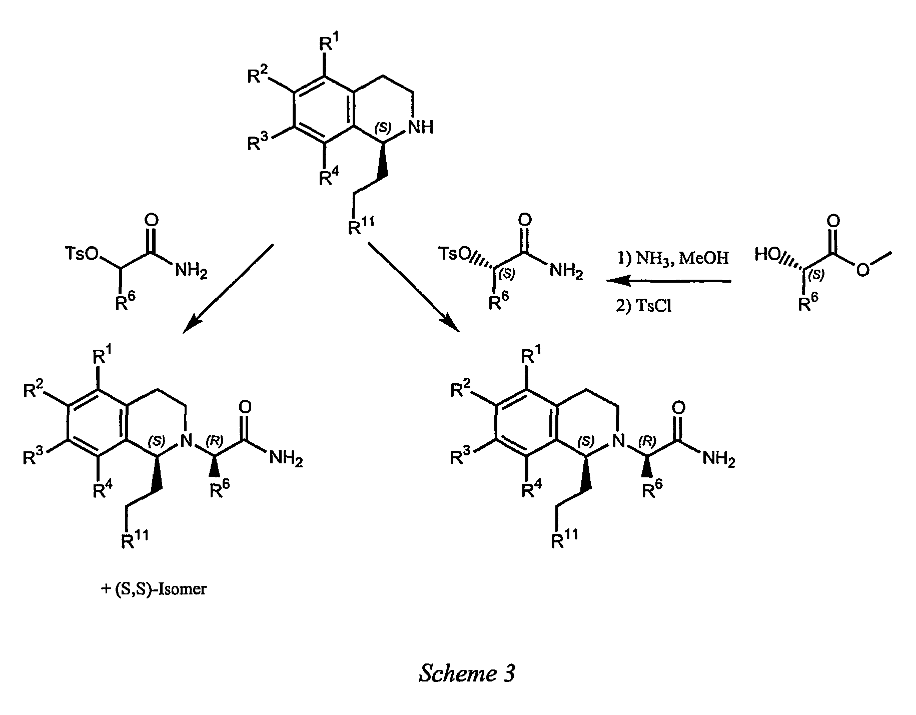

The 1,2,3,4-tetrahydroisoquinoline derivatives exemplified in this invention may be prepared from readily available starting materials using the following general methods and procedures. It will be appreciated that where typical or preferred experimental conditions (i.e., reaction temperatures, time, moles of reagents, solvents, etc.) are given, other experimental conditions can also be used unless otherwise stated. Optimum reaction conditions may vary with the particular reactants or solvents used, but such conditions can be determined by one skilled in the art using routine optimisation procedures.

### Experimental Section

### Abbreviations:

| | |
|---|---|
| BSA | Bovine serum albumine |
| CHO | Chinese hamster ovary |
| d | day(s) |
| dia | diastereoisomer |
| DCM | Dichloromethane |
| DIPEA | Diisopropylethylamine |
| DMAP | Dimethylaminopyridine |
| DMF | *N,N*-dimethylformamide |
| DMSO | Dimethylsulfoxide |
| EDC | 1-(3-Dimethylaminopropyl)-3-ethylcarbodiimide |
| ES | Electron spray |
| FCS | Foetal calf serum |
| FLIPR | Fluorescent imaging plate reader |
| h | Hour(s) |
| HBSS | Hank's balanced salt solution |
| HEPES | 4-(2-Hydroxyethyl)-piperazine-1-ethanesulfonic acid |
| HPLC | High pressure/performance liquid chromatography |
| MS | Mass spectroscopy |
| LC | Liquid chromatography |
| LDA | Lithium diisopropylamide |
| min | Minute(s) |
| prep | preparative |
| rt | retention time |
| RT | Room temperature |
| sat | saturated |
| THF | Tetrahydrofuran |

### I. Biology

### Determination of Orexin receptor antagonistic activity

The Orexin receptor antagonistic activity of the compounds of formula (I) is determined in accordance with the following experimental method.

### Experimental method:

### Intracellular calcium measurements

Chinese hamster ovary (CHO) cells expressing the human orexin-1 receptor and the human orexin-2 receptor, respectively, are grown in culture medium (Ham F-12 with L-Glutamine) containing 300 µg/ml G418, 100 U/ml penicillin, 100 µg/ml streptomycin and 10 % inactivated foetal calf serum (FCS).
The cells are seeded at 80'000 cells / well into 96-well black clear bottom sterile plates (Costar) which have been precoated with 1% gelatine in Hanks' Balanced Salt Solution (HBSS). All reagents are from Gibco BRL.
The seeded plates were incubated overnight at 37°C in 5% CO₂.
Human orexin-A as an agonist is prepared as 1 mM stock solution in methanol:water (1:1), diluted in HBSS containing 0.1 % bovine serum albumin (BSA) and 2 mM HEPES for use in the assay at a final concentration of 10 nM.
Antagonists are prepared as 10 mM stock solution in DMSO, then diluted in 96-well plates, first in DMSO, then in HBSS containing 0.1 % bovine serum albumin (BSA) and 2 mM HEPES.
On the day of the assay, 100 µl of loading medium (HBSS containing 1% FCS, 2 mM HEPES, 5 mM probenecid (Sigma) and 3 µM of the fluorescent calcium indicator fluo-3 AM (1 mM stock solution in DMSO with 10% pluronic acid) (Molecular Probes) is added to each well.
The 96-well plates are incubated for 60 min at 37° C in 5% CO₂. The loading solution is then aspirated and cells are washed 3 times with 200 µl HBSS containing 2.5 mM probenecid, 0.1% BSA, 2 mM HEPES. 100 µl of that same buffer is left in each well.
Within the Fluorescent Imaging Plate Reader (FLIPR, Molecular Devices), antagonists are added to the plate in a volume of 50 µl, incubated for 20 min and finally 100 µl of agonist is added. Fluorescence is measured for each well at 1 second intervals, and the height of each fluorescence peak is compared to the height of the fluorescence peak induced by 10 nM orexin-A with buffer in place of antagonist. For each antagonist, IC₅₀ value (the concentration of compound needed to inhibit 50 % of the agonistic response) is determined. The compounds exhibit as an average an antagonistic activity regarding the OX₁ and OX₂ receptor in the range of an IC₅₀ of 1 to 100 nM.

### II. Chemistry

The following examples illustrate the preparation of pharmacologically active compounds of the invention but do not at all limit the scope thereof.
All temperatures are stated in °C.
All analytical and preparative HPLC investigations on non-chiral phases are performed using RP-C18 based columns. Analytical HPLC investigations are performed on two different instruments with cycle-times of ∼2.5 min and ∼13 min respectively. For HPLC separations on chiral phases a Chiralcel OD column from Daicel Chemical Industries is used.

### A Synthesis of Phenylethylamide Derivatives:

**Procedure 1:**
   A solution of the respective phenylethylamine (82.8 mmol) and the respective carboxylic acid (82.8 mmol) in toluene (330 mL) is treated with 2 crystals of p-toluenesulfonic acid and refluxed for 14 h in the presence of a Dean-Stark. The solvent is removed in vacuo and the residue is recrystalized from toluene to give the following amides:
   ***N*-[2-(3,4-Dimethoxy-phenyl)-ethyl]-2-naphthalen-2-yl-acetamide** 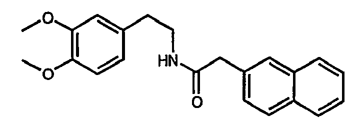 prepared by reaction of 3,4-dimethoxyphenylethylamine and 2-naphthylacetic acid.
   **3-(3,4-Dimethoxy-phenyl)-N-[2-(3,4-dimethoxy-phenyl)-ethyl]-propionamide:** 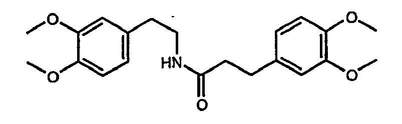 prepared by reaction of 3-(3,4-dimethoxyphenyl)-propionic acid and 3,4-dimethoxy-phenylethylamine.
      LC-MS: rt = 0.89 min, 374 (M+1, ES+).
   **N-[2-(3,4-Dimethoxy-phenyl)-ethyl]-3-(4-trifluoromethyl-phenyl)-propionamide:** 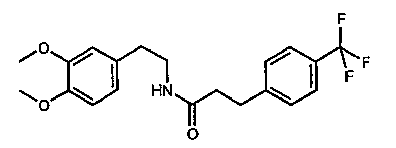 prepared by reaction of 3-(4-trifluoromethylphenyl)-propionic acid and 3,4-dimethoxy-phenylethylamine.
      LC-MS: rt = 1.03 min, 382 (M+1, ES+).
   **N-[2-(3,4-Dimethoxy-phenyl)-ethyl]-2-(4-fluoro-phenyl)-acetamide:** 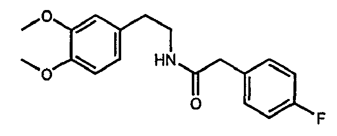 prepared by reaction of 2-(4-fluorophenyl)-acetic acid and 3,4-dimethoxyphenylethylamine.
      LC-MS: rt = 4.11 min, 318 (M+1, ES+).
**Procedure 2:**
   A solution of the respective carboxylic acid (11.0 mmol) in THF (20 mL) is treated with EDC hydrochloride (11.0 mmol). After 5 min the respective amine (11.0 mmol) is added and the reaction mixture is stirred for 14 h. Ethyl acetate is added and the organic layer is washed with sat. aqueous NaHCO₃ solution, 10% aqueous citric acid solution and brine. The organic layer is dried with MgSO₄ and filtered. After evaporation of the solvents the following crude amides are obtained which are used in the following step without further purification:
   **3-(3,4-Difluoro-phenyl)-N-[2-(3,4-dimethoxy-phenyl)-ethyl]-propionamide:** 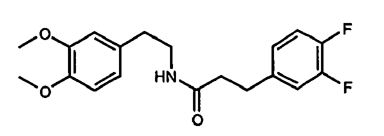 prepared by reaction of 3-(3,4-difluorophenyl)-propionic acid and 3,4-dimethoxy-phenylethylamine.
   **3-[2,5-Bis(trifluoromethyl)-phenyl]-N-[2-(3,4-dimethoxy-phenyl)-ethyl]-propionamide:** 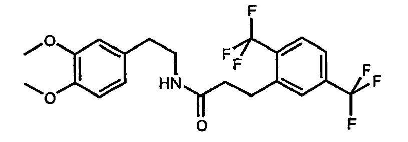 prepared by reaction of 3-[2,5-bis(trifluoromethyl)-phenyl]-propionic acid and 3,4-dimethoxyphenylethylamine.
   **3-(2,5-Difluoro-phenyl)-N-[2-(3,4-dimethoxy-phenyl)-ethyl]-propionamide:** 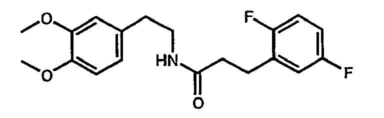 prepared by reaction of 3-(2,5-difluorophenyl)-propionic acid and 3,4-dimethoxy-phenylethylamine.
   **N-[2-(3,4-Dimethoxy-phenyl)-ethyl]-3-(2-methoxy-phenyl)-propionamide:** 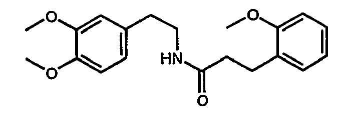 prepared by reaction of 3-(2-methoxyphenyl)-propionic acid and 3,4-dimethoxy-phenylethylamine.
   **N-[2-(3,4-Dimethoxy-phenyl)-ethyl]-3-(4-fluoro-phenyl)-propionamide:** 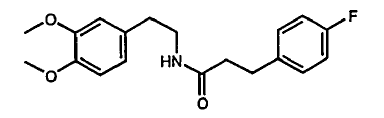 prepared by reaction of 3-(4-fluorophenyl)-propionic acid and 3,4-dimethoxy-phenylethylamine.
   **3-(2,3-Difluoro-phenyl)-N-[2-(3,4-dimethoxy-phenyl)-ethyl]-acrylamide:** 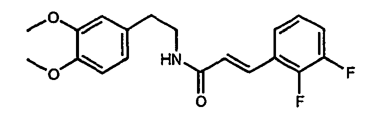 prepared by reaction of 3-(2,3-difluoro-phenyl)-acrylic acid and 3,4-dimethoxy-phenylethylamine.
   **N-[2-(3,4-Dimethoxy-phenyl)-ethyl]-2-(3-fluoro-4-methoxy-phenyl)-acetamide:** 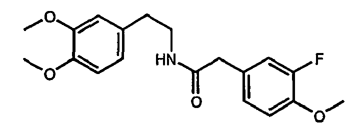 prepared by reaction of 2-(3-fluoro-4-methoxy-phenyl)-acetic acid and 3,4-dimethoxy-phenylethylamine.
   **3-(2,3-Difluoro-phenyl)-N-[2-(3,4-dimethoxy-phenyl)-ethyl]-propionamide:** 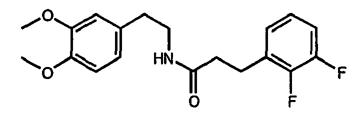 prepared by reaction of 3-(2,3-difluorophenyl)-propionic acid and 3,4-dimethoxy-phenylethylamine.
   **N-[2-(3,4-Dimethoxy-phenyl)-ethyl]-3-(3-methoxy-phenyl)-propionamide:** 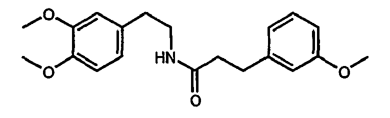 prepared by reaction of 3-(3-methoxyphenyl)-propionic acid and 3,4-dimethoxy-phenylethylamine.
   **3-(2,5-Difluoro-phenyl)-N-[2-(3,4-dimethoxy-phenyl)-ethyl]-acrylamide:** 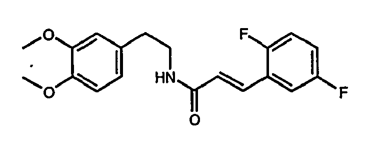 prepared by reaction of 3-(2,5-difluoro-phenyl)-acrylic acid and 3,4-dimethoxy-phenylethylamine.
**Procedure 3:**
   To a solution of the respective carboxylic acid (21.4 mmol) in DMF (110 mL) is added successively PyBOP (23.6 mmol), 3,4-dimethoxy-phenylethylamine (21.4 mmol) and N-diisopropylethylamine (49.3 mmol). After stirring for 8 h at RT ethyl acetate (100 mL) is added and the organic layer is washed three times with brine (3x70 mL). The organic layer is dried with MgSO₄ and filtered. The solvent is removed in vacuo und the residue is purified by flash-chromatography (DCM/MeOH 36/1) to give the following amides:
   **N-[2-(3,4-Dimethoxy-phenyl)-ethyl]-3-furan-2-yl-propionamide:** 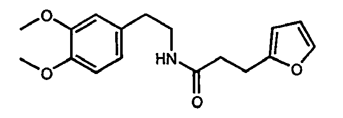 prepared by reaction of 3-furan-2-yl-propionic acid and 3,4-dimethoxy-phenylethylamine.
      LC-MS: rt = 3.96 min, 304 (M+1, ES+).
   **N-[2-(3,4-Dimethoxy-phenyl)-ethyl]-4-phenyl-butyramide:** 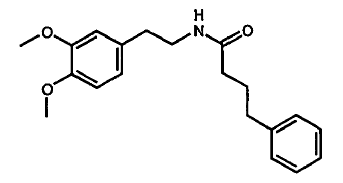 prepared by reaction of 4-phenyl-butyric acid and 3,4-dimethoxy-phenylethylamine.
      LC-MS: rt = 4.47 min, 328 (M+1, ES+).
**Procedure 4:**
   A solution of the respective phenylethylamine (80 mmol) and of triethylamine (90 mmol) in THF (120 mL) is cooled to 0°C and treated portionwise with the respective carboxylic acid chloride (80 mmol). After stirring for 10 min at 0°C and for 14 h at RT a sat. aqueous NaHCO₃ solution is added, the layers are separated and the aqueous layer is extracted three times with ethyl acetate (150 mL). The solvent is removed in vacuo and the residue is either recrystallized from toluene or purified by flash chromatography to give the following amides:
   **2-(2,5-Dimethoxy-phenyl)-N-[2-(2,5-dimethoxy-phenyl)-ethyl]-acetamide:** 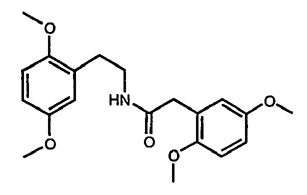 prepared by reaction of (2,5-dimethoxy-phenyl)-acetyl chloride and 2,5-dimethoxy-phenylethylamine.
      LC-MS: rt = 4.62 min, 360 (M+1, ES+).
   **N-[2-(3-Methoxy-phenyl)-ethyl]-2-phenyl-acetamide:** 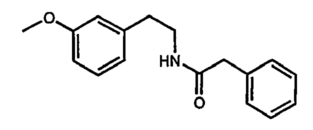 prepared by reaction of phenyl-acetyl chloride and 3-methoxy-phenylethylamine.
      LC-MS: rt = 4.36 min, 270 (M+1, ES+).
   **2-(2,5-Dimethoxy-phenyl)-N-[2-(3,4-dimethoxy-phenyl)-ethyl]-acetamide:** 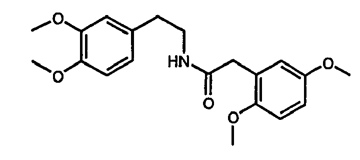 prepared by reaction of (2,5-dimethoxy-phenyl)-acetyl chloride and 3,4-dimethoxy-phenylethylamine.
      LC-MS: rt = 4.11 min, 360 (M+1, ES+).
   **N-[2-(3,4-Dimethoxy-phenyl)-ethyl]-2-(4-methoxy-phenyl)-acetamide:** 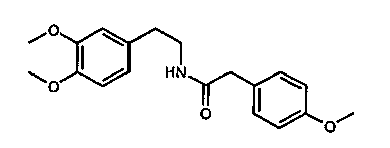 prepared by reaction of (4-methoxy-phenyl)-acetyl chloride and 3,4-dimethoxy-phenylethylamine.
      LC-MS: rt = 3.99 min, 330 (M+1, ES+).
   **N-[2-(3,4-Dimethoxy-phenyl)-ethyl]-2-(3-methoxy-phenyl)-acetamide:** 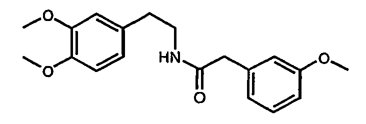 prepared by reaction of (3-methoxy-phenyl)-acetyl chloride and 3,4-dimethoxy-phenylethylamine.
      LC-MS: rt = 4.03 min, 330 (M+1, ES+).
   **N-[2-(2,3-Dihydro-benzo[1,4]dioxin-6-yl)-ethyl]-2-(3,4-dimethoxy-phenyl)-acetamide:** 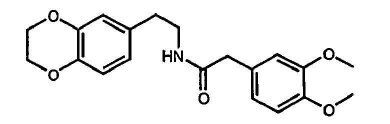 prepared by reaction of (3,4-dimethoxy-phenyl)-acetyl chloride and 2-(2,3-dihydro-benzo[1,4]dioxin-6-yl)-ethylamine.
      LC-MS: rt = 4.03 min, 358 (M+1, ES+).
   **N-[2-(3,4-Dimethoxy-phenyl)-ethyl]-2-phenoxy-acetamide:** 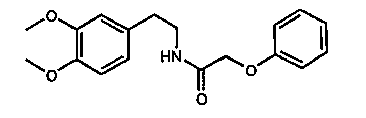 prepared by reaction of phenoxy-acetyl chloride and 3,4-dimethoxy-phenylethylamine.
      LC-MS: rt = 4.24 min, 316 (M+1, ES+).

### B Synthesis of 3,4-Dihydroisoquinoline Derivatives (general procedure):

Phosphorus oxychloride (123 mmol) is added to a suspension of the respective amide (55.3 mmol) in acetonitrile (300 mL). The mixture is refluxed for 90 min and the solvents are removed in vacuo. Methanol (100 mL) is added and evaporated again. The obtained product is recrystallized from dioxane or dioxane/ethanol. After filtration the obtained hydrochloride salt is converted to the free base by addition of saturated aqueous NaHCO₃ solution and extraction with dichloromethane. The solvents are removed in vacuo to give the respective 3,4-dihydroisoquinoline.

### 6,7-dimethoxy-1-[2-(4-trifluoromethyl-phenyl)-ethyl]-3,4-dihydroisoquinoline:

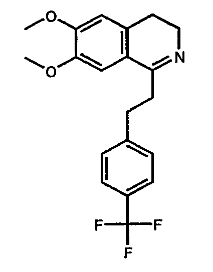

prepared by cyclisation of *N*-[2-(3,4-dimethoxy-phenyl)-ethyl]-3-(4-trifluoromethyl-phenyl)-propionamide.
LC-MS: rt = 0.81 min, 364 (M+1, ES+).

### C Synthesis of 1,2,3,4-Tetrahydroisoquinoline Derivatives:

### C.1 Synthesis of 1,2,3,4-Tetrahydroisoquinolines via Bischler-Napieralski-reaction (general procedure):

To a suspension of the respective acetamide (6.0 mmol) in acetonitrile (80 mL) is added phosphorus oxychloride (2.8 mL, 30 mmol). The mixture is heated to reflux for 2 h and the solvent is removed in vacuo. The resulting oil is taken up in MeOH (10 mL), evaporated to dryness, dissolved in MeOH (35 mL) and cooled to 0°C. NaBH₄ (30 mmol) is added in small (!) portions and the reaction mixture is stirred for 14 h. The solvent is removed in vacuo, ethyl acetate (100 mL) and water (150 mL) are added, the layers are separated and the aqueous layer is extracted three times with ethyl acetate (50 mL). The combined organic extracts are concentrated in vacuo to give the following tetrahydroisoquinolines as racemic mixtures, which are purified by crystallization as hydrochloride salt:
**6,7-Dimethoxy-1-naphthalen-2-ylmethyl-1,2,3,4-tetrahydroisoquinoline:** 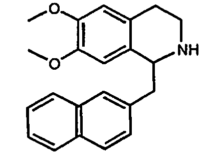 prepared by cyclisation of *N*-[2-(3,4-dimethoxy-phenyl)-ethyl]-2-naphthalen-2-yl-acetamide.
   LC-MS: rt = 0.75 min, 334 (M+1, ES+), 375 (M+CH₃CN, ES+).
**1-[2-(3,4-Difluoro-phenyl)-ethyl]-6,7-dimethoxy-1,2,3,4-tetrahydroisoquinoline:** 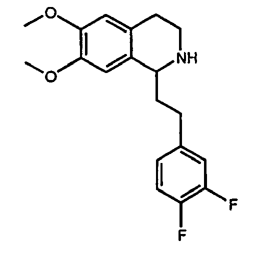 prepared by cyclisation of 3-(3,4-difluoro-phenyl)-N-[2-(3,4-dimethoxy-phenyl)-ethyl]-propionamide.
   LC-MS: rt = 0.82 min, 334 (M+1, ES+), 375 (M+CH₃CN, ES+).
**1-[2-(2,5-Bis-trifluoromethyl-phenyl)-ethyl]-6,7-dimethoxy-1,2,3,4-tetrahydroisoquinoline:** 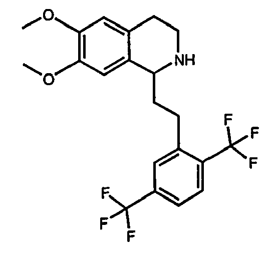 prepared by cyclisation of 3-[2,5-bis(trifluoromethyl)-phenyl]-N-[2-(3,4-dimethoxy-phenyl)-ethyl]-propionamide.
   LC-MS: rt = 0.89 min, 434 (M+1, ES+), 475 (M+CH₃CN, ES+).
**1-[2-(2,5-Difluoro-phenyl)-ethyl]-6,7-dimethoxy-1,2,3,4-tetrahydroisoquinoline:** 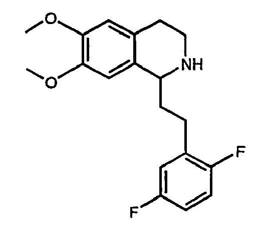 prepared by cyclisation of 3-(2,5-difluoro-phenyl)-N-[2-(3,4-dimethoxy-phenyl)-ethyl]-propionamide.
   LC-MS: rt = 0.80 min, 334 (M+1, ES+).
**6,7-Dimethoxy-1-[2-(2-methoxy-phenyl)-ethyl]-1,2,3,4-tetrahydroisoquinoline:** 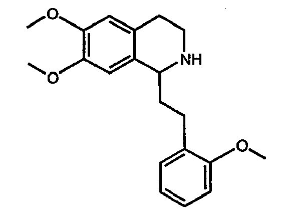 prepared by cyclisation of N-[2-(3,4-dimethoxy-phenyl)-ethyl]-3-(2-methoxyphenyl)-propionamide.
   LC-MS: rt = 0.81 min, 328 (M+1, ES+).
**1-[2-(4-Fluoro-phenyl)-ethyl]-6,7-dimethoxy-1,2,3,4-tetrahydro-isoquinoline:** 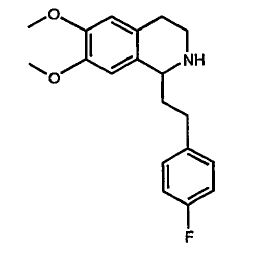 prepared by cyclisation of N-[2-(3,4-dimethoxy-phenyl)-ethyl]-3-(4-fluorophenyl)-propionamide.
   LC-MS: rt = 0.81 min, 316 (M+1, ES+).
**1-[2-(2,3-Difluoro-phenyl)-vinyl]-6,7-dimethoxy-1,2,3,4-tetrahydroisoquinoline:** 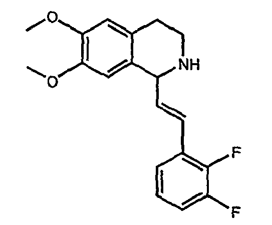 prepared by cyclisation of 3-(2,3-difluoro-phenyl)-N-[2-(3,4-dimethoxy-phenyl)-ethyl]-acrylamide.
   LC-MS: rt = 0.81 min, 332 (M+1, ES+).
**1-(3-Fluoro-4-methoxy-benzyl)-6,7-dimethoxy-1,2,3,4-tetrahydroisoquinoline:** 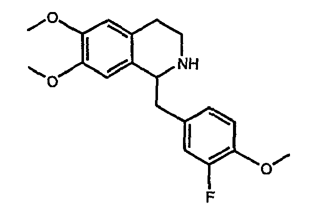 prepared by cyclisation of N-[2-(3,4-dimethoxy-phenyl)-ethyl]-2-(3-fluoro-4-methoxy-phenyl)-acetamide.
   LC-MS: rt = 0.78 min, 332 (M+1, ES+).
**1-[2-(3,4-Dimethoxy-phenyl)-ethyl]-6,7-dimethoxy-1,2,3,4-tetrahydroisoquinoline:** 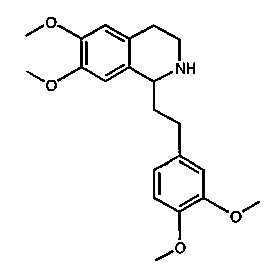 prepared by cyclisation of 3-(3,4-dimethoxy-phenyl)-N-[2-(3,4-dimethoxyphenyl)-ethyl]-propionamide.
   LC-MS: rt = 0.76 min, 358 (M+1, ES+).
**1-[2-(2,3-Difluoro-phenyl)-ethyl]-6,7-dimethoxy-1,2,3,4-tetrahydroisoquinoline:** 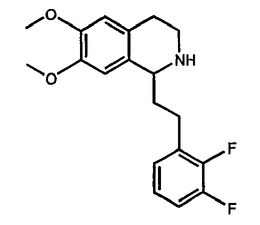 prepared by cyclisation of 3-(2,3-difluoro-phenyl)-N-[2-(3,4-dimethoxy-phenyl)-ethyl]-propionamide.
   LC-MS: rt = 0.80 min, 334 (M+1, ES+).
**6,7-Dimethoxy-1-[2-(4-trifluoromethyl-phenyl)-ethyl]-1,2,3,4-tetrahydroisoquinoline:** 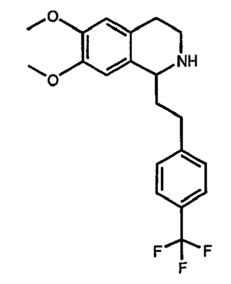 prepared by cyclisation of N-[2-(3,4-dimethoxy-phenyl)-ethyl]-3-(4-trifluoromethyl-phenyl)-propionamide.
   LC-MS: rt = 0.85 min, 366 (M+1, ES+).
**6,7-Dimethoxy-1-[2-(3-methoxy-phenyl)-ethyl]-1,2,3,4-tetrahydroisoquinoline:** 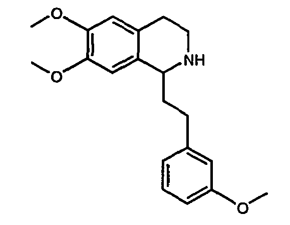 prepared by cyclisation of N-[2-(3,4-dimethoxy-phenyl)-ethyl]-3-(3-methoxyphenyl)-propionamide.
   LC-MS: rt = 0.79 min, 328 (M+1, ES+).
**1-[2-(2,5-Difluoro-phenyl)-vinyl]-6,7-dimethoxy-1,2,3,4-tetrahydroisoquinoline:** 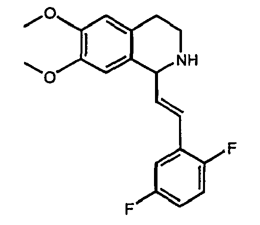 prepared by cyclisation of 3-(2,5-difluoro-phenyl)-N-[2-(3,4-dimethoxy-phenyl)-ethyl]-acrylamide.
   LC-MS: rt = 0.80 min, 332 (M+1, ES+).
**1-(2-Furan-2-yl-ethyl)-6,7-dimethoxy-1,2,3,4-tetrahydro-isoquinoline:** 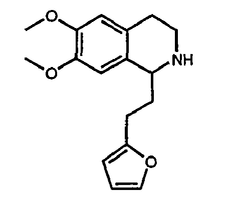 prepared by cyclisation of N-[2-(3,4-dimethoxy-phenyl)-ethyl]-3-furan-2-yl-propionamide.
   LC-MS: rt = 2.70 min, 288 (M+1, ES+).
**1-(2,5-Dimethoxy-benzyl)-5,8-dimethoxy-1,2,3,4-tetrahydro-isoquinoline:** 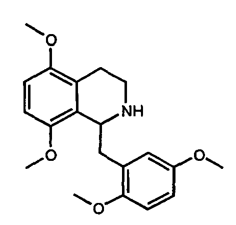 prepared by cyclisation of 2-(2,5-dimethoxy-phenyl)-N-[2-(2,5-dimethoxyphenyl)-ethyl]-acetamide.
   LC-MS: rt = 3.57 min, 344 (M+1, ES+).
**1-Benzyl-6-methoxy-1,2,3,4-tetrahydro-isoquinoline:** 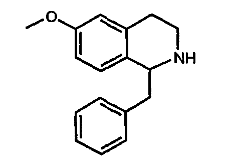 prepared by cyclisation of N-[2-(3-methoxy-phenyl)-ethyl]-2-phenyl-acetamide.
   LC-MS: rt = 3.12 min, 254 (M+1, ES+).
**1-(4-Fluoro-benzyl)-6,7-dimethoxy-1,2,3,4-tetrahydro-isoquinoline:** 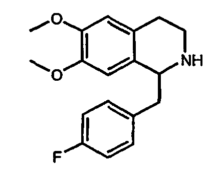 prepared by cyclisation of N-[2-(3,4-dimethoxy-phenyl)-ethyl]-2-(4-fluorophenyl)-acetamide.
   LC-MS: rt = 3.05 min, 302 (M+1, ES+).
**1-(2,5-Dimethoxy-benzyl)-6,7-dimethoxy-1,2,3,4-tetrahydro-isoquinoline:** 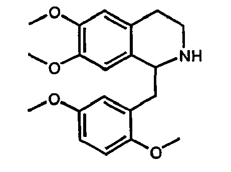 prepared by cyclisation of 2-(2,5-dimethoxy-phenyl)-N-[2-(3,4-dimethoxyphenyl)-ethyl]-acetamide.
   LC-MS: rt = 3.18 min, 344 (M+1, ES+).
**6,7-Dimethoxy-1-(4-methoxy-benzyl)-1,2,3,4-tetrahydro-isoquinoline:** 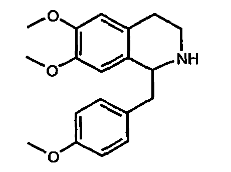 prepared by cyclisation of N-[2-(3,4-dimethoxy-phenyl)-ethyl]-2-(4-methoxyphenyl)-acetamide.
   LC-MS: rt = 3.01 min, 314 (M+1, ES+).
**6,7-Dimethoxy-1-phenoxymethyl-1,2,3,4-tetrahydro-isoquinoline:** 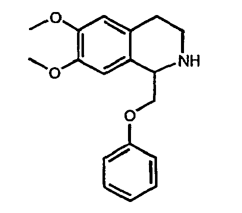 prepared by cyclisation of N-[2-(3,4-dimethoxy-phenyl)-ethyl]-2-phenoxy-acetamide.
   LC-MS: rt = 3.02 min, 300 (M+1, ES+).
**6,7-Dimethoxy-1-(3-methoxy-benzyl)-1,2,3,4-tetrahydro-isoquinoline:** 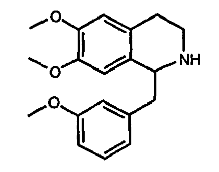 prepared by cyclisation of N-[2-(3,4-dimethoxy-phenyl)-ethyl]-2-(3-methoxyphenyl)-acetamide.
   LC-MS: rt = 3.04 min, 314 (M+1, ES+).
**6-(3,4-Dimethoxy-benzyl)-2,3,6,7,8,9-hexahydro-[1,4]dioxino[2,3-g]isoquinoline:** 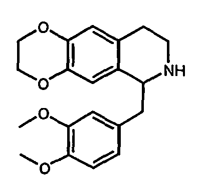 prepared by cyclisation of N-[2-(2,3-dihydro-benzo[1,4]dioxin-6-yl)-ethyl]-2-(3,4-dimethoxy-phenyl)-acetamide.
   LC-MS: rt = 3.07 min, 342 (M+1, ES+).
**6,7-Dimethoxy-1-(3-phenyl-propyl)-1,2,3,4-tetrahydro-isoquinoline:** 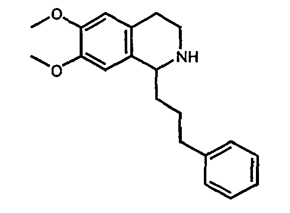 prepared by cyclisation of N-[2-(3,4-dimethoxy-phenyl)-ethyl]-4-phenyl-butyramide.
   LC-MS: rt = 3.15 min, 312 (M+1, ES+).

### C.2 Stereoselective Synthesis of 1,2,3,4-Tetrahydroisoquinoline Derivatives via Transfer Hydrogenation (general procedure):

Dichloro-(*p*-cymene)ruthenium (II) dimer (0.20 mmol) is added to a solution of *N-*((1R,2R)-2-amino-1,2-diphenyl-ethyl)-2,4,6-trimethylbenzene-sulfonamide (0.40 mmol) and triethylamine (0.80 mmol) in acetonitrile (3.0 mL). The mixture is stirred for 1h at 80°C and added to a solution of the respective dihydroisoquinoline (28.0 mmol) in dichloromethane (30 mL). An azeotropic mixture of formic acid and triethylamine (5:2, 14 mL) is added (gas evolution). After 90 min a sat. aqueous NaHCO₃ solution (200 mL) is added to the dark red solution. The layers are separated, the aqueous layer is extracted twice with DCM (2x200 mL) and the combined organic extracts are concentrated in vacuo. The residue is dissolved in isopropanol (1600 mL) and treated with a solution of HCl in isopropanol (5-6 M, 10 mL). The obtained hydrochloride salt is recrystallized to give the respective 1,2,3,4-tetrahydroisoquinoline with high enantiomeric excess as determined by chiral HPLC. The hydrochloride salt is converted to the free base by extraction with sat. NaHCO₃ solution/dichloromethane. The absolute configuration of the respective product is assigned in analogy to the literature (N. Uematsu, A. Fujii, S. Hashiguchi, T. Ikariya, R. Noyori, J. Am. Chem. Soc. 1996, 118, 4916-4917).
**(S)-6,7-Dimethoxy-1-[2-(4-trifluoromethyl-phenyl)-ethyl]-1,2,3,4-tetrahydroisoquinoline:** 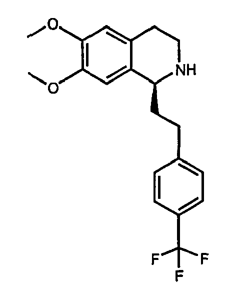 prepared by transfer hydrogenation of 6,7-dimethoxy-1-[2-(4-trifluoromethylphenyl)-ethyl]-3,4-dihydroisoquinoline.
   LC-MS: rt = 0.80 min, 366 (M+1, ES+).
   chiral HPLC: rt = 12.0 min (hexane/ethanol 9/1; enantiomer: rt = 17.1 min).

### C.3 Stereoselective Synthesis of 1,2,3,4-Tetrahydroisoquinoline Derivatives via Alkylation of 1-Methyl-3,4-dihydroisoquinolines (general procedure):

At 0°C a solution of n-BuLi in hexane (1.6M, 0.63 mmol) is added drop wise to a mixture of 6,7-dimethoxy-1-methyl-3,4-dihydroisoquinoline (0.50 mmol) and diisopropylamine (0.63 mmol) in THF (1.0 mL). The reaction mixture is stirred at RT for 1h and added at 0°C to a solution of the respective benzyl bromide (0.50 mmol) in THF (1.0 mL). The solution is stirred for 1h, warmed up to RT and diluted with DCM (3.0 mL).

In a second flask dichloro(*p*-cymene)ruthenium (II) dimer (0.15 mmol) is added to a solution of *N*-((1R,2R)-2-amino-1,2-diphenyl-ethyl)-2,4,6-trimethyl-benzenesulfonamide (0.30 mmol) and triethylamine (0.60 mmol) in acetonitrile (3.3 mL). The mixture is stirred for 1h at 80°C. A portion of this solution (0.10 mL) is added to the solution of the respective dihydroisoquinoline (described above). An azeotropic mixture of formic acid and triethylamine (5:2, 0.3 mL) is added (gas evolution). After 2d the mixture is concentrated in vacuo and purified by prep. HPLC to give the respective 1,2,3,4-tetrahydroisoquinoline.

The absolute configuration of the respective product is assigned in analogy to the literature (N. Uematsu, A. Fujii, S. Hashiguchi, T. Ikariya, R. Noyori, J. Am. Chem. Soc 1996, 118, 4916-4917).
**(S)-6,7-Dimethoxy-1-[2-(2-methyl-5-trifluoromethyl-phenyl)-ethyl]-1,2,3,4-tetrahydro-isoquinoline:** 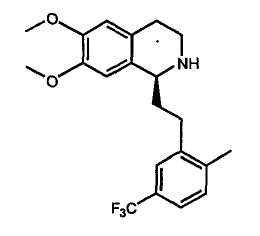 prepared from 6,7-dimethoxy-1-methyl-3,4-dihydro-isoquinoline and 2-chloromethyl-1-methyl-4-trifluoromethyl-benzene.
   LC-MS: rt = 0.84 min, 380 (M+1, ES+).
**(S)-1-[2-(3-Chloro-2-fluoro-phenyl)-ethyl]-6,7-dimethoxy-1,2,3,4-tetrahydroisoquinoline:** 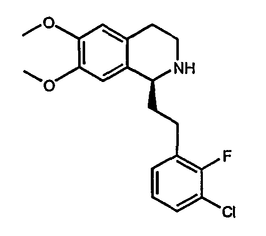 prepared from 6,7-dimethoxy-1-methyl-3,4-dihydro-isoquinoline and 1-bromomethyl-3-chloro-2-fluoro-benzene.
   LC-MS: rt = 0.80 min, 350 (M+1, ES+).
**(S)-1-[2-(3-Fluoro-4-methyl-phenyl)-ethyl]-6,7-dimethoxy-1,2,3,4-tetrahydroisoquinoline:** 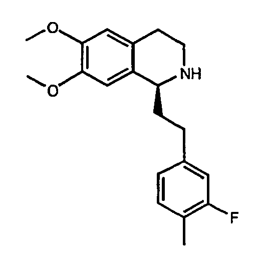 prepared from 6,7-dimethoxy-1-methyl-3,4-dihydro-isoquinoline and 4-bromomethyl-2-fluoro-1-methyl-benzene.
   LC-MS: rt = 0.80 min, 330 (M+1, ES+).
**(S)-1-[2-(4-Fluoro-2-trifluoromethyl-phenyl)-ethyl]-6,7-dimethoxy-1,2,3,4-tetrahydro-isoquinoline:** 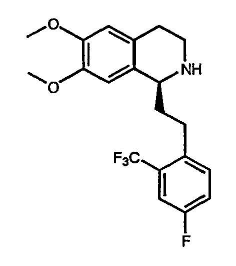 prepared from 6,7-dimethoxy-1-methyl-3,4-dihydro-isoquinoline and 1-bromomethyl-4-fluoro-2-trifluoromethyl-benzene.
   LC-MS: rt = 0.82 min, 384 (M+1, ES+).
**(S)-1-[2-(5-Fluoro-2-trifluoromethyl-phenyl)-ethyl]-6,7-dimethoxy-1,2,3,4-tetrahydro-isoquinoline:** 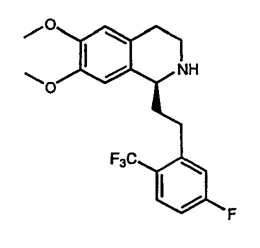 prepared from 6,7-dimethoxy-1-methyl-3,4-dihydro-isoquinoline and 2-bromomethyl-4-fluoro-1-trifluoromethyl-benzene.
   LC-MS: rt = 0.81 min, 384 (M+1, ES+).
**(S)-1-[2-(2-Fluoro-5-trifluoromethyl-phenyl)-ethyl]-6,7-dimethoxy-1,2,3,4-tetrahydro-isoquinoline:** 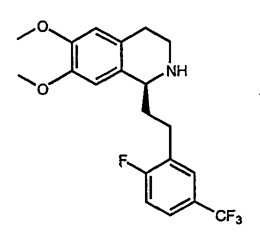 prepared from 6,7-dimethoxy-1-methyl-3,4-dihydro-isoquinoline and 2-bromomethyl-1-fluoro-4-trifluoromethyl-benzene.
   LC-MS: rt = 0.82 min, 384 (M+1, ES+).
**(S)-1-[2-(3-Fluoro-4-trifluoromethyl-phenyl)-ethyl]-6,7-dimethoxy-1,2,3,4-tetrahydro-isoquinoline:** 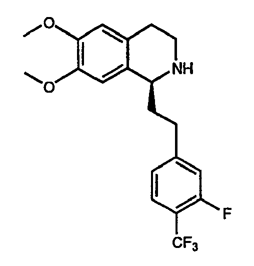 prepared from 6,7-dimethoxy-1-methyl-3,4-dihydro-isoquinoline and 4-bromomethyl-2-fluoro-1-trifluoromethyl-benzene.
   LC-MS: rt = 0.83 min, 384 (M+1, ES+).
**(S)-1-[2-(4-Fluoro-3-methyl-phenyl)-ethyl]-6,7-dimethoxy-1,2,3,4-tetrahydroisoquinoline:** 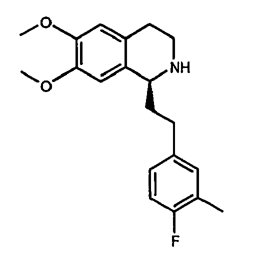 prepared from 6,7-dimethoxy-1-methyl-3,4-dihydro-isoquinoline and 4-bromomethyl-1-fluoro-2-methyl-benzene.
   LC-MS: rt = 0.80 min, 330 (M+1, ES+).
**(S)-6,7-Dimethoxy-1-[2-(2,3,5-trifluoro-phenyl)-ethyl]-1,2,3,4-tetrahydroisoquinoline:** 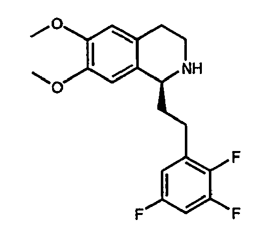 prepared from 6,7-dimethoxy-1-methyl-3,4-dihydro-isoquinoline and 1-bromomethyl-2,3,5-trifluoro-benzene.
   LC-MS: rt = 0.78 min, 352 (M+1, ES+).
**(S)-1-[2-(4-Fluoro-3-trifluoromethyl-phenyl)-ethyl]-6,7-dimethoxy-1,2,3,4-tetrahydro-isoquinoline:** 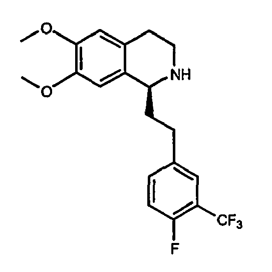 prepared from 6,7-dimethoxy-1-methyl-3,4-dihydro-isoquinoline and 4-bromomethyl-1-fluoro-2-trifluoromethyl-benzene.
   LC-MS: rt = 0.83 min, 384 (M+1, ES+).
**(S)-1-[2-(3-Fluoro-5-trifluoromethyl-phenyl)-ethyl]-6,7-dimethoxy-1,2,3,4-tetrahydro-isoquinoline:** 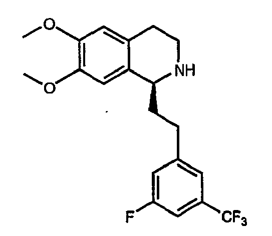 prepared from 6,7-dimethoxy-1-methyl-3,4-dihydro-isoquinoline and 1-bromomethyl-3-fluoro-5-trifluoromethyl-benzene.
   LC-MS: rt = 0.83 min, 384 (M+1, ES+).
**(S)-1-[2-(5-Chloro-2-trifluoromethyl-phenyl)-ethyl]-6,7-dimethoxy-1,2,3,4-tetrahydro-isoquinoline:** 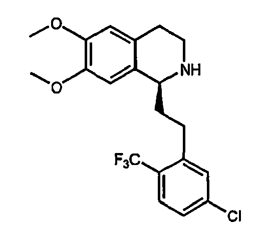 prepared from 6,7-dimethoxy-1-methyl-3,4-dihydro-isoquinoline and 2-bromomethyl-4-chloro-1-trifluoromethyl-benzene.
   LC-MS: rt = 0.84 min, 400 (M+1, ES+).
**(S)-1-[2-(2-Fluoro-3-trifluoromethyl-phenyl)-ethyl]-6,7-dimethoxy-1,2,3,4-tetrahydro-isoquinoline:** 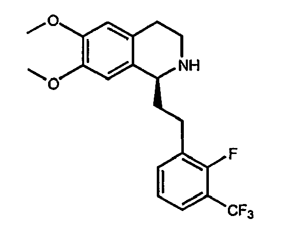 prepared from 6,7-dimethoxy-1-methyl-3,4-dihydro-isoquinoline and 1-bromomethyl-2-fluoro-3-trifluoromethyl-benzene.
   LC-MS: rt = 0.82 min, 384 (M+1, ES+).
**(S)-1-[2-(2-Fluoro-4-trifluoromethyl-phenyl)-ethyl]-6,7-dimethoxy-1,2,3,4-tetrahydro-isoquinoline:** 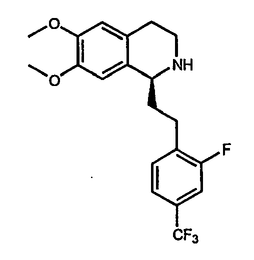 prepared from 6,7-dimethoxy-1-methyl-3,4-dihydro-isoquinoline and 1-bromomethyl-2-fluoro-4-trifluoromethyl-benzene.
   LC-MS: rt = 0.83 min, 384 (M+1, ES+).
**(S)-1-[2-(2-Difluoromethoxy-phenyl)-ethyl]-6,7-dimethoxy-1,2,3,4-tetrahydroisoquinoline:** 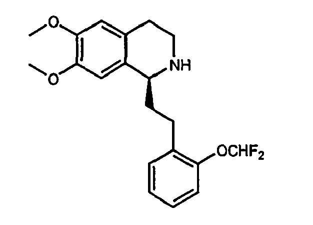 prepared from 6,7-dimethoxy-1-methyl-3,4-dihydro-isoquinoline and 1-bromomethyl-2-difluoromethoxy-benzene.
   LC-MS: rt = 0.78 min, 364 (M+1, ES+).
**(S)-1-[2-(3-Fluoro-2-trifluoromethyl-phenyl)-ethyl]-6,7-dimethoxy-1,2,3,4-tetrahydro-isoquinoline:** 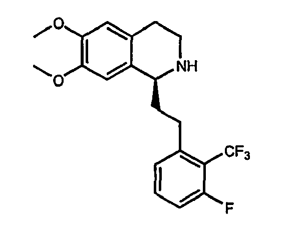 prepared from 6,7-dimethoxy-1-methyl-3,4-dihydro-isoquinoline and 1-bromomethyl-3-fluoro-2-trifluoromethyl-benzene.
   LC-MS: rt = 0.81 min, 384 (M+1, ES+).
**(S)-1-[2-(2-Chloro-3-trifluoromethyl-phenyl)-ethyl]-6,7-dimethoxy-1,2,3,4-tetrahydro-isoquinoline:** 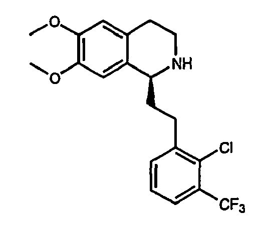 prepared from 6,7-dimethoxy-1-methyl-3,4-dihydro-isoquinoline and 1-bromomethyl-2-chloro-3-trifluoromethyl-benzene.
   LC-MS: rt = 0.84 min, 400 (M+1, ES+).
**(S)-1-[2-(2-Fluoro-6-trifluoromethyl-phenyl)-ethyl]-6,7-dimethoxy-1,2,3,4-tetrahydro-isoquinoline:** 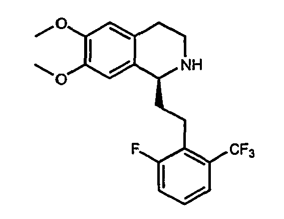 prepared from 6,7-dimethoxy-1-methyl-3,4-dihydro-isoquinoline and 2-bromomethyl-1-fluoro-3-trifluoromethyl-benzene.
   LC-MS: rt = 0.79 min, 384 (M+1, ES+).
**(S)-1-[2-(4-Chloro-3-trifluoromethyl-phenyl)-ethyl]-6,7-dimethoxy-1,2,3,4-tetrahydro-isoquinoline:** 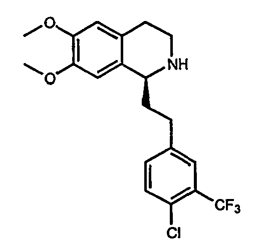 prepared from 6,7-dimethoxy-1-methyl-3,4-dihydro-isoquinoline and 4-bromomethyl-1-chloro-2-trifluoromethyl-benzene.
   LC-MS: rt = 0.85 min, 400 (M+1, ES+).
**(S)-1-[2-(2,3-Difluoro-4-methyl-phenyl)-ethyl]-6,7-dimethoxy-1,2,3,4-**tetrahydro-isoquinoline: 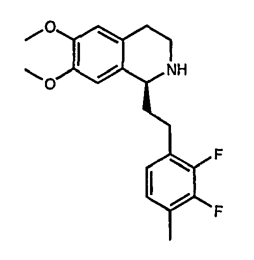 prepared from 6,7-dimethoxy-1-methyl-3,4-dihydro-isoquinoline and 1-bromomethyl-2,3-difluoro-4-methyl-benzene.
   LC-MS: rt = 0.80 min, 348 (M+1, ES+).
**(S)-1-[2-(4-Difluoromethoxy-phenyl)-ethyl]-6,7-dimethoxy-1,2,3,4-tetrahydroisoquinoline:** 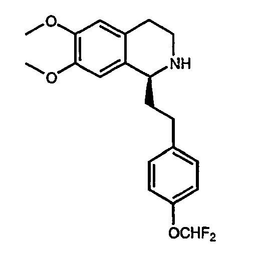 prepared from 6,7-dimethoxy-1-methyl-3,4-dihydro-isoquinoline and 1-bromomethyl-4-difluoromethoxy-benzene.
   LC-MS: rt = 0.79 min, 364 (M+1, ES+).
**(S)-1-[2-(3,4-Dimethyl-phenyl)-ethyl]-6,7-dimethoxy-1,2,3,4-tetrahydroisoquinoline:** 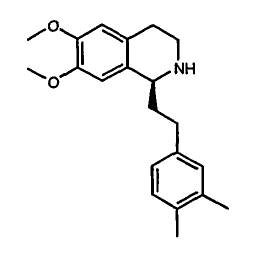 prepared from 6,7-dimethoxy-1-methyl-3,4-dihydro-isoquinoline and 4-chloromethyl-1,2-dimethyl-benzene.
   LC-MS: rt = 0.79 min, 326 (M+1, ES+).
**(S)-1-[2-(3-Chloro-4-methyl-phenyl)-ethyl]-6,7-dimethoxy-1,2,3,4-tetrahydroisoquinoline:** 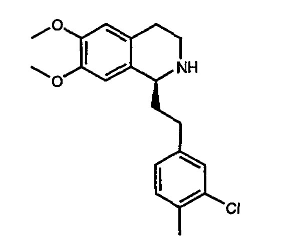 prepared from 6,7-dimethoxy-1-methyl-3,4-dihydro-isoquinoline and 2-chloro-4-chloromethyl-1-methyl-benzene.
   LC-MS: rt = 0.79 min, 346 (M+1, ES+).
**(S)-1-[2-(2-Chloro-6-fluoro-phenyl)-ethyl]-6,7-dimethoxy-1,2,3,4-tetrahydroisoquinoline:** 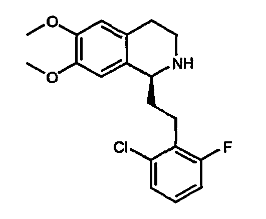 prepared from 6,7-dimethoxy-1-methyl-3,4-dihydro-isoquinoline and 1-chloro-2-chloromethyl-3-fluoro-benzene.
   LC-MS: rt = 0.76 min, 350 (M+1, ES+).
**(S)-6,7-Dimethoxy-1-(2-o-tolyl-ethyl)-1,2,3,4-tetrahydro-isoquinoline:** 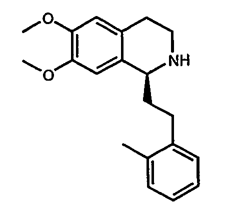 prepared from 6,7-dimethoxy-1-methyl-3,4-dihydro-isoquinoline and 1-bromomethyl-2-methyl-benzene.
   LC-MS: rt = 0.77 min, 312 (M+1, ES+).
**(S)-6,7-Dimethoxy-1-(2-m-tolyl-ethyl)-1,2,3,4-tetrahydro-isoquinoline:** 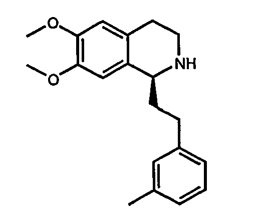 prepared from 6,7-dimethoxy-1-methyl-3,4-dihydro-isoquinoline and 1-bromomethyl-3-methyl-benzene.
   LC-MS: rt = 0.78 min, 312 (M+1, ES+).
**(S)-6,7-Dimethoxy-1-(2-p-tolyl-ethyl)-1,2,3,4-tetrahydro-isoquinoline:** 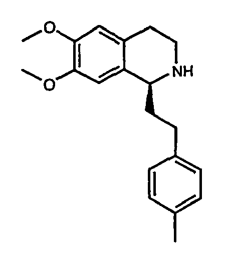 prepared from 6,7-dimethoxy-1-methyl-3,4-dihydro-isoquinoline and 1-bromomethyl-4-methyl-benzene.
   LC-MS: rt = 0.78 min, 312 (M+1, ES+).
**(S)-1-[2-(2-Fluoro-phenyl)-ethyl]-6,7-dimethoxy-1,2,3,4-tetrahydroisoquinoline:** 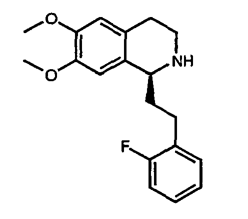 prepared from 6,7-dimethoxy-1-methyl-3,4-dihydro-isoquinoline and 1-bromomethyl-2-fluoro-benzene.
   LC-MS: rt = 0.74 min, 316 (M+1, ES+).
**(S)-1-[2-(3-Fluoro-phenyl)-ethyl]-6,7-dimethoxy-1,2,3,4-tetrahydroisoquinoline:** 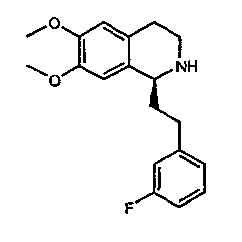 prepared from 6,7-dimethoxy-1-methyl-3,4-dihydro-isoquinoline and 1-bromomethyl-3-fluoro-benzene.
   LC-MS: rt = 0.75 min, 316 (M+1, ES+).
**(S)-1-[2-(2,6-Dichloro-phenyl)-ethyl]-6,7-dimethoxy-1,2,3,4-tetrahydroisoquinoline:** 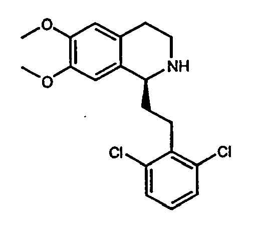 prepared from 6,7-dimethoxy-1-methyl-3,4-dihydro-isoquinoline and 1,3-dichloro-2-chloromethyl-benzene.
   LC-MS: rt = 0.79 min, 366 (M+1, ES+).
**(S)-1-[2-(3,4-Dichloro-phenyl)-ethyl]-6,7-dimethoxy-1,2,3,4-tetrahydroisoquinoline:** 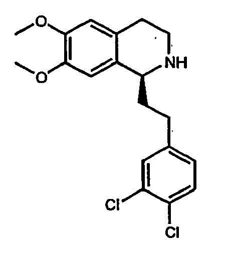 prepared from 6,7-dimethoxy-1-methyl-3,4-dihydro-isoquinoline and 1,2-dichloro-4-chloromethyl-benzene.
   LC-MS: rt = 1.19 min, 366 (M+1, ES+).
**(S)-1-[2-(3,5-Dimethyl-phenyl)-ethyl]-6,7-dimethoxy-1,2,3,4-tetrahydroisoquinoline:** 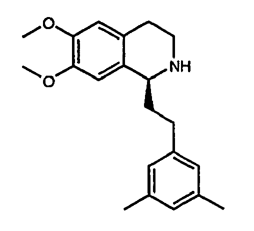 prepared from 6,7-dimethoxy-1-methyl-3,4-dihydro-isoquinoline and 1-bromomethyl-3,5-dimethyl-benzene.
   LC-MS: rt = 1.15 min, 326 (M+1, ES+).
**(S)-6,7-Dimethoxy-1-[2-(2-trifluoromethyl-phenyl)-ethyl]-1,2,3,4-tetrahydroisoquinoline:** 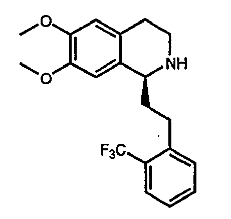 prepared from 6,7-dimethoxy-1-methyl-3,4-dihydro-isoquinoline and 1-bromomethyl-2-trifluoromethyl-benzene.
   LC-MS: rt =1.11 min, 366 (M+1, ES+).
**(S)-1-[2-(2,4-Difluoro-phenyl)-ethyl]-6,7-dimethoxy-1,2,3,4-tetrahydroisoquinoline:** 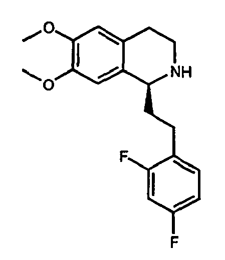 prepared from 6,7-dimethoxy-1-methyl-3,4-dihydro-isoquinoline and 1-bromomethyl-2,4-difluoro-benzene.
   LC-MS: rt = 0.85 min, 334 (M+1, ES+).
**(S)-6,7-Dimethoxy-1-[2-(2,3,6-trifluoro-phenyl)-ethyl]-1,2,3,4-tetrahydroisoquinoline:** 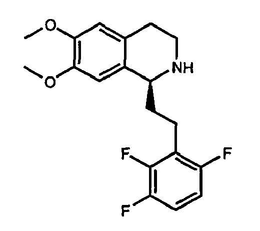 prepared from 6,7-dimethoxy-1-methyl-3,4-dihydro-isoquinoline and 2-bromomethyl-1,3,4-trifluoro-benzene.
   LC-MS: rt = 0.84 min, 352 (M+1, ES+).
**(S)-6,7-Dimethoxy-1-[2-(4-trifluoromethoxy-phenyl)-ethyl]-1,2,3,4-tetrahydroisoquinoline:** 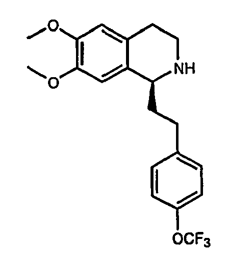 prepared from 6,7-dimethoxy-1-methyl-3,4-dihydro-isoquinoline and 1-bromomethyl-4-trifluoromethoxy-benzene.
   LC-MS: rt = 0.93 min, 382 (M+1, ES+).
**(S)-1-[2-(2-Fluoro-3-methyl-phenyl)-ethyl]-6,7-dimethoxy-1,2,3,4-tetrahydroisoquinoline:** 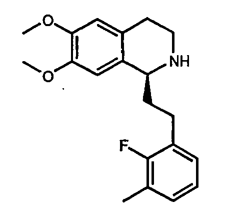 prepared from 6,7-dimethoxy-1-methyl-3,4-dihydro-isoquinoline and 1-bromomethyl-2-fluoro-3-methyl-benzene.
   LC-MS: rt = 0.86 min, 330 (M+1, ES+).
**(S)-1-[2-(4-Chloro-phenyl)-ethyl]-6,7-dimethoxy-1,2,3,4-tetrahydroisoquinoline:** 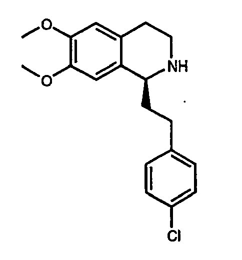 prepared from 6,7-dimethoxy-1-methyl-3,4-dihydro-isoquinoline and 1-bromomethyl-4-chloro-benzene.
   LC-MS: rt = 0.88 min, 332 (M+1, ES+).
**(S)-1-[2-(3-Chloro-phenyl)-ethyl]-6,7-dimethoxy-1,2,3,4-tetrahydroisoquinoline:** 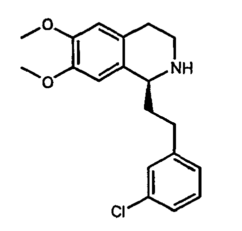 prepared from 6,7-dimethoxy-1-methyl-3,4-dihydro-isoquinoline and 1-bromomethyl-3-chloro-benzene.
   LC-MS: rt = 0.88 min, 332 (M+1, ES+).
**(S)-6,7-Dimethoxy-1-[2-(3-trifluoromethoxy-phenyl)-ethyl]-1,2,3,4-tetrahydroisoquinoline:** 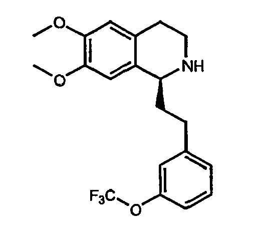 prepared from 6,7-dimethoxy-1-methyl-3,4-dihydro-isoquinoline and 1-bromomethyl-3-trifluoromethoxy-benzene.
   LC-MS: rt = 0.92 min, 382 (M+1, ES+).
**(S)-6,7-Dimethoxy-1-[2-(3,4,5-trifluoro-phenyl)-ethyl]-1,2,3,4-tetrahydroisoquinoline:** 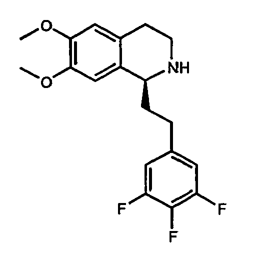 prepared from 6,7-dimethoxy-1-methyl-3,4-dihydro-isoquinoline and 5-bromomethyl-1,2,3-trifluoro-benzene.
   LC-MS: rt = 0.88 min, 352 (M+1, ES+).
**(S)-6,7-Dimethoxy-1-[2-(3-trifluoromethyl-phenyl)-ethyl]-1,2,3,4-tetrahydroisoquinoline:** 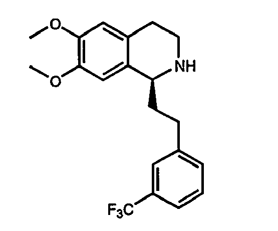 prepared from 6,7-dimethoxy-1-methyl-3,4-dihydro-isoquinoline and 1-bromomethyl-3-trifluoromethyl-benzene.
   LC-MS: rt = 0.88 min, 366 (M+1, ES+).
**(S)-6,7-Dimethoxy-1-[2-(2,3,4-trifluoro-phenyl)-ethyl]-1,2,3,4-tetrahydroisoquinoline:** 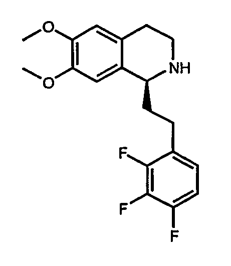 prepared from 6,7-dimethoxy-1-methyl-3,4-dihydro-isoquinoline and 1-bromomethyl-2,3,4-trifluoro-benzene.
   LC-MS: rt = 0.86 min, 352 (M+1, ES+).
**(S)-1-[2-(4-Bromo-2-fluoro-phenyl)-ethyl]-6,7-dimethoxy-1,2,3,4-tetrahydroisoquinoline:** 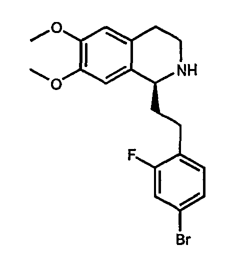 prepared from 6,7-dimethoxy-1-methyl-3,4-dihydro-isoquinoline and 4-promo-1-bromomethyl-2-fluoro-benzene.
   LC-MS: rt = 0.90 min, 394 (M+1, ES+).
**(S)-1-[2-(2,6-Difluoro-phenyl)-ethyl]-6,7-dimethoxy-1,2,3,4-tetrahydroisoquinoline:** 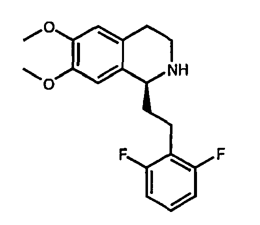 prepared from 6,7-dimethoxy-1-methyl-3,4-dihydro-isoquinoline and 2-bromomethyl-1,3-difluoro-benzene.
   LC-MS: rt = 0.82 min, 334 (M+1, ES+).
**(S)-6,7-Dimethoxy-1-[2-(2-trifluoromethoxy-phenyl)-ethyl]-1,2,3,4-tetrahydroisoquinoline:** 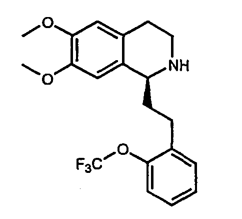 prepared from 6,7-dimethoxy-1-methyl-3,4-dihydro-isoquinoline and 1-bromomethyl-2-trifluoromethoxy-benzene.
   LC-MS: rt = 0.90 min, 382 (M+1, ES+).
**(S)-1-[2-(2,4-Dichloro-phenyl)-ethyl]-6,7-dimethoxy-1,2,3,4-tetrahydroisoquinoline:** 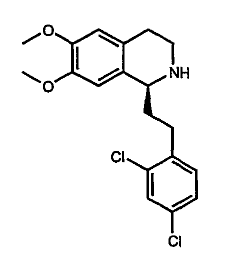 prepared from 6,7-dimethoxy-1-methyl-3,4-dihydro-isoquinoline and 2,4-dichloro-1-chloromethyl-benzene.
   LC-MS: rt = 0.92 min, 366 (M+1, ES+).
**(S)-6,7-Dimethoxy-1-[2-(2,4,5-trifluoro-phenyl)-ethyl]-1,2,3,4-tetrahydroisoquinoline:** 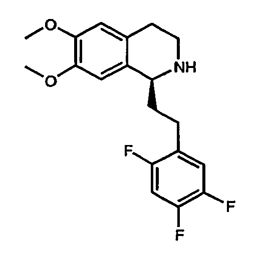 prepared from 6,7-dimethoxy-1-methyl-3,4-dihydro-isoquinoline and 1-bromomethyl-2,4,5-trifluoro-benzene.
   LC-MS: rt = 0.86 min, 352 (M+1, ES+).
**(S)-1-[2-(3-Bromo-phenyl)-ethyl]-6,7-dimethoxy-1,2,3,4-tetrahydroisoquinoline:** 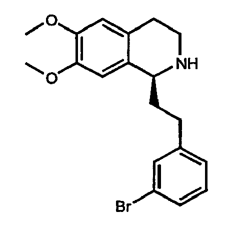 prepared from 6,7-dimethoxy-1-methyl-3,4-dihydro-isoquinoline and 1-bromo-3-bromomethyl-benzene.
   LC-MS: rt = 0.89 min, 376 (M+1, ES+).
**(S)-1-[2-(4-tert-Butyl-phenyl)-ethyl]-6,7-dimethoxy-1,2,3,4-tetrahydroisoquinoline:** 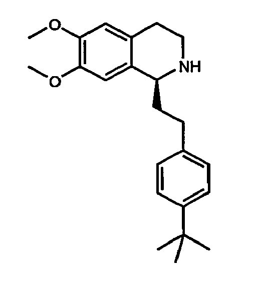 prepared from 6,7-dimethoxy-1-methyl-3,4-dihydro-isoquinoline and 1-bromomethyl-4-tert-butyl-benzene.
   LC-MS: rt = 0.98 min, 354 (M+1, ES+).
**(S)-1-[2-(2-Bromo-phenyl)-ethyl]-6,7-dimethoxy-1,2,3,4-tetrahydroisoquinoline:** 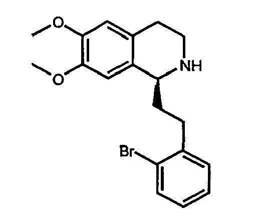 prepared from 6,7-dimethoxy-1-methyl-3,4-dihydro-isoquinoline and 1-bromo-2-bromomethyl-benzene.
   LC-MS: rt = 0.87 min, 376 (M+1, ES+).
**(S)-1-[2-(4-Bromo-phenyl)-ethyl]-6,7-dimethoxy-1,2,3,4-tetrahydroisoquinoline:** 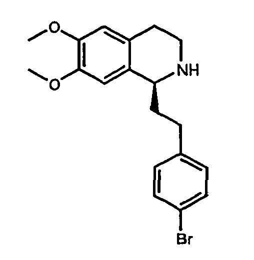 prepared from 6,7-dimethoxy-1-methyl-3,4-dihydro-isoquinoline and 1-bromo-4-bromomethyl-benzene.
   LC-MS: rt = 0.89 min, 376 (M+1, ES+).

### D Synthesis of (3,4-Dihydro-1H-isoquinolin-2-yl)-acetic acid derivatives:

### D.1 Synthesis of acetic acid methyl ester derivatives (general procedure):

Potassium *tert*-butoxide (5.0 mmol) is added to a suspension of the respective tetrahydroisoquinoline hydrochloride (5.0 mmol) in THF (50 mL). After 5 min DIPEA (10.0 mmol) and the respective methyl bromoacetate (5.0 mmol) are added. The reaction mixture is stirred at 60°C for 14 h. Water (80 mL) and ethyl acetate (150 mL) are added, the layers are separated and the aqueous layer is extracted three times with ethyl acetate (50 mL each). The combined organic extracts are dried with Na₂SO₄ and the solvent is removed in vacuo. The following esters that are used in the next step without further purification are obtained as mixtures of racemic diastereoisomers:
**(6,7-Dimethoxy-1-naphthalen-2-ylmethyl-3,4-dihydro-1H-isoquinolin-2-yl)-phenyl-acetic acid methyl ester:** 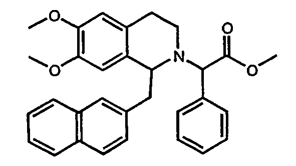 prepared by reaction of 6,7-dimethoxy-1-naphthalen-2-ylmethyl-1,2,3,4-tetrahydroisoquinoline with methyl α-bromophenylacetate.
   LC-MS: rt = 0.89 min (dia 1), 0.92 min (dia 2), 482 (M+1, ES+).
**{1-[2-(3,4-Difluoro-phenyl)-ethyl]-6,7-dimethoxy-3,4-dihydro-1H-isoquinolin-2-yl}-phenyl-acetic acid methyl ester:** 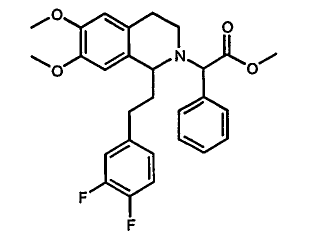 prepared by reaction of 1-[2-(3,4-difluoro-phenyl)-ethyl]-6,7-dimethoxy-1,2,3,4-tetrahydroisoquinoline with methyl α-bromophenylacetate.
   LC-MS: rt = 0.89 min, 482 (M+1, ES+).
**[1-(2-Furan-2-yl-ethyl)-6,7-dimethoxy-3,4-dihydro-1H-isoquinolin-2-yl]-phenyl-acetic acid methyl ester:** 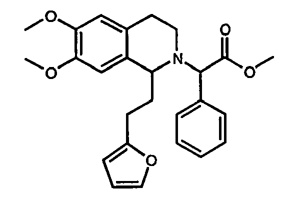 prepared by reaction of 1-(2-furan-2-yl-ethyl)-6,7-dimethoxy-1,2,3,4-tetrahydroisoquinoline with methyl α-bromophenylacetate.
   LC-MS: rt = 4.53 min, 436 (M+1, ES+).
**[6,7-Dimethoxy-1-(3-phenyl-propyl)-3,4-dihydro-1H-isoquinolin-2-yl]-phenylacetic acid methyl ester:** 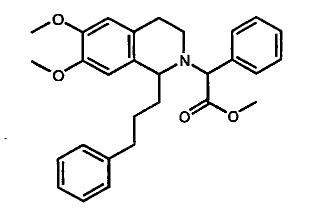 prepared by reaction of 6,7-dimethoxy-1-(3-phenyl-propyl)-1,2,3,4-tetrahydroisoquinoline with methyl α-bromophenylacetate.
   LC-MS: dia1: rt = 5.04, 460 (M+1, ES+); dia2: rt = 5.44, 460 (M+1, ES+).

### D.2 Synthesis of acetic acid derivatives (general procedure):

A solution of the respective ester (5.0 mmol) in MeOH (200 mL) is treated with sodium hydroxide solution (1.0 M, 30 mL), stirred at 60°C for 16 h and concentrated in vacuo to a volume of about 40 mL. Sodium hydroxide solution (1.0 M, 50 mL) and ethyl acetate (100 mL) are added, the layers are separated and the aqueous layer is extracted three times with ethyl acetate (100 mL each). The combined organic extracts are concentrated in vacuo. The residue is purified either by crystallization from ethyl acetate or by prep. HPLC to give the following acetic acid derivatives as mixtures of racemic diastereoisomers:
**(6,7-Dimethoxy-1-naphthalen-2-ylmethyl-3,4-dihydro-1H-isoquinolin-2-yl)-phenyl-acetic acid :** 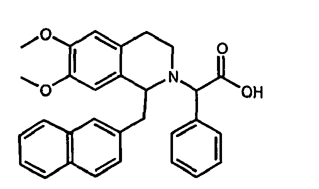 prepared by saponification of (6,7-dimethoxy-1-naphthalen-2-ylmethyl-3,4-dihydro-1H-isoquinolin-2-yl)-phenyl-acetic acid methyl ester.
   LC-MS: rt = 0.83 min (dia 1), 0.85 min (dia 2), 468 (M+1, ES+).
**{1-[2-(3,4-Difluoro-phenyl)-ethyl]-6,7-dimethoxy-3,4-dihydro-1H-isoquinolin-2-yl}-phenyl-acetic acid:** 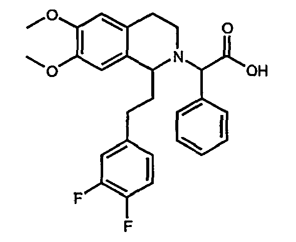 prepared by saponification of {1-[2-(3,4-difluoro-phenyl)-ethyl]-6,7-dimethoxy-3,4-dihydro-1H-isoquinolin-2-yl}-phenyl-acetic acid methyl ester.
   LC-MS: rt = 0.83 min, 468 (M+1, ES+).
**[1-(2-Furan-2-yl-ethyl)-6,7-dimethoxy-3,4-dihydro-1H-isoquinolin-2-yl]-phenyl-acetic acid:** 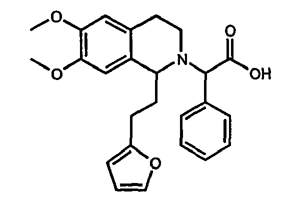 prepared by saponification of [1-(2-furan-2-yl-ethyl)-6,7-dimethoxy-3,4-dihydro-1H-isoquinolin-2-yl]-phenyl-acetic acid methyl ester.
   LC-MS: rt = 3.68 min, 422 (M+1, ES+).
**[6,7-Dimethoxy-1-(3-phenyl-propyl)-3,4-dihydro-1H-isoquinolin-2-yl]-phenylacetic acid:** 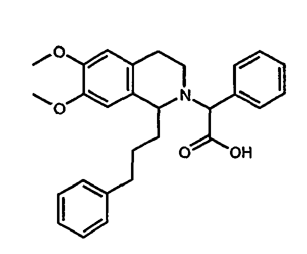 prepared by saponification of [6,7-dimethoxy-1-(3-phenyl-propyl)-3,4-dihydro-1H-isoquinolin-2-yl]-phenyl-acetic acid methyl ester.
   LC-MS: rt = 4.14, 446 (M+1, ES+).

### D.3 One-pot Synthesis of acetic acid derivatives (general procedure):

A solution of potassium *tert*-butoxide (0.30 mmol) in THF (0.50 mL) is added to the respective tetrahydroisoquinoline hydrochloride (0.30 mmol). After 5 min DIPEA (0.60 mmol) and a solution of the respective methyl bromoacetate (0.30 mmol) in THF (0.50 mL) are added. The reaction mixture is stirred at 60°C for 14 h. An aqueous solution of sodium hydroxide (3.0 mmol, 1.5 mL) and ethanol (0.5 mL) are added and the mixture is shaken vigorously at 60°C over night. The layers are separated, the solvent is removed in vacuo and the residue is purified by prep. HPLC to give the following acetic acid derivatives as mixtures of racemic diastereoisomers:
**(1-Benzyl-6-methoxy-3,4-dihydro-1H-isoquinolin-2-yl)-phenyl-acetic acid:** 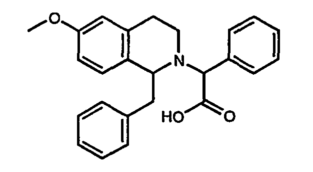 prepared by reaction of 1-benzyl-6-methoxy-1,2,3,4-tetrahydro-isoquinoline with methyl α-bromophenylacetate.
   LC-MS: rt = 0.81 min (dia 1), 0.82 min (dia 2), 388 (M+1, ES+).
**{1-[2-(2,3-Difluoro-phenyl)-ethyl]-6,7-dimethoxy-3,4-dihydro-1H-isoquinolin-2-yl}-phenyl-acetic acid:** 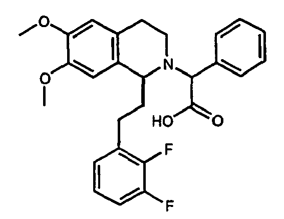 prepared by reaction of 1-[2-(2,3-difluoro-phenyl)-ethyl]-6,7-dimethoxy-1,2,3,4-tetrahydro-isoquinoline with methyl α-bromophenylacetate.
   LC-MS: rt = 0.83 min, 468 (M+1, ES+).
**[1-(4-Fluoro-benzyl)-6,7-dimethoxy-3,4-dihydro-1H-isoquinolin-2-yl]-phenyl**acetic acid: 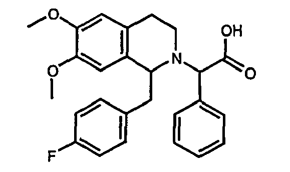 prepared by reaction of 1-(4-fluoro-benzyl)-6,7-dimethoxy-1,2,3,4-tetrahydroisoquinoline with methyl α-bromophenylacetate.
   LC-MS: rt = 0.78 min (dia 1), 0.79 min (dia 2), 436 (M+1, ES+).
**[1-(2,5-Dimethoxy-benzyl)-6,7-dimethoxy-3,4-dihydro-1H-isoquinolin-2-yl]-phenyl-acetic acid:** 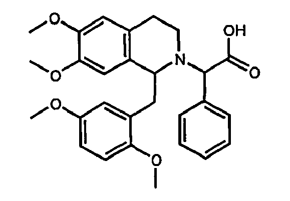 prepared by reaction of 1-(2,5-dimethoxy-benzyl)-6,7-dimethoxy-1,2,3,4-tetrahydro-isoquinoline with methyl α-bromophenylacetate.
   LC-MS: rt = 0.78 min (dia 1), 0.82 min (dia 2), 478 (M+1, ES+).
**{6,7-Dimethoxy-1-[2-(4-trifluoromethyl-phenyl)-ethyl]-3,4-dihydro-1H-isoquinolin-2-yl}-phenyl-acetic acid:** 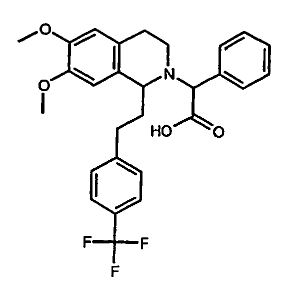 prepared by reaction of 6,7-dimethoxy-1-[2-(4-trifluoromethyl-phenyl)-ethyl]-1,2,3,4-tetrahydro-isoquinoline with methyl α-bromophenylacetate.
   LC-MS: rt = 0.88 min, 500 (M+1, ES+).
**{6,7-Dimethoxy-1-[2-(3-methoxy-phenyl)-ethyl]-3,4-dihydro-1H-isoquinolin-2-yl}-phenyl-acetic acid:** 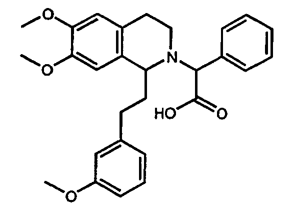 prepared by reaction of 6,7-dimethoxy-1-[2-(3-methoxy-phenyl)-ethyl]-1,2,3,4-tetrahydro-isoquinoline with methyl α-bromophenylacetate.
   LC-MS: rt = 0.81 min, 462 (M+1, ES+).
**[6,7-Dimethoxy-1-(4-methoxy-benzyl)-3,4-dihydro-1H-isoquinolin-2-yl]-phenyl-acetic acid:** 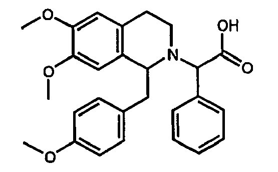 prepared by reaction of 6,7-dimethoxy-1-(4-methoxy-benzyl)-1,2,3,4-tetrahydroisoquinoline with methyl α-bromophenylacetate.
   LC-MS: rt = 0.77 min (dia 1), 0.79 min (dia 2), 448 (M+1, ES+).
**{1-[2-(2,5-Difluoro-phenyl)-ethyl]-6,7-dimethoxy-3,4-dihydro-1H-isoquinolin-2-yl}-phenyl-acetic acid:** 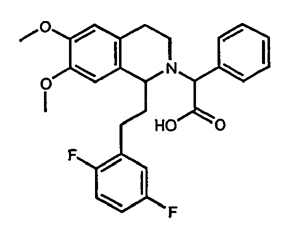 prepared by reaction of 1-[2-(2,5-difluoro-phenyl)-ethyl]-6,7-dimethoxy-1,2,3,4-tetrahydro-isoquinoline with methyl α-bromophenylacetate.
   LC-MS: rt = 0.83 min, 468 (M+1, ES+).
**{6,7-Dimethoxy-1-[2-(2-methoxy-phenyl)-ethyl]-3,4-dihydro-1H-isoquinolin-2-yl}-phenyl-acetic acid:** 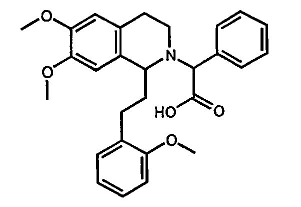 prepared by reaction of 6,7-dimethoxy-1-[2-(2-methoxy-phenyl)-ethyl]-1,2,3,4-tetrahydro-isoquinoline with methyl α-bromophenylacetate.
   LC-MS: rt = 0.83 min, 462 (M+1, ES+).
**{1-[2-(4-Fluoro-phenyl)-ethyl]-6,7-dimethoxy-3,4-dihydro-1H-isoquinolin-2-yl}-phenyl-acetic acid:** 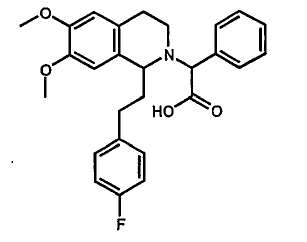 prepared by reaction of 1-[2-(4-fluoro-phenyl)-ethyl]-6,7-dimethoxy-1,2,3,4-tetrahydro-isoquinoline with methyl α-bromophenylacetate.
   LC-MS: rt = 0.82 min, 450 (M+1, ES+).
**{1-[2-(2,3-Difluoro-phenyl)-vinyl]-6,7-dimethoxy-3,4-dihydro-1H-isoquinolin-2-yl}-phenyl-acetic acid:** 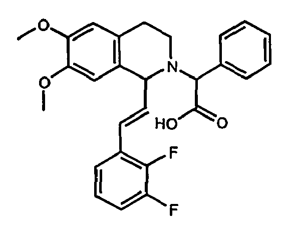 prepared by reaction of 1-[2-(2,3-difluoro-phenyl)-vinyl]-6,7-dimethoxy-1,2,3,4-tetrahydro-isoquinoline with methyl α-bromophenylacetate.
   LC-MS: rt = 0.83 min, 466 (M+1, ES+).
**[1-(3-Fluoro-4-methoxy-benzyl)-6,7-dimethoxy-3,4-dihydro-1H-isoquinolin-2-yl]-phenyl-acetic acid:** 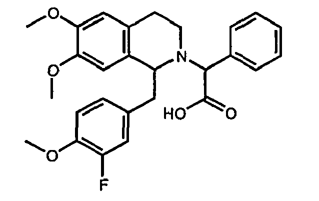 prepared by reaction of 1-(3-fluoro-4-methoxy-benzyl)-6,7-dimethoxy-1,2,3,4-tetrahydro-isoquinoline with methyl α-bromophenylacetate.
   LC-MS: rt = 0.77 min (dia 1), 0.79 min (dia 2), 466 (M+1, ES+).
**{1-[2-(3,4-Dimethoxy-phenyl)-ethyl]-6,7-dimethoxy-3,4-dihydro-1H-isoquinolin-2-yl}-phenyl-acetic acid:** 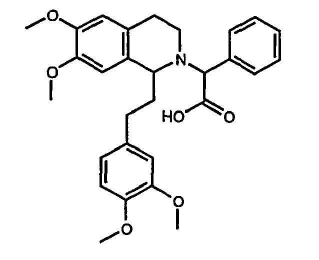 prepared by reaction of 1-[2-(3,4-dimethoxy-phenyl)-ethyl]-6,7-dimethoxy-1,2,3,4-tetrahydro-isoquinoline with methyl α-bromophenylacetate.
   LC-MS: rt = 0.77 min, 492 (M+1, ES+).
**[1-(2,5-Dimethoxy-benzyl)-5,8-dimethoxy-3,4-dihydro-1H-isoquinolin-2-yl]-phenyl-acetic acid:** 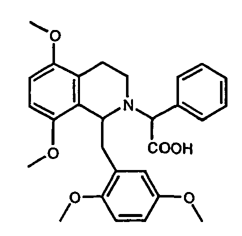 prepared by reaction of 1-(2,5-dimethoxy-benzyl)-5,8-dimethoxy-1,2,3,4-tetrahydro-isoquinoline with methyl α-bromophenylacetate.
   LC-MS: rt = 0.86 min (dia 1), 0.89 min (dia 2), 478 (M+1, ES+).
**{1-[2-(2,5-Difluoro-phenyl)-vinyl]-6,7-dimethoxy-3,4-dihydro-1H-isoquinolin-2-yl}-phenyl-acetic acid:** 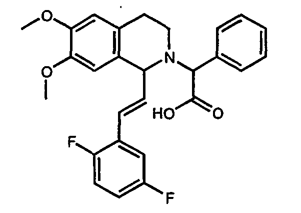 prepared by reaction of 1-[2-(2,5-difluoro-phenyl)-vinyl]-6,7-dimethoxy-1,2,3,4-tetrahydro-isoquinoline with methyl α-bromophenylacetate.
   LC-MS: rt = 0.83 min, 466 (M+1, ES+).
**(6,7-Dimethoxy-1-phenoxymethyl-3,4-dihydro-1H-isoquinolin-2-yl)-phenylacetic acid:** 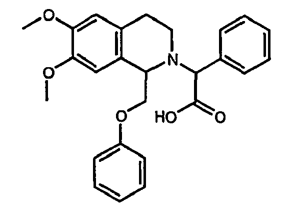 prepared by reaction of 6,7-dimethoxy-1-phenoxymethyl-1,2,3,4-tetrahydroisoquinoline with methyl α-bromophenylacetate.
   LC-MS: rt = 0.80 min (dia 1), 0.81 min (dia 2), 434 (M+1, ES+).
**[6,7-Dimethoxy-1-(3-methoxy-benzyl)-3,4-dihydro-1H-isoquinolin-2-yl]-phenyl-acetic acid:** 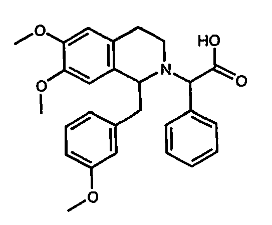 prepared by reaction of 6,7-dimethoxy-1-(3-methoxy-benzyl)-1,2,3,4-tetrahydroisoquinoline with methyl α-bromophenylacetate.
   LC-MS: rt = 0.77 min (dia 1), 0.79 min (dia 2), 448 (M+1, ES+).
**[6-(3,4-Dimethoxy-benzyl)-2,3,8,9-tetrahydro-6H-[1,4]dioxino[2,3-g]isoquinolin-7-yl]-phenyl-acetic acid:** 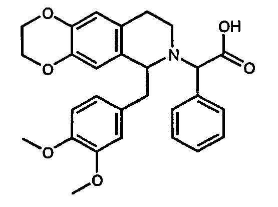 prepared by reaction of 6-(3,4-dimethoxy-benzyl)-2,3,6,7,8,9-hexahydro-[1,4]dioxino[2,3-g]isoquinoline with methyl α-bromophenylacetate.
   LC-MS: rt = 0.77 min (dia 1), 0.79 min (dia 2), 476 (M+1, ES+).

### E Synthesis of (3,4-Dihydro-1H-isoquinolin-2-yl)-acetonitrile derivatives (general procedure):

To a solution of sodium metabisulfite (5.7 mmol) in water (20 mL) is added the respective aldehyde (10.7 mmol), methanol (1.5 mL) and sodium cyanide (11.0 mmol). After 15 min a solution of the respective tetrahydroisoquinoline (10.7 mmol) in methanol (15 mL) is added and the mixture is stirred for 3 h. Water (50 mL) and ethyl acetate (50 mL) are added, the layers are separated and the aqueous layer is extracted twice with ethyl acetate (50 mL each). The combined organic extracts are concentrated in vacuo to give the following acetonitrile derivatives which are used in the next step without further purification as mixtures of racemic diastereoisomers:
**(2-Chloro-phenyl)-[1-(3,4-dimethoxy-benzyl)-6,7-dimethoxy-3,4-dihydro-1H-isoquinolin-2-yl]-acetonitrile:** 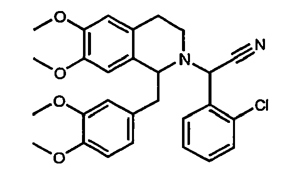 prepared by reaction of tetrahydropapaverine with 2-chlorobenzaldehyde.
   LC-MS: rt = 1.23 min, 493 (M+1, ES+).

### F Synthesis of Tosylated Mandelamide Derivatives:

### F.1 Synthesis of Toluene-4-sulfonic acid carbamoyl-phenyl-methyl ester:

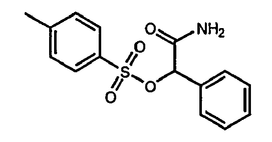

To a suspension of mandelamide (25 mmol) and DMAP (2.0 mmol) in DCM (200 mL) DIPEA (27.5 mmol) is added. The mixture is treated portion wise with p-toluenesulfonyl chloride (27.5 mmol) and stirred for 20 h. The solvents are removed in vacuo. The residue is diluted with ethyl acetate (350 mL) and washed three times with sat. NaHCO₃ solution (3x150 mL) and once with brine (100 mL). The organic extracts are dried with Na₂SO₄ and concentrated in vacuo. The residue is recrystallized from ethyl acetate/heptane 4/1 to give the desired tosylate as white crystals.
LC-MS: rt = 0.87 min, 306 (M+1, ES+).

### F.2 Synthesis of (S)-2-Hydroxy-2-phenyl-acetamide:

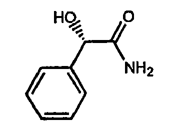

Methyl (S)-(+)-mandelate (120 mmol) is dissolved in a solution of ammonia in methanol (7.0 M, 1.2 mol) at RT. After 1 d nitrogen is bubbled through the solution for 30 min and the solvents are removed in vacuo to give the desired amide as a white solid.
LC-MS: rt = 0.33 min, 152 (M+1, ES+).

### F.3 Synthesis of (S)-Toluene-4-sulfonic acid carbamoyl-phenyl-methyl ester:

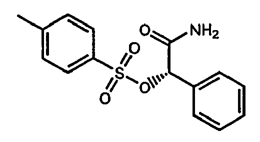

To a solution of (S)-2-Hydroxy-2-phenyl-acetamide (120 mmol) in dioxane (200 mL) are added DIPEA (133 mmol) and DMAP (10 mmol). A solution of p-toluenesulfonyl chloride (121 mmol) in dioxane (50 mL) is added at RT. After 1 d ethyl acetate (400 mL) is added and the organic layer is washed several times with sat. NaHCO₃ solution. Silica gel (50 g) is added, the solvents are removed in vacuo and the residue is purified by flash chromatography (ethyl acetate/heptane 1:2) to give the desired tosylate.
LC-MS: rt = 0.87 min, 306 (M+1, ES+).

### G Synthesis of substituted and unsubstituted glycinamide derivatives:

### G.1 Synthesis of unsubstituted glycinamides (general procedure):

To a suspension of the respective tetrahydroisoquinoline hydrochloride (0.1 mmol) in THF (0.5 mL) is added a solution of DIPEA (0.3 mmol) in THF (0.5 mL) and a solution of 2-bromoacetamide (0.1 mmol) in THF (0.5 mL). The suspension is stirred at 65°C for 14 h, the solvent is removed in vacuo and the residue is purified by prep. HPLC to give the following amides as racemic mixtures:

### Example 1:

### 2-{1-[2-(2,5-Bis-trifluoromethyl-phenyl)-ethyl]-6,7-dimethoxy-3,4-dihydro-1H-isoquinolin-2-yl}-acetamide:

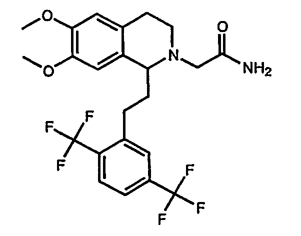

prepared by reaction of 1-[2-(2,5-bis-trifluoromethyl-phenyl)-ethyl]-6,7-dimethoxy-1,2,3,4-tetrahydroisoquinoline with 2-bromoacetamide.
LC-MS: rt = 0.83 min, 491 (M+1, ES+).

### G.2 Synthesis of racemic α-substituted glycinamides via acetic acid derivatives (general procedure):

To a solution of the respective carboxylic acid (3.8 mmol) in DCM (40 mL) is added DMAP (15 mmol) and EDC x HCl (5.7 mmol). After 5 min ammonium bromide (5.0 mmol) is added and the reaction mixture is stirred for 14 h. Water (100 mL) is added, the layers are separated and the aqueous layer is extracted three times with ethyl acetate (100 mL each). The combined organic extracts are concentrated in vacuo and the residue is purified by prep. HPLC to give the following amide derivatives as mixtures of racemic diastereoisomers:

### Reference Example 2:

### 2-(6,7-Dimethoxy-1-naphthalen-2-ylmethyl-3,4-dihydro-1H-isoquinolin-2-yl)-2-phenyl-acetamide:

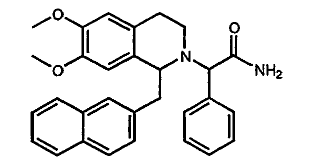

prepared by reaction of (6,7-dimethoxy-1-naphthalen-2-ylmethyl-3,4-dihydro-1H-isoquinolin-2-yl)-phenyl-acetic acid with ammonium bromide.
LC-MS: rt = 0.83 min, 467 (M+1, ES+).

### Example 3:

### 2-{1-[2-(3,4-Difluoro-phenyl)-ethyl]-6,7-dimethoxy-3,4-dihydro-1H-isoquinolin-2-yl}-2-phenyl-acetamide:

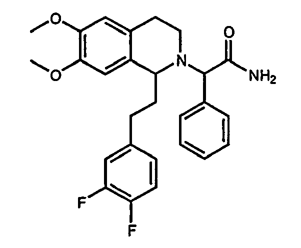

prepared by reaction of {1-[2-(3,4-difluoro-phenyl)-ethyl]-6,7-dimethoxy-3,4-dihydro-1H-isoquinolin-2-yl}-phenyl-acetic acid with ammonium bromide.
LC-MS: rt = 0.81 min, 467 (M+1, ES+).

### Reference Example 4:

### 2-[1-(2-Furan-2-yl-ethyl)-6,7-dimethoxy-3,4-dihydro-1H-isoquinolin-2-yl]-2-phenyl-acetamide:

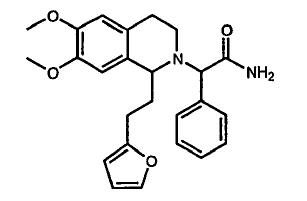

prepared by reaction of [1-(2-furan-2-yl-ethyl)-6,7-dimethoxy-3,4-dihydro-1H-isoquinolin-2-yl]-phenyl-acetic acid with ammonium bromide.
LC-MS: rt = 3.54 min, 421 (M+1, ES+).

### Reference Example 5:

### 2-(1-Benzyl-6-methoxy-3,4-dihydro-1H-isoquinolin-2-yl)-2-phenyl-acetamide:

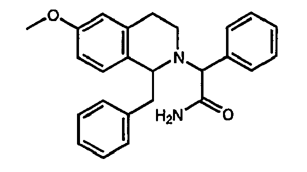

prepared by reaction of (1-benzyl-6-methoxy-3,4-dihydro-1H-isoquinolin-2-yl)-phenyl-acetic acid with ammonium bromide.
LC-MS: rt = 0.82 min, 387 (M+1, ES+).

### Example 6:

### 2-{1-[2-(2,3-Difluoro-phenyl)-ethyl]-6,7-dimethoxy-3,4-dihydro-1H-isoquinolin-2-yl}-2-phenyl-acetamide:

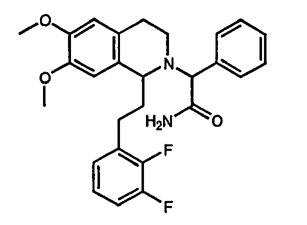

prepared by reaction of {1-[2-(2,3-difluoro-phenyl)-ethyl]-6,7-dimethoxy-3,4-dihydro-1H-isoquinolin-2-yl}-phenyl-acetic acid with ammonium bromide.
LC-MS: rt = 0.83 min, 467 (M+1, ES+).

### Reference Example 7:

### 2-[1-(4-Fluoro-benzyl)-6,7-dimethoxy-3,4-dihydro-1H-isoquinolin-2-yl]-2-phenyl-acetamide:

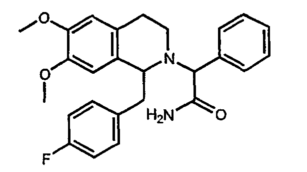

prepared by reaction of [1-(4-fluoro-benzyl)-6,7-dimethoxy-3,4-dihydro-1H-isoquinolin-2-yl]-phenyl-acetic acid with ammonium bromide.
LC-MS: rt = 0.79 min, 435 (M+1, ES+).

### Reference Example 8:

### 2-[1-(2,5-Dimethoxy-benzyl)-6,7-dimethoxy-3,4-dihydro-1H-isoquinolin-2-yl]-2-phenyl-acetamide:

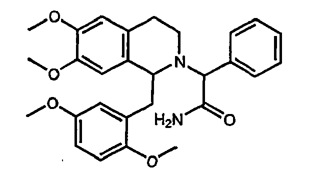

prepared by reaction of [1-(2,5-dimethoxy-benzyl)-6,7-dimethoxy-3,4-dihydro-1H-isoquinolin-2-yl]-phenyl-acetic acid with ammonium bromide.
LC-MS: rt = 0.79 min (dia 1), 0.80 min (dia 2), 477 (M+1, ES+).

### Example 9:

### 2-{6,7-Dimethoxy-1-[2-(4-trifluoromethyl-phenyl)-ethyl]-3,4-dihydro-1H-isoquinolin-2-yl}-2-phenyl-acetamide:

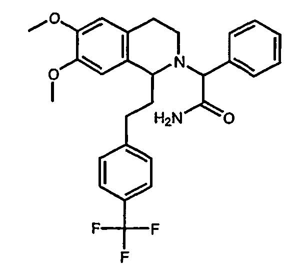

prepared by reaction of {6,7-dimethoxy-1-[2-(4-trifluoromethyl-phenyl)-ethyl]-3,4-dihydro-1H-isoquinolin-2-yl}-phenyl-acetic acid with ammonium bromide.
LC-MS: rt = 0.87 min, 499 (M+1, ES+).

### Example 10:

### 2-{6,7-Dimethoxy-1-[2-(3-methoxy-phenyl)-ethyl]-3,4-dihydro-1H-isoquinolin-2-yl}-2-phenyl-acetamide:

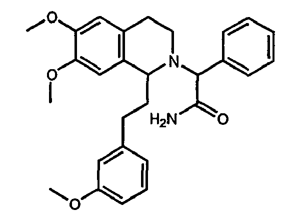

prepared by reaction of {6,7-dimethoxy-1-[2-(3-methoxy-phenyl)-ethyl]-3,4-dihydro-1H-isoquinolin-2-yl}-phenyl-acetic acid with ammonium bromide.
LC-MS: rt = 0.81 min, 461 (M+1, ES+).

### Reference Example 11:

### 2-[6,7-Dimethoxy-1-(4-methoxy-benzyl)-3,4-dihydro-1H-isoquinolin-2-yl]-2-phenyl-acetamide:

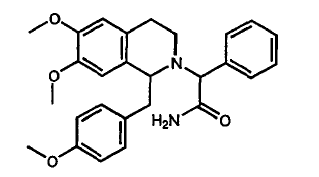

prepared by reaction of [6,7-dimethoxy-1-(4-methoxy-benzyl)-3,4-dihydro-1H-isoquinolin-2-yl]-phenyl-acetic acid with ammonium bromide.
LC-MS: rt = 0.78 min, 447 (M+1, ES+).

### Example 12:

### 2-{1-[2-(2,5-Difluoro-phenyl)-ethyl]-6,7-dimethoxy-3,4-dihydro-1H-isoquinolin-2-yl}-2-phenyl-acetamide:

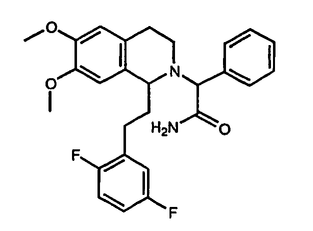

prepared by reaction of {1-[2-(2,5-difluoro-phenyl)-ethyl]-6,7-dimethoxy-3,4-dihydro-1H-isoquinolin-2-yl}-phenyl-acetic acid with ammonium bromide.
LC-MS: rt = 0.83 min, 467 (M+1, ES+).

### Example 13:

### 2-{6,7-Dimethoxy-1-[2-(2-methoxy-phenyl)-ethyl]-3,4-dihydro-1H-isoquinolin-2-yl}-2-phenyl-acetamide:

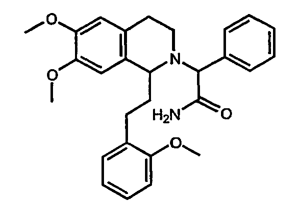

prepared by reaction of {6,7-dimethoxy-1-[2-(2-methoxy-phenyl)-ethyl]-3,4-dihydro-1H-isoquinolin-2-yl}-phenyl-acetic acid with ammonium bromide.
LC-MS: rt = 0.82 min, 461 (M+1, ES+).

### Example 14:

### 2-{1-[2-(4-Fluoro-phenyl)-ethyl]-6,7-dimethoxy-3,4-dihydro-1H-isoquinolin-2-yl}-2-phenyl-acetamide:

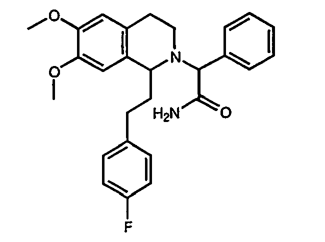

prepared by reaction of {1-[2-(4-fluoro-phenyl)-ethyl]-6,7-dimethoxy-3,4-dihydro-1H-isoquinolin-2-yl}-phenyl-acetic acid with ammonium bromide.
LC-MS: rt = 0.82 min, 449 (M+1, ES+).

### Reference Example 15:

### 2-{1-[2-(2,3-Difluoro-phenyl)-vinyl]-6,7-dimethoxy-3,4-dihydro-1H-isoquinolin-2-yl}-2-phenyl-acetamide:

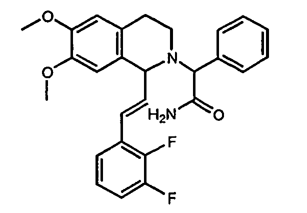

prepared by reaction of {1-[2-(2,3-difluoro-phenyl)-vinyl]-6,7-dimethoxy-3,4-dihydro-1H-isoquinolin-2-yl}-phenyl-acetic acid with ammonium bromide.
LC-MS: rt = 0.82 min, 465 (M+1, ES+).

### Reference Example 16:

### 2-[1-(3-Fluoro-4-methoxy-benzyl)-6,7-dimethoxy-3,4-dihydro-1H-isoquinolin-2-yl]-2-phenyl-acetamide:

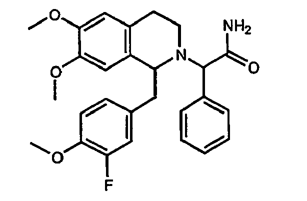

prepared by reaction of [1-(3-fluoro-4-methoxy-benzyl)-6,7-dimethoxy-3,4-dihydro-1H-isoquinolin-2-yl]-phenyl-acetic acid with ammonium bromide.
LC-MS: rt = 0.79 min, 465 (M+1, ES+).

### Example 17:

### 2-{1-[2-(3,4-Dimethoxy-phenyl)-ethyl]-6,7-dimethoxy-3,4-dihydro-1H-isoquinolin-2-yl}-2-phenyl-acetamide:

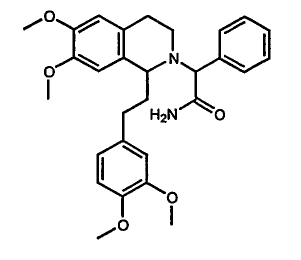

prepared by reaction of {1-[2-(3,4-dimethoxy-phenyl)-ethyl]-6,7-dimethoxy-3,4-dihydro-1H-isoquinolin-2-yl}-phenyl-acetic acid with ammonium bromide.
LC-MS: rt = 0.76 min (dia 1), 0.78 min (dia 2), 491 (M+1, ES+).

### Reference Example 18:

### 2-[1-(2,5-Dimethoxy-benzyl)-5,8-dimethoxy-3,4-dihydro-1H-isoquinolin-2-yl]-2-phenyl-acetamide:

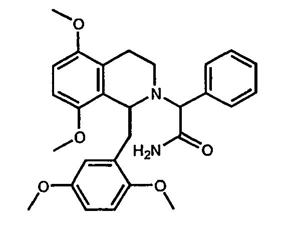

prepared by reaction of [1-(2,5-dimethoxy-benzyl)-5,8-dimethoxy-3,4-dihydro-1H-isoquinolin-2-yl]-phenyl-acetic acid with ammonium bromide.
LC-MS: rt = 0.87 min, 477 (M+1, ES+).

### Reference Example 19:

### 2-{1-[2-(2,5-Difluoro-phenyl)-vinyl]-6,7-dimethoxy-3,4-dihydro-1H-isoquinolin-2-yl}-2-phenyl-acetamide:

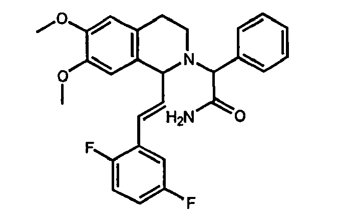

prepared by reaction of {1-[2-(2,5-difluoro-phenyl)-vinyl]-6,7-dimethoxy-3,4-dihydro-1H-isoquinolin-2-yl}-phenyl-acetic acid with ammonium bromide.
LC-MS: rt = 0.82 min, 465 (M+1, ES+).

### Reference Example 20:

### 2-(6,7-Dimethoxy-1-phenoxymethyl-3,4-dihydro-1H-isoquinolin-2-yl)-2-phenyl-acetamide:

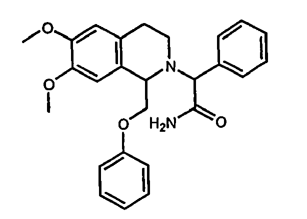

prepared by reaction of (6,7-dimethoxy-1-phenoxymethyl-3,4-dihydro-1H-isoquinolin-2-yl)-phenyl-acetic acid with ammonium bromide.
LC-MS: rt = 0.81 min, 433 (M+1, ES+).

### Reference Example 21:

### 2-[6,7-Dimethoxy-1-(3-methoxy-benzyl)-3,4-dihydro-1H-isoquinolin-2-yl]-2-phenyl-acetamide:

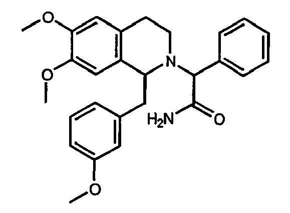

prepared by reaction of [6,7-dimethoxy-1-(3-methoxy-benzyl)-3,4-dihydro-1H-isoquinolin-2-yl]-phenyl-acetic acid with ammonium bromide.
LC-MS: rt = 0.78 min (dia 1), 0.79 min (dia 2), 447 (M+1, ES+).

### Reference Example 22:

### 2-[6-(3,4-Dimethoxy-benzyl)-2,3,8,9-tetrahydro-6H-[1,4]dioxino[2,3-g]isoquinolin-7-yl]-2-phenyl-acetamide:

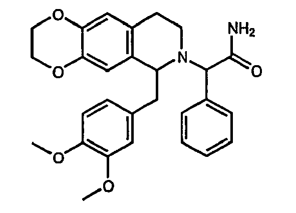

prepared by reaction of [6-(3,4-dimethoxy-benzyl)-2,3,8,9-tetrahydro-6H-[1,4]dioxino[2,3-g]isoquinolin-7-yl]-phenyl-acetic acid with ammonium bromide.
LC-MS: rt = 0.77 min (dia 1), 0.78 min (dia 2), 475 (M+1, ES+).

### Reference Example 23:

### 2-[6,7-Dimethoxy-1-(3-phenyl-propyl)-3,4-dihydro-1H-isoquinolin-2-yl]-2-phenyl-acetamide:

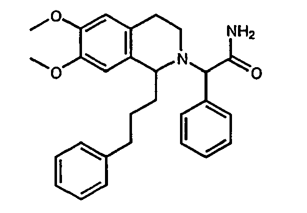

prepared by reaction of [6,7-dimethoxy-1-(3-phenyl-propyl)-3,4-dihydro-1H-isoquinolin-2-yl]-phenyl-acetic acid with ammonium bromide.
LC-MS: rt = 3.83 min, 445 (M+1, ES+).

### G.3 Synthesis of racemic α-substituted glycinamides via acetonitrile derivatives (general procedure):

The crude acetonitrile derivative (10.7 mmol) is dissolved in DMSO (25 mL). Potassium carbonate (4.6 mmol) and hydrogen peroxide solution (30%, 1.8 mL) are added and the mixture is stirred for 21 h. Water (50 mL) and ethyl acetate (100 mL) are added, the layers are separated and the aqueous layer is extracted twice with ethyl acetate (100 mL each). The combined organic extracts are concentrated in vacuo and the residue is purified by flash chromatography to give the following glycinamide derivatives as mixtures of racemic diastereoisomers:

### Reference Example 24:

### 2-(2-Chloro-phenyl)-2-[1-(3,4-dimethoxy-benzyl)-6,7-dimethoxy-3,4-dihydro-1H-isoquinolin-2-yl]-acetamide:

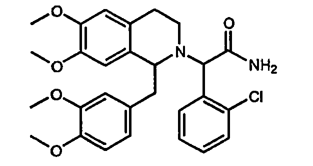

prepared by saponification of (2-chloro-phenyl)-[1-(3,4-dimethoxy-benzyl)-6,7-dimethoxy-3,4-dihydro-1H-isoquinolin-2-yl]-acetonitrile.
LC-MS: rt = 0.82 min, 511 (M+1, ES+).

### G.4 Synthesis of α-substituted glycinamides via alkylation of enantiomerically enriched 1,2,3,4-tetrahydroisoquinolines with racemic tosylates (general procedure):

DIPEA (0.300 mmol) and toluene-4-sulfonic acid carbamoyl-phenyl-methyl ester (0.125 mmol) are added to a solution of the respective enantiomerically enriched 1,2,3,4-tetrahydroisoquinoline derivative (0.125 mmol) in THF (2.0 mL). The mixture is refluxed for 4 d and cooled to RT. The obtained mixture of enantiomerically enriched diastereoisomers is separated by prep. HPLC to give the following amides; datas are given for the more polar (HPLC) and more active (IC₅₀, FLIPR) diastereoisomer:

### Example 25:

### (R)-2-{(S)-6,7-Dimethoxy-1-[2-(2-methyl-5-trifluoromethyl-phenyl)-ethyl]-3,4-dihydro-1H-isoquinolin-2-yl}-2-phenyl-acetamide:

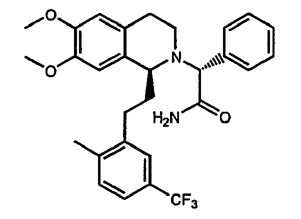

prepared by reaction of toluene-4-sulfonic acid carbamoyl-phenyl-methyl ester with (S)-6,7-dimethoxy-1-[2-(2-methyl-5-trifluoromethyl-phenyl)-ethyl]-1,2,3,4-tetrahydro-isoquinoline.
LC-MS: rt = 0.90 min, 513 (M+1, ES+).

### Example 26:

### (R)-2-{(S)-1-[2-(3-Chloro-2-fluoro-phenyl)-ethyl]-6,7-dimethoxy-3,4-dihydro-1H-isoquinolin-2-yl}-2-phenyl-acetamide:

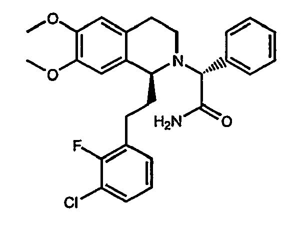

prepared by reaction of toluene-4-sulfonic acid carbamoyl-phenyl-methyl ester with (S)-1-[2-(3-chloro-2-fluoro-phenyl)-ethyl]-6,7-dimethoxy-1,2,3,4-tetrahydroisoquinoline.
LC-MS: rt = 0.86 min, 483 (M+1, ES+).

### Example 27:

### (R)-2-{(S)-1-[2-(3-Fluoro-4-methyl-phenyl)-ethyl]-6,7-dimethoxy-3,4-dihydro-1H-isoquinolin-2-yl}-2-phenyl-acetamide:

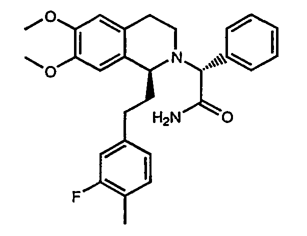

prepared by reaction of toluene-4-sulfonic acid carbamoyl-phenyl-methyl ester with (S)-1-[2-(3-fluoro-4-methyl-phenyl)-ethyl]-6,7-dimethoxy-1,2,3,4-tetrahydroisoquinoline.
LC-MS: rt = 0.85 min, 463 (M+1, ES+).

### Example 28:

### (R)-2-{(S)-1-[2-(4-Fluoro-2-trifluoromethyl-phenyl)-ethyl]-6,7-dimethoxy-3,4-dihydro-1H-isoquinolin-2-yl}-2-phenyl-acetamide:

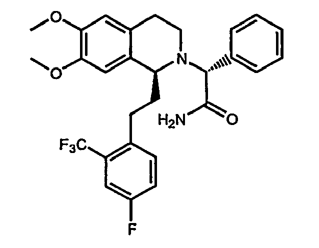

prepared by reaction of toluene-4-sulfonic acid carbamoyl-phenyl-methyl ester with (S)-1-[2-(4-fluoro-2-trifluoromethyl-phenyl)-ethyl]-6,7-dimethoxy-1,2,3,4-tetrahydro-isoquinoline.
LC-MS: rt = 0.88 min, 517 (M+1, ES+).

### Example 29:

### (R)-2-{(S)-1-[2-(5-Fluoro-2-trifluoromethyl-phenyl)-ethyl]-6,7-dimethoxy-3,4-dihydro-1H-isoquinolin-2-yl}-2-phenyl-acetamide:

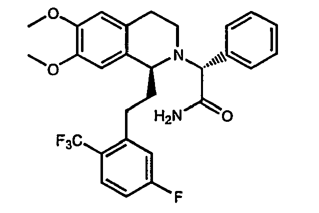

prepared by reaction of toluene-4-sulfonic acid carbamoyl-phenyl-methyl ester with (S)-1-[2-(5-fluoro-2-trifluoromethyl-phenyl)-ethyl]-6,7-dimethoxy-1,2,3,4-tetrahydro-isoquinoline.
LC-MS: rt = 0.87 min, 517 (M+1, ES+).

### Example 30:

### (R)-2-{(S)-1-[2-(2-Fluoro-5-trifluoromethyl-phenyl)-ethyl]-6,7-dimethoxy-3,4-dihydro-1H-isoquinolin-2-yl}-2-phenyl-acetamide:

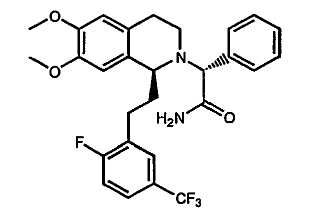

prepared by reaction of toluene-4-sulfonic acid carbamoyl-phenyl-methyl ester with (S)-1-[2-(2-fluoro-5-trifluoromethyl-phenyl)-ethyl]-6,7-dimethoxy-1,2,3,4-tetrahydro-isoquinoline.
LC-MS: rt = 0.88 min, 517 (M+1, ES+).

### Example 31:

### (R)-2-{(S)-[2-(3-Fluoro-4-trifluoromethyl-phenyl)-ethyl]-6,7-dimethoxy-3,4-dihydro-1H-isoquinolin-2-yl}-2-phenyl-acetamide:

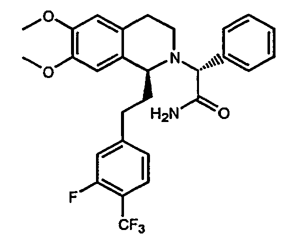

prepared by reaction of toluene-4-sulfonic acid carbamoyl-phenyl-methyl ester with (S)-1-[2-(3-fluoro-4-trifluoromethyl-phenyl)-ethyl]-6,7-dimethoxy-1,2,3,4-tetrahydro-isoquinoline.
LC-MS: rt = 0.88 min, 517 (M+1, ES+).

### Example 32:

### (R)-2-{(S)-1-[2-(4-Fluoro-3-methyl-phenyl)-ethyl]-6,7-dimethoxy-3,4-dihydro-1H-isoquinolin-2-yl}-2-phenyl-acetamide:

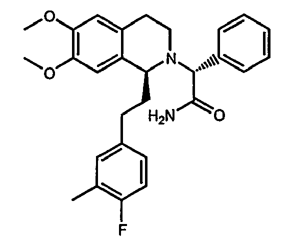

prepared by reaction of toluene-4-sulfonic acid carbamoyl-phenyl-methyl ester with (S)-1-[2-(4-fluoro-3-methyl-phenyl)-ethyl]-6,7-dimethoxy-1,2,3,4-tetrahydroisoquinoline.
LC-MS: rt = 0.85 min, 463 (M+1, ES+).

### Example 33:

### (R)-2-{(S)-6,7-Dimethoxy-1-[2-(2,3,5-trifluoro-phenyl)-ethyl]-3,4-dihydro-1H-isoquinolin-2-yl}-2-phenyl-acetamide:

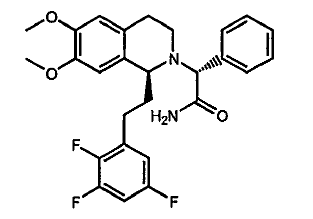

prepared by reaction of toluene-4-sulfonic acid carbamoyl-phenyl-methyl ester with (S)-6,7-dimethoxy-1-[2-(2,3,5-trifluoro-phenyl)-ethyl]-1,2,3,4-tetrahydroisoquinoline.
LC-MS: rt = 0.84 min, 485 (M+1, ES+).

### Example 34:

### (R)-2-{(S)-1-[2-(4-Fluoro-3-trifluoromethyl-phenyl)-ethyl]-6,7-dimethoxy-3,4-dihydro-1H-isoquinolin-2-yl}-2-phenyl-acetamide:

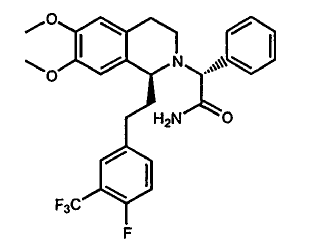

prepared by reaction of toluene-4-sulfonic acid carbamoyl-phenyl-methyl ester with (S)-1-[2-(4-fluoro-3-trifluoromethyl-phenyl)-ethyl]-6,7-dimethoxy-1,2,3,4-tetrahydro-isoquinoline.
LC-MS: rt = 0.88 min, 517 (M+1, ES+).

### Example 35:

### (R)-2-{(S)-1-[2-(3-Fluoro-5-trifluoromethyl-phenyl)-ethyl]-6,7-dimethoxy-3,4-dihydro-1H-isoquinolin-2-yl}-2-phenyl-acetamide:

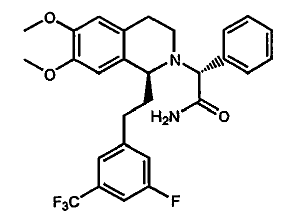

prepared by reaction of toluene-4-sulfonic acid carbamoyl-phenyl-methyl ester with (S)-1-[2-(3-fluoro-5-trifluoromethyl-phenyl)-ethyl]-6,7-dimethoxy-1,2,3,4-tetrahydro-isoquinoline.
LC-MS: rt = 0.88 min, 517 (M+1, ES+).

### Example 36:

### (R)-2-{(S)-1-[2-(5-Chloro-2-trifluoromethyl-phenyl)-ethyl]-6,7-dimethoxy-3,4-dihydro-1H-isoquinolin-2-yl}-2-phenyl-acetamide:

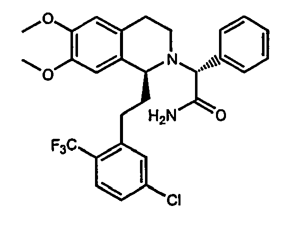

prepared by reaction of toluene-4-sulfonic acid carbamoyl-phenyl-methyl ester with (S)-1-[2-(5-chloro-2-trifluoromethyl-phenyl)-ethyl]-6,7-dimethoxy-1,2,3,4-tetrahydro-isoquinoline.
LC-MS: rt = 0.90 min, 533 (M+1, ES+).

### Example 37:

### (R)-2-{(S)-1-[2-(2-Fluoro-3-trifluoromethyl-phenyl)-ethyl]-6,7-dimethoxy-3,4-dihydro-1H-isoquinolin-2-yl}-2-phenyl-acetamide:

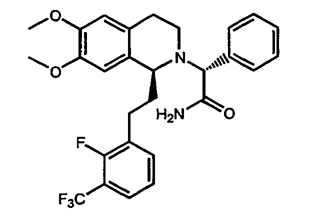

prepared by reaction of toluene-4-sulfonic acid carbamoyl-phenyl-methyl ester with (S)-1-[2-(2-fluoro-3-trifluoromethyl-phenyl)-ethyl]-6,7-dimethoxy-1,2,3,4-tetrahydro-isoquinoline.
LC-MS: rt = 0.88 min, 517 (M+1, ES+).

### Example 38:

### (R)-2-{(S)-1-[2-(2-Fluoro-4-trifluoromethyl-phenyl)-ethyl]-6,7-dimethoxy-3,4-dihydro-1H-isoquinolin-2-yl}-2-phenyl-acetamide:

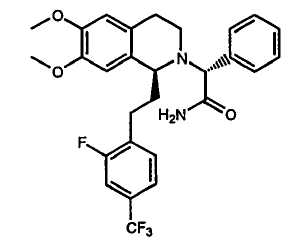

prepared by reaction of toluene-4-sulfonic acid carbamoyl-phenyl-methyl ester with (S)-1-[2-(2-fluoro-4-trifluoromethyl-phenyl)-ethyl]-6,7-dimethoxy-1,2,3,4-tetrahydro-isoquinoline.
LC-MS: rt = 0.88 min, 517 (M+1, ES+).

### Example 39:

### (R)-2-{(S)-1-[2-(2-Difluoromethoxy-phenyl)-ethyl]-6,7-dimethoxy-3,4-dihydro-1H-isoquinolin-2-yl}-2-phenyl-acetamide:

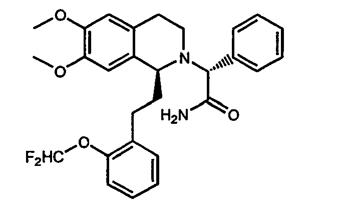

prepared by reaction of toluene-4-sulfonic acid carbamoyl-phenyl-methyl ester with (S)-1-[2-(2-difluoromethoxy-phenyl)-ethyl]-6,7-dimethoxy-1,2,3,4-tetrahydro-isoquinoline.
LC-MS: rt = 0.84 min, 497 (M+1, ES+).

### Example 40:

### (R)-2-{(S)-1-[2-(3-Fluoro-2-trifluoromethyl-phenyl)-ethyl]-6,7-dimethoxy-3,4-dihydro-1H-isoquinolin-2-yl}-2-phenyl-acetamide:

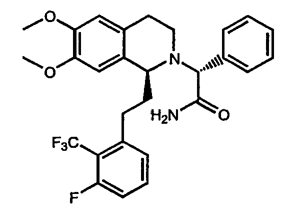

prepared by reaction of toluene-4-sulfonic acid carbamoyl-phenyl-methyl ester with (S)-1-[2-(3-fluoro-2-trifluoromethyl-phenyl)-ethyl]-6,7-dimethoxy-1,2,3,4-tetrahydro-isoquinoline.
LC-MS: rt = 0.86 min, 517 (M+1, ES+).

### Example 41:

### (R)-2-{(S)-1-[2-(2-Chloro-3-trifluoromethyl-phenyl)-ethyl]-6,7-dimethoxy-3,4-dihydro-1H-isoquinolin-2-yl}-2-phenyl-acetamide:

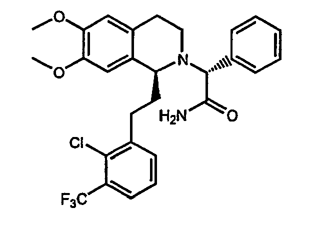

prepared by reaction of toluene-4-sulfonic acid carbamoyl-phenyl-methyl ester with (S)-1-[2-(2-chloro-3-trifluoromethyl-phenyl)-ethyl]-6,7-dimethoxy-1,2,3,4-tetrahydro-isoquinoline.
LC-MS: rt = 0.89 min, 533 (M+1, ES+).

### Example 42:

### (R)-2-{(S)-1-[2-(2-Fluoro-6-trifluoromethyl-phenyl)-ethyl]-6,7-dimethoxy-3,4-dihydro-1H-isoquinolin-2-yl}-2-phenyl-acetamide:

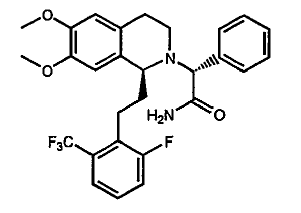

prepared by reaction of toluene-4-sulfonic acid carbamoyl-phenyl-methyl ester with (S)-1-[2-(2-fluoro-6-trifluoromethyl-phenyl)-ethyl]-6,7-dimethoxy-1,2,3,4-tetrahydro-isoquinoline.
LC-MS: rt = 0.86 min, 517 (M+1, ES+).

### Example 43:

### (R)-2-{(S)-1-[2-(4-Chloro-3-trifluoromethyl-phenyl)-ethyl]-6,7-dimethoxy-3,4-dihydro-1H-isoquinolin-2-yl}-2-phenyl-acetamide:

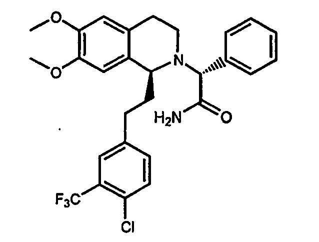

prepared by reaction of toluene-4-sulfonic acid carbamoyl-phenyl-methyl ester with (S)-1-[2-(4-chloro-3-trifluoromethyl-phenyl)-ethyl]-6,7-dimethoxy-1,2,3,4-tetrahydro-isoquinoline.
LC-MS: rt = 0.90 min, 533 (M+1, ES+).

### Example 44:

### (R)-2-{(S)-1-[2-(2,3-Difluoro-4-methyl-phenyl)-ethyl]-6,7-dimethoxy-3,4-dihydro-1H-isoquinolin-2-yl}-2-phenyl-acetamide:

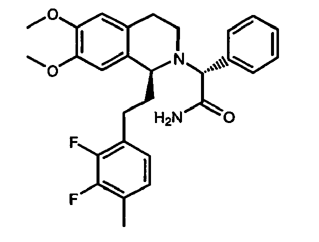

prepared by reaction of toluene-4-sulfonic acid carbamoyl-phenyl-methyl ester with (S)-1-[2-(2,3-difluoro-4-methyt-phenyl)-ethyl]-6,7-dimethoxy-1,2,3,4-tetrahydro-isoquinoline.
LC-MS: rt = 0.86 min, 481 (M+1, ES+).

### Example 45:

### (R)-2-{(S)-1-[2-(4-Difluoromethoxy-phenyl)-ethyl]-6,7-dimethoxy-3,4-dihydro-1H-isoquinolin-2-yl}-2-phenyl-acetamide:

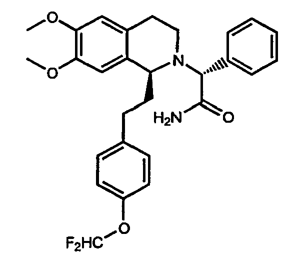

prepared by reaction of toluene-4-sulfonic acid carbamoyl-phenyl-methyl ester with (S)-1-[2-(4-difluoromethoxy-phenyl)-ethyl]-6,7-dimethoxy-1,2,3,4-tetrahydro-isoquinoline.
LC-MS: rt = 0.84 min, 497 (M+1, ES+).

### Example 46:

### (R)-2-{(S)-1-[2-(3,4-Dimethyl-phenyl)-ethyl]-6,7-dimethoxy-3,4-dihydro-1H-isoquinolin-2-yl}-2-phenyl-acetamide:

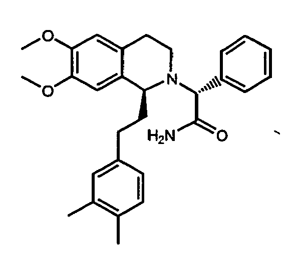

prepared by reaction of toluene-4-sulfonic acid carbamoyl-phenyl-methyl ester with (S)-1-[2-(3,4-dimethyl-phenyl)-ethyl]-6,7-dimethoxy-1,2,3,4-tetrahydroisoquinoline.
LC-MS: rt = 0.87 min, 459 (M+1, ES+).

### Example 47:

### (R)-2-{(S)-1-[2-(3-Chloro-4-methyl-phenyl)-ethyl]-6,7-dimethoxy-3,4-dihydro-1H-isoquinolin-2-yl}-2-phenyl-acetamide:

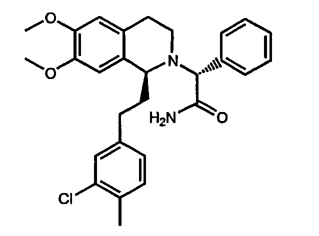

prepared by reaction of toluene-4-sulfonic acid carbamoyl-phenyl-methyl ester with (S)-1-[2-(3-chloro-4-methyl-phenyl)-ethyl]-6,7-dimethoxy-1,2,3,4-tetrahydro-isoquinoline.
LC-MS: rt = 0.88 min, 479 (M+1, ES+).

### Example 48:

### (R)-2-{(S)-1-[2-(2-Chloro-6-fluoro-phenyl)-ethyl]-6,7-dimethoxy-3,4-dihydro-1H-isoquinolin-2-yl}-2-phenyl-acetamide:

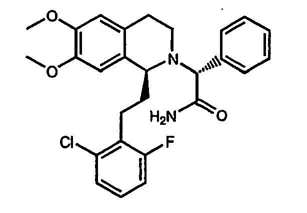

prepared by reaction of toluene-4-sulfonic acid carbamoyl-phenyl-methyl ester with (S)-1-[2-(2-chloro-6-fluoro-phenyl)-ethyl]-6,7-dimethoxy-1,2,3,4-tetrahydroisoquinoline.
LC-MS: rt = 0.85 min, 483 (M+1, ES+).

### Example 49:

### (R)-2-[(S)-6,7-Dimethoxy-1-(2-o-tolyl-ethyl)-3,4-dihydro-1H-isoquinolin-2-yl]-2-phenyl-acetamide:

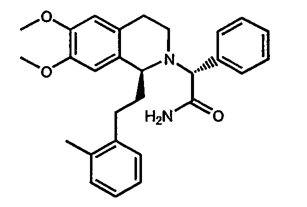

prepared by reaction of toluene-4-sulfonic acid carbamoyl-phenyl-methyl ester with (S)-6,7-dimethoxy-1-(2-o-tolyl-ethyl)-1,2,3,4-tetrahydro-isoquinoline.
LC-MS: rt = 0.84 min, 445 (M+1, ES+).

### Example 50:

### (R)-2-[(S)-6,7-Dimethoxy-1-(2-m-tolyl-ethyl)-3,4-dihydro-1H-isoquinolin-2-yl]-2-phenyl-acetamide:

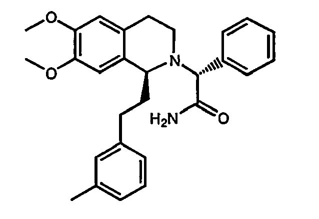

prepared by reaction of toluene-4-sulfonic acid carbamoyl-phenyl-methyl ester with (S)-6,7-dimethoxy-1-(2-m-tolyl-ethyl)- 1,2,3,4-tetrahydro-isoquinoline.
LC-MS: rt = 0.84 min, 445 (M+1, ES+).

### Example 51:

### (R)-2-[(S)-6,7-Dimethoxy-1-(2-p-tolyl-ethyl)-3,4-dihydro-1H-isoquinolin-2-yl]-2-phenyl-acetamide:

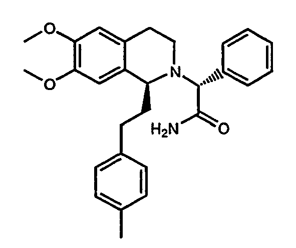

prepared by reaction of toluene-4-sulfonic acid carbamoyl-phenyl-methyl ester with (S)-6,7-dimethoxy-1-(2-p-tolyl-ethyl)-1,2,3,4-tetrahydro-isoquinoline.
LC-MS: rt = 0.84 min, 445 (M+1, ES+).

### Example 52:

### (R)-2-{(S)-1-[2-(2-Fluoro-phenyl)-ethyl]-6,7-dimethoxy-3,4-dihydro-1H-isoquinolin-2-yl}-2-phenyl-acetamide:

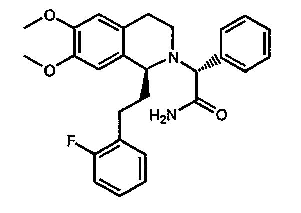

prepared by reaction of toluene-4-sulfonic acid carbamoyl-phenyl-methyl ester with (S)-1-[2-(2-fluoro-phenyl)-ethyl]-6,7-dimethoxy-1,2,3,4-tetrahydroisoquinoline.
LC-MS: rt = 0.82 min, 449 (M+1, ES+).

### Example 53:

### (R)-2-{(S)-1-[2-(3-Fluoro-phenyl)-ethyl]-6,7-dimethoxy-3,4-dihydro-1H-isoquinolin-2-yl}-2-phenyl-acetamide:

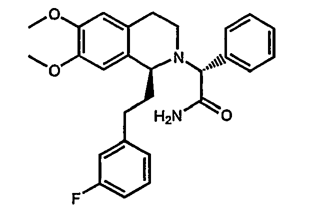

prepared by reaction of toluene-4-sulfonic acid carbamoyl-phenyl-methyl ester with (S)-1-[2-(3-fluoro-phenyl)-ethyl]-6,7-dimethoxy-1,2,3,4-tetrahydroisoquinoline.
LC-MS: rt = 0.81 min, 449 (M+1, ES+).

### Example 54:

### (R)-2-{(S)-1-[2-(2,6-Dichloro-phenyl)-ethyl]-6,7-dimethoxy-3,4-dihydro-1H-isoquinolin-2-yl}-2-phenyl-acetamide:

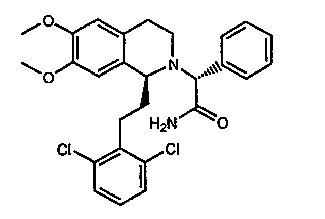

prepared by reaction of toluene-4-sulfonic acid carbamoyl-phenyl-methyl ester with (S)-1-[2-(2,6-dichloro-phenyl)-ethyl]-6,7-dimethoxy-1,2,3,4-tetrahydroisoquinoline.
LC-MS: rt = 0.87 min, 499 (M+1, ES+).

### Example 55:

### (R)-2-{(S)-1-[2-(3,4-Dichloro-phenyl)-ethyl]-6,7-dimethoxy-3,4-dihydro-1H-isoquinolin-2-yl}-2-phenyl-acetamide:

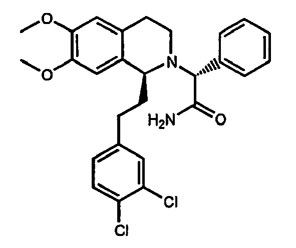

prepared by reaction of toluene-4-sulfonic acid carbamoyl-phenyl-methyl ester with (S)-1-[2-(3,4-dichloro-phenyl)-ethyl]-6,7-dimethoxy-1,2,3,4-tetrahydroisoquinoline.
LC-MS: rt = 0.89 min, 499 (M+1, ES+).

### Example 56:

### (R)-2-{(S)-1-[2-(3,5-Dimethyl-phenyl)-ethyl]-6,7-dimethoxy-3,4-dihydro-1H-isoquinolin-2-yl}-2-phenyl-acetamide:

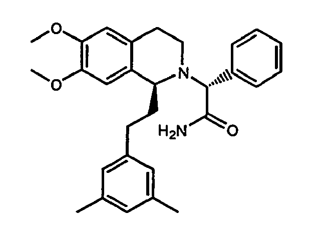

prepared by reaction of toluene-4-sulfonic acid carbamoyl-phenyl-methyl ester with (S)-1-[2-(3,5-dimethyl-phenyl)-ethyl]-6,7-dimethoxy-1,2,3,4-tetrahydroisoquinoline.
LC-MS: rt = 0.87 min, 459 (M+1, ES+).

### Example 57:

### (R)-2-{(S)-6,7-Dimethoxy-1-[2-(2-trifluoromethyl-phenyl)-ethyl]-3,4-dihydro-1H-isoquinolin-2-yl}-2-phenyl-acetamide:

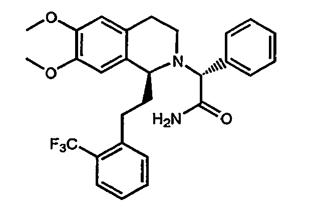

prepared by reaction of toluene-4-sulfonic acid carbamoyl-phenyl-methyl ester with (S)-6,7-dimethoxy-1-[2-(2-trifluoromethyl-phenyl)-ethyl]-1,2,3,4-tetrahydroisoquinoline.
LC-MS: rt = 0.86 min, 499 (M+1, ES+).

### Example 58:

### (R)-2-{(S)-1-[2-(2,4-Difluoro-phenyl)-ethyl]-6,7-dimethoxy-3,4-dihydro-1H-isoquinolin-2-yl}-2-phenyl-acetamide:

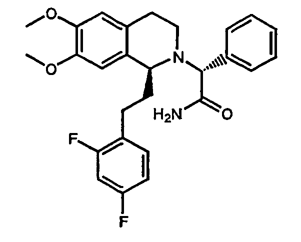

prepared by reaction of toluene-4-sulfonic acid carbamoyl-phenyl-methyl ester with (S)-1-[2-(2,4-difluoro-phenyl)-ethyl]-6,7-dimethoxy-1,2,3,4-tetrahydroisoquinoline.
LC-MS: rt = 0.83 min, 467 (M+1, ES+).

### Example 59:

### (R)-2-{(S)-6,7-Dimethoxy-1-[2-(2,3,6-trifluoro-phenyl)-ethyl]-3,4-dihydro-1H-isoquinolin-2-yl}-2-phenyl-acetamide:

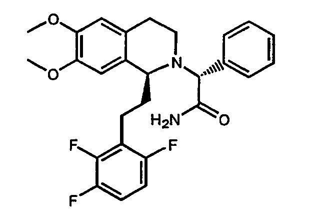

prepared by reaction of toluene-4-sulfonic acid carbamoyl-phenyl-methyl ester with (S)-6,7-dimethoxy-1-[2-(2,3,6-trifluoro-phenyl)-ethyl]-1,2,3,4-tetrahydroisoquinoline.
LC-MS: rt = 0.83 min, 485 (M+1, ES+).

### Example 60:

### (R)-2-{(S)-6,7-Dimethoxy-1-[2-(4-trifluoromethoxy-phenyl)-ethyl]-3,4-dihydro-1H-isoquinolin-2-yl}-2-phenyl-acetamide:

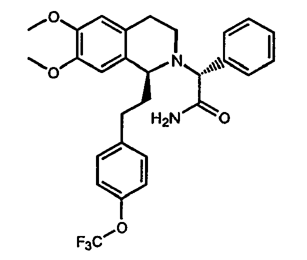

prepared by reaction of toluene-4-sulfonic acid carbamoyl-phenyl-methyl ester with (S)-6,7-dimethoxy-1-[2-(4-trifluoromethoxy-phenyl)-ethyl]-1,2,3,4-tetrahydro-isoquinoline.
LC-MS: rt = 0.88 min, 515 (M+1, ES+).

### Example 61:

### (R)-2-{(S)-1-[2-(2-Fluoro-3-methyl-phenyl)-ethyl]-6,7-dimethoxy-3,4-dihydro-1H-isoquinolin-2-yl}-2-phenyl-acetamide:

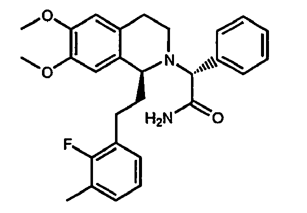

prepared by reaction of toluene-4-sulfonic acid carbamoyl-phenyl-methyl ester with (S)-1-[2-(2-fluoro-3-methyl-phenyl)-ethyl]-6,7-dimethoxy-1,2,3,4-tetrahydroisoquinoline.
LC-MS: rt = 0.85 min, 463 (M+1, ES+).

### Example 62:

### (R)-2-{(S)-1-[2-(4-Chloro-phenyl)-ethyl]-6,7-dimethoxy-3,4-dihydro-1H-isoquinolin-2-yl}-2-phenyl-acetamide:

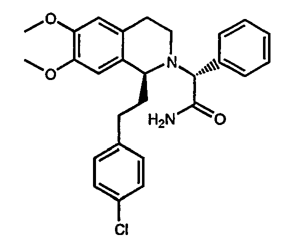

prepared by reaction of toluene-4-sulfonic acid carbamoyl-phenyl-methyl ester with (S)-1-[2-(4-chloro-phenyl)-ethyl]-6,7-dimethoxy-1,2,3,4-tetrahydroisoquinoline.
LC-MS: rt = 0.85 min, 465 (M+1, ES+).

### Example 63:

### (R)-2-{(S)-1-[2-(3-Chloro-phenyl)-ethyl]-6,7-dimethoxy-3,4-dihydro-1H-isoquinolin-2-yl}-2-phenyl-acetamide:

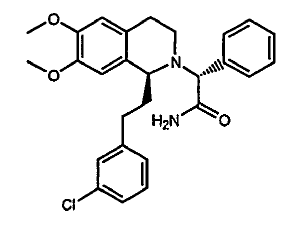

prepared by reaction of toluene-4-sulfonic acid carbamoyl-phenyl-methyl ester with (S)-1-[2-(3-chloro-phenyl)-ethyl]-6,7-dimethoxy-1,2,3,4-tetrahydroisoquinoline.
LC-MS: rt = 0.85 min, 465 (M+1, ES+).

### Example 64:

### (R)-2-{(S)-6,7-Dimethoxy-1-[2-(3-trifluoromethoxy-phenyl)-ethyl]-3,4-dihydro-1H-isoquinolin-2-yl}-2-phenyl-acetamide:

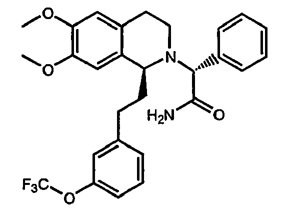

prepared by reaction of toluene-4-sulfonic acid carbamoyl-phenyl-methyl ester with (S)-6,7-dimethoxy-1-[2-(3-trifluoromethoxy-phenyl)-ethyl]-1,2,3,4-tetrahydro-isoquinoline.
LC-MS: rt = 0.89 min, 515 (M+1, ES+).

### Example 65:

### (R)-2-{(S)-6,7-Dimethoxy-1-[2-(3,4,5-trifluoro-phenyl)-ethyl]-3,4-dihydro-1H-isoquinolin-2-yl}-2-phenyl-acetamide:

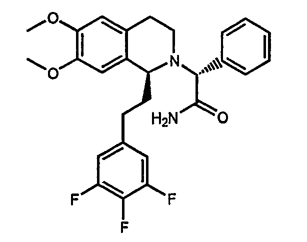

prepared by reaction of toluene-4-sulfonic acid carbamoyl-phenyl-methyl ester with (S)-6,7-dimethoxy-1-[2-(3,4,5-trifluoro-phenyl)-ethyl]-1,2,3,4-tetrahydroisoquinoline.
LC-MS: rt = 0.85 min, 485 (M+1, ES+).

### Example 66:

### (R)-2-{(S)-6,7-Dimethoxy-1-[2-(3-trifluoromethyl-phenyl)-ethyl]-3,4-dihydro-1H-isoquinolin-2-yl}-2-phenyl-acetamide:

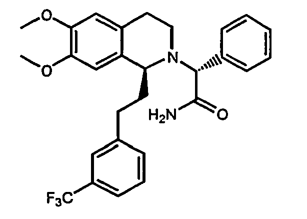

prepared by reaction of toluene-4-sulfonic acid carbamoyl-phenyl-methyl ester with (S)-6,7-dimethoxy-1-[2-(3-trifluoromethyl-phenyl)-ethyl]-1,2,3,4-tetrahydroisoquinoline.
LC-MS: rt = 0.87 min, 499 (M+1, ES+).

### Example 67:

### (R)-2-{(S)-6,7-Dimethoxy-1-[2-(2,3,4-trifluoro-phenyl)-ethyl]-3,4-dihydro-1H-isoquinolin-2-yl}-2-phenyl-acetamide:

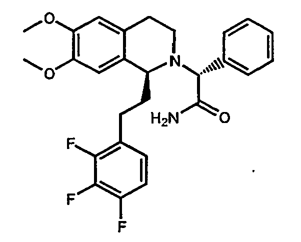

prepared by reaction of toluene-4-sulfonic acid carbamoyl-phenyl-methyl ester with (S)-6,7-dimethoxy-1-[2-(2,3,4-trifluoro-phenyl)-ethyl]-1,2,3,4-tetrahydroisoquinoline.
LC-MS: rt = 0.85 min, 485 (M+1, ES+).

### Example 68:

### (R)-2-{(S)-1-[2-(4-Bromo-2-fluoro-phenyl)-ethyl]-6,7-dimethoxy-3,4-dihydro-1H-isoquinolin-2-yl}-2-phenyl-acetamide:

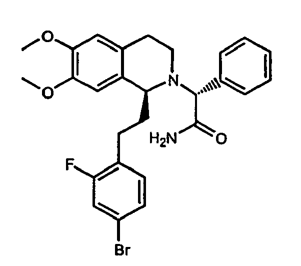

prepared by reaction of toluene-4-sulfonic acid carbamoyl-phenyl-methyl ester with (S)-1-[2-(4-bromo-2-fluoro-phenyl)-ethyl]-6,7-dimethoxy-1,2,3,4-tetrahydroisoquinoline.
LC-MS: rt = 0.87 min, 527 (M+1, ES+).

### Example 69:

### (R)-2-{(S)-1-[2-(2,6-Difluoro-phenyl)-ethyl]-6,7-dimethoxy-3,4-dihydro-1H-isoquinolin-2-yl}-2-phenyl-acetamide:

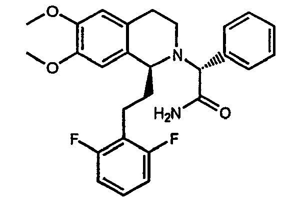

prepared by reaction of toluene-4-sulfonic acid carbamoyl-phenyl-methyl ester with (S)-1-[2-(2,6-difluoro-phenyl)-ethyl]-6,7-dimethoxy-1,2,3,4-tetrahydroisoquinoline.
LC-MS: rt = 0.82 min, 467 (M+1, ES+).

### Example 70:

### (R)-2-{(S)-6,7-Dimethoxy-1-[2-(2-trifluoromethoxy-phenyl)-ethyl]-3,4-dihydro-1H-isoquinolin-2-yl}-2-phenyl-acetamide:

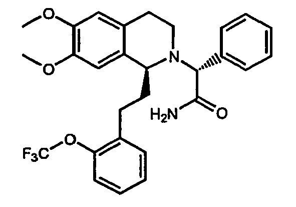

prepared by reaction of toluene-4-sulfonic acid carbamoyl-phenyl-methyl ester with (S)-6,7-dimethoxy-1-[2-(2-trifluoromethoxy-phenyl)-ethyl]-1,2,3,4-tetrahydro-isoquinoline.
LC-MS: rt = 0.87 min, 515 (M+1, ES+).

### Example 71:

### (R)-2-{(S)-1-[2-(2,4-Dichloro-phenyl)-ethyl]-6,7-dimethoxy-3,4-dihydro-1H-isoquinolin-2-yl}-2-phenyl-acetamide:

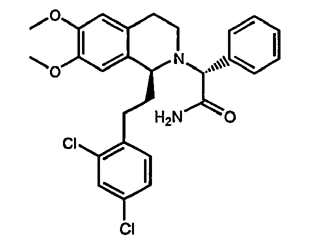

prepared by reaction of toluene-4-sulfonic acid carbamoyl-phenyl-methyl ester with (S)-1-[2-(2,4-dichloro-phenyl)-ethyl]-6,7-dimethoxy-1,2,3,4-tetrahydroisoquinoline.
LC-MS: rt = 0.89 min, 499 (M+1, ES+).

### Example 72:

### (R)-2-{(S)-6,7-Dimethoxy-1-[2-(2,4,5-trifluoro-phenyl)-ethyl]-3,4-dihydro-1H-isoquinolin-2-yl}-2-phenyl-acetamide:

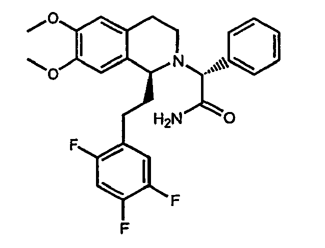

prepared by reaction of toluene-4-sulfonic acid carbamoyl-phenyl-methyl ester with (S)-6,7-dimethoxy-1-[2-(2,4,5-trifluoro-phenyl)-ethyl]-1,2,3,4-tetrahydroisoquinoline.
LC-MS: rt = 0.84 min, 485 (M+1, ES+).

### Example 73:

### (R)-2-{(S)-1-[2-(3-Bromo-phenyl)-ethyl]-6,7-dimethoxy-3,4-dihydro-1H-isoquinolin-2-yl}-2-phenyl-acetamide:

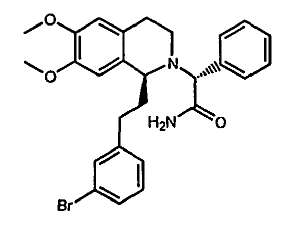

prepared by reaction of toluene-4-sulfonic acid carbamoyl-phenyl-methyl ester with (S)-1-[2-(3-bromo-phenyl)-ethyl]-6,7-dimethoxy-1,2,3,4-tetrahydroisoquinoline.
LC-MS: rt = 0.86 min, 509 (M+1, ES+).

### Example 74:

### (R)-2-{(S)-1-[2-(4-tert-Butyl-phenyl)-ethyl]-6,7-dimethoxy-3,4-dihydro-1H-isoquinolin-2-yl}-2-phenyl-acetamide:

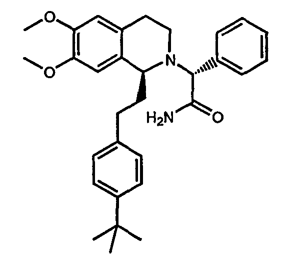

prepared by reaction of toluene-4-sulfonic acid carbamoyl-phenyl-methyl ester with (S)-1-[2-(4-tert-butyl-phenyl)-ethyl]-6,7-dimethoxy-1,2,3,4-tetrahydroisoquinoline.
LC-MS: rt = 0.92 min, 487 (M+1, ES+).

### Example 75:

### (R)-2-{(S)-1-[2-(2-Bromo-phenyl)-ethyl]-6,7-dimethoxy-3,4-dihydro-1H-isoquinolin-2-yl}-2-phenyl-acetamide:

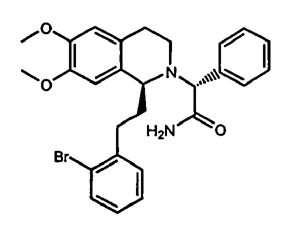

prepared by reaction of toluene-4-sulfonic acid carbamoyl-phenyl-methyl ester with (S)-1-[2-(2-bromo-phenyl)-ethyl]-6,7-dimethoxy-1,2,3,4-tetrahydroisoquinoline.
LC-MS: rt = 0.85 min, 509 (M+1, ES+).

### Example 76:

### (R)-2-{(S)-1-[2-(4-Bromo-phenyl)-ethyl]-6,7-dimethoxy-3,4-dihydro-1H-isoquinolin-2-yl}-2-phenyl-acetamide:

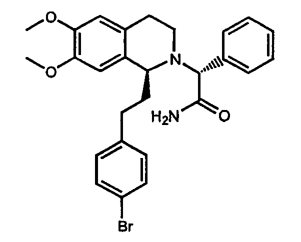

prepared by reaction of toluene-4-sulfonic acid carbamoyl-phenyl-methyl ester with (S)-1-[2-(4-bromo-phenyl)-ethyl]-6,7-dimethoxy-1,2,3,4-tetrahydroisoquinoline.
LC-MS: rt = 0.86 min, 509 (M+1, ES+).

### G.5 Synthesis of α-substituted glycinamides via alkylation of enantiomerically enriched 1,2,3,4-tetrahydroisoquinolines with (S)-Toluene-4-sulfonic acid carbamoyl-phenyl-methyl ester (general procedure):

A solution of the respective enantiomerically enriched 1,2,3,4-tetrahydroisoquinoline derivative (19.7 mmol), DIPEA (27.6 mmol) and (S)-toluene-4-sulfonic acid carbamoyl-phenyl-methyl ester (23.6 mmol) in 2-butanone is stirred under reflux for 2 d. Silica gel (106 g) and DCM (100 mL) are added, the solvents are removed in vacuo and the residue is purified by flash chromatography (gradient: ethyl acetate/heptane 1/2 to 4/1) to give the following acetamide derivatives:

### Example 77:

### (R)-2-{(S)-6,7-Dimethoxy-1-[2-(4-trifluoromethyl-phenyl)-ethyl]-3,4-dihydro-1H-isoquinolin-2-yl}-2-phenyl-acetamide:

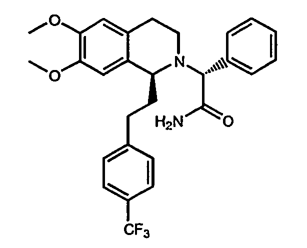

prepared by reaction of (S)-toluene-4-sulfonic acid carbamoyl-phenyl-methyl ester with (S)-6,7-dimethoxy-1-[2-(4-trifluoromethyl-phenyl)-ethyl]-1,2,3,4-tetrahydroisoquinoline.
LC-MS: rt = 0.86 min, 499 (M+1, ES+).

## Claims

1. Compounds of formula (I) 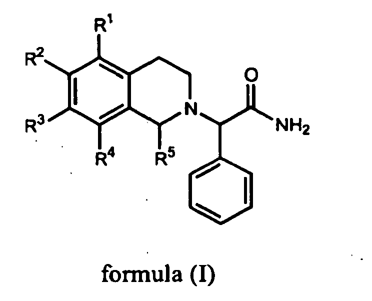 wherein
R¹, R², R³, R⁴ independently represent hydrogen; cyano; halogen; hydroxyl; C1-C4 alkyl; C2-C4 alkenyl; C1-C4 alkoxy; vinyloxy; allyloxy; trifluoromethoxy; C3-C6 cycloalkyloxy; or R¹ and R² together as well as R² and R³ together or R³ and R⁴ together may form with the phenyl ring, to which they are attached, a five, six or seven-membered ring containing one or two oxygen atoms; and
R⁵ represents a mono-, di- or trisubstituted phenyl-ethyl group, substituted independently with C1-C4 alkyl, C1-C4 alkoxy, C2-C4 alkenyl, trifluoromethyl, trifluormethoxy, difluoromethoxy or halogen;
and pharmaceutically acceptable salts thereof.

2. Compounds of formula (I) according to claim 1, wherein
R¹ and R⁴ represent hydrogen;
R² and R³ represent C1-C4 alkoxy; and
R⁵ represents a 2-phenylethyl group in which the phenyl group carries one, two or three substituents, each independently selected from C1-C4 alkyl or halogen;
and pharmaceutically acceptable salts thereof.

3. Compounds of formula (I) according to claim 1, wherein
R¹ and R⁴ represent hydrogen;
R² and R³ represent C1-C4 alkoxy; and
R⁵ represents a 2-phenylethyl group in which the phenyl group carries one or two substituents, each independently selected from C1-C4 alkoxy or halogen;
and pharmaceutically acceptable salts thereof.

4. Compounds of formula (I) according to claim 1, wherein
R¹ and R⁴ represent hydrogen;
R² and R³ represent C1-C4 alkoxy; and
R⁵ represents a 2-phenylethyl group in which the phenyl group carries one or two substituents, each independently selected from trifluoromethyl or halogen;
and pharmaceutically acceptable salts thereof.

5. Compounds of formula (I) according to claim 1, wherein
R¹ and R⁴ represent hydrogen;
R² and R³ represent C1-C4 alkoxy; and
R⁵ represents a 2-phenylethyl group in which the phenyl group carries one or two substituents, each independently selected from difluoromethoxy or halogen;
and pharmaceutically acceptable salts thereof.

6. Compounds of formula (I) according to claim 1, wherein
R¹ and R⁴ represent hydrogen;
R² and R³ represent C1-C4 alkoxy; and
R⁵ represents a 2-phenylethyl group in which the phenyl group carries one or two substituents, each independently selected from trifluoromethoxy or halogen;
and pharmaceutically acceptable salts thereof.

7. Compounds according to claim 1 selected from the group consisting of:
2-{1-[2-(3,4-Difluoro-phenyl)-ethyl]-6,7-dimethoxy-3,4-dihydro-1H-isoquinolin-2-yl}-2-phenyl-acetamide;
2-{1-[2-(2,3-Difluoro-phenyl)-ethyl]-6,7-dimethoxy-3,4-dihydro-1H-isoquinolin-2-yl}-2-phenyl-acetamide;
2-{6,7-Dimethoxy-1-[2-(4-trifluoromethyl-phenyl)-ethyl]-3,4-dihydro-1H-isoquinolin-2-yl}-2-phenyl-acetamide;
2-{6,7-Dimethoxy-1-[2-(3-methoxy-phenyl)-ethyl]-3,4-dihydro-1H-isoquinolin-2-yl}-2-phenyl-acetamide;
2-{1-[2-(2,5-Difluoro-phenyl)-ethyl]-6,7-dimethoxy-3,4-dihydro-1H-isoquinolin-2-yl}-2-phenyl-acetamide;
2-{6,7-Dimethoxy-1-[2-(2-methoxy-phenyl)-ethyl]-3,4-dihydro-1H-isoquinolin-2-yl}-2-phenyl-acetamide;
2-{1-[2-(4-Fluoro-phenyl)-ethyl]-6,7-dimethoxy-3,4-dihydro-1H-isoquinolin-2-yl}-2-phenyl-acetamide;
2-{1-[2-(3,4-Dimethoxy-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2-methyl-5-trifluoromethyl-phenyl)-ethyl]-3,4-dihydro-1H-isoquinolin-2-yl}-2-phenyl-acetamide;
(R)-2-{(S)-1-[2-(3-Chloro-2-fluoro-phenyl)-ethyl]-6,7-dimethoxy-3,4-dihydro-1H-isoquinolin-2-yl}-2-phenyl-acetamide;
(R)-2-{(S)-1-[2-(3-Fluoro-4-methyl-phenyl)-ethyl]-6,7-dimethoxy-3,4-dihydro-1H-isoquinolin-2-yl}-2-phenyl-acetamide;
(R)-2-{(S)-1-[2-(4-Fluoro-2-trifluoromethyl-phenyl)-ethyl]-6,7-dimethoxy-3,4-dihydro-1H-isoquinolin-2-yl}-2-phenyl-acetamide;
(R)-2-{(S)-1-[2-(5-Fluoro-2-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Fluoro-5-trifluoromethyl-phenyl)-ethyl]-6,7-dimethoxy-3,4-dihydro-1H-isoquinolin-2-yl}-2-phenyl-acetamide;
(R)-2-{(S)-1-[2-(3-Fluoro-4-trifluoromethyl-phenyl)-ethyl]-6,7-dimethoxy-3,4-dihydro-1H-isoquinolin-2-yl}-2-phenyl-acetamide;
(R)-2-{(S)-1-[2-(4-Fluoro-3-methyl-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2,3,5-trifluoro-phenyl)-ethyl]-3,4-dihydro-1H-isoquinolin-2-yl}-2-phenyl-acetamide;
(R)-2-{(S)-1-[2-(4-Fluoro-3-trifluoromethyl-phenyl)-ethyl]-6,7-dimethoxy-3,4-dihydro-1H-isoquinolin-2-yl}-2-phenyl-acetamide;
(R)-2-{(S)-1-[2-(3-Fluoro-5-trifluoromethyl-phenyl)-ethyl]-6,7-dimethoxy-3,4-dihydro-1H-isoquinolin-2-yl}-2-phenyl-acetamide;
(R)-2-{(S)-1-[2-(5-Chloro-2-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Fluoro-3-trifluoromethyl-phenyl)-ethyl]-6,7-dimethoxy-3,4-dihydro-1H-isoquinolin-2-yl}-2-phenyl-acetamide;
(R)-2- {(S)-1-[2-(2-Fluoro-4-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Difluoromethoxy-phenyl)-ethyl]-6,7-dimethoxy-3,4-dihydro-1H-isoquinolin-2-yl}-2-phenyl-acetamide;
(R)-2-{(S)-1-[2-(3-Fluoro-2-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Chloro-3-trifluoromethyl-phenyl)-ethyl]-6,7-dimethoxy-3,4-dihydro-1H-isoquinolin-2-yl}-2-phenyl-acetamide;
(R)-2-{(S)-1-[2-(2-Fluoro-6-trifluoromethyl-phenyl)-ethyl]-6,7-dimethoxy-3,4-dihydro-1H-isoquinolin-2-yl}-2-phenyl-acetamide;
(R)-2-{(S)-1-[2-(4-Chloro-3-trifluoromethyl-phenyl)-ethyl]-6,7-dimethoxy-3,4-dihydro-1H-isoquinolin-2-yl}-2-phenyl-acetamide;
(R)-2-{(S)-1-[2-(2,3-Difluoro-4-methyl-phenyl)-ethyl]-6,7-dimethoxy-3,4-dihydro-1H-isoquinolin-2-yl}-2-phenyl-acetamide;
(R)-2-{(S)-1-[2-(4-Difluoromethoxy-phenyl)-ethyl]-6,7-dimethoxy-3,4-dihydro-1H-isoquinolin-2-yl}-2-phenyl-acetamide;
(R)-2-{(S)-1-[2-(3,4-Dimethyl-phenyl)-ethyl]-6,7-dimethoxy-3,4-dihydro-1H-isoquinolin-2-yl}-2-phenyl-acetamide;
(R)-2-{(S)-1-[2-(3-Chloro-4-methyl-phenyl)-ethyl]-6,7-dimethoxy-3,4-dihydro-1H-isoquinolin-2-yl}-2-phenyl-acetamide;
(R)-2-{(S)-1-[2-(2-Chloro-6-fluoro-phenyl)-ethyl]-6,7-dimethoxy-3,4-dihydro-1H-isoquinolin-2-yl}-2-phenyl-acetamide;
(R)-2-[(S)-6,7-Dimethoxy-1-(2-o-tolyl-ethyl)-3,4-dihydro-1H-isoquinolin-2-yl]-2-phenyl-acetamide;
(R)-2-[(S)-6,7-Dimethoxy-1-(2-m-tolyl-ethyl)-3,4-dihydro-1H-isoquinolin-2-yl]-2-phenyl-acetamide;
(R)-2-[(S)-6,7-Dimethoxy-1-(2-p-tolyl-ethyl)-3,4-dihydro-1H-isoquinolin-2-yl]-2-phenyl-acetamide;
(R)-2-{(S)-1-[2-(2-Fluoro-phenyl)-ethyl]-6,7-dimethoxy-3,4-dihydro-1H-isoquinolin-2-yl}-2-phenyl-acetamide;
(R)-2-{(S)-1-[2-(3-Fluoro-phenyl)-ethyl]-6,7-dimethoxy-3,4-dihydro-1H-isoquinolin-2-yl}-2-phenyl-acetamide;
(R)-2-{(S)-1-[2-(3,4-Dichloro-phenyl)-ethyl]-6,7-dimethoxy-3,4-dihydro-1H-isoquinolin-2-yl}-2-phenyl-acetamide;
(R)-2-{(S)-1-[2-(3,5-Dimethyl-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2-trifluoromethyl-phenyl)-ethyl]-3,4-dihydro-1H-isoquinolin-2-yl}-2-phenyl-acetamide;
(R)-2-{(S)-1-[2-(2,4-Difluoro-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2,3,6-trifluoro-phenyl)-ethyl]-3,4-dihydro-1H-isoquinolin-2-yl}-2-phenyl-acetamide;
(R)-2-{(S)-6,7-Dimethoxy-1-[2-(4-trifluoromethoxy-phenyl)-ethyl]-3,4-dihydro-1H-isoquinolin-2-yl}-2-phenyl-acetamide;
(R)-2-{(S)-1-[2-(2-Fluoro-3-methyl-phenyl)-ethyl]-6,7-dimethoxy-3,4-dihydro-1H-isoquinolin-2-yl}-2-phenyl-acetamide;
(R)-2-{(S)-1-[2-(4-Chloro-phenyl)-ethyl]-6,7-dimethoxy-3,4-dihydro-1H-isoquinolin-2-yl}-2-phenyl-acetamide;
(R)-2-{(S)-1-[2-(3-Chloro-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(3-trifluoromethoxy-phenyl)-ethyl]-3,4-dihydro-1H-isoquinolin-2-yl}-2-phenyl-acetamide;
(R)-2-{(S)-6,7-Dimethoxy-1-[2-(3,4,5-trifluoro-phenyl)-ethyl]-3,4-dihydro-1H-isoquinolin-2-yl}-2-phenyl-acetamide;
(R)-2-{(S)-6,7-Dimethoxy-1-[2-(3-trifluoromethyl-phenyl)-ethyl]-3,4-dihydro-1H-isoquinolin-2-yl}-2-phenyl-acetamide;
(R)-2-{(S)-6,7-Dimethoxy-1-[2-(2,3,4-trifluoro-phenyl)-ethyl]-3,4-dihydro-1H-isoquinolin-2-yl}-2-phenyl-acetamide;
(R)-2-{(S)-1-[2-(4-Bromo-2-fluoro-phenyl)-ethyl]-6,7-dimethoxy-3,4-dihydro-1H-isoquinolin-2-yl}-2-phenyl-acetamide;
(R)-2-{(S)-1-[2-(2,6-Difluoro-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2-trifluoromethoxy-phenyl)-ethyl]-3,4-dihydro-1H-isoquinolin-2-yl}-2-phenyl-acetamide;
(R)-2-{(S)-1-[2-(2,4-Dichloro-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(2,4,5-trifluoro-phenyl)-ethyl]-3,4-dihydro-1H-isoquinolin-2-yl}-2-phenyl-acetamide;
(R)-2-{(S)-1-[2-(3-Bromo-phenyl)-ethyl]-6,7-dimethoxy-3,4-dihydro-1H-isoquinolin-2-yl}-2-phenyl-acetamide;
(R)-2-{(S)-1-[2-(2-Bromo-phenyl)-ethyl]-6,7-dimethoxy-3,4-dihydro-1H-isoquinolin-2-yl}-2-phenyl-acetamide;
(R)-2-{(S)-1-[2-(4-Bromo-phenyl)-ethyl]-6,7-dimethoxy-3,4-dihydro-1H-isoquinolin-2-yl}-2-phenyl-acetamide;
(R)-2-{(S)-6,7-Dimethoxy-1-[2-(4-trifluoromethyl-phenyl)-ethyl]-3,4-dihydro-1H-isoquinolin-2-yl}-2-phenyl-acetamide;
(R)-2-{(S)-1-[2-(2,6-Dichloro-phenyl)-ethyl]-6,7-dimethoxy-3,4-dihydro-1H-isoquinolin-2-yl}-2-phenyl-acetamide; and
(R)-2-{(S)-1-[2-(4-tert-Butyl-phenyl)-ethyl]-6,7-dimethoxy-3,4-dihydro-1H-isoquinolin-2-yl}-2-phenyl-acetamide.

8. Pharmaceutical compositions for the treatment of disorders which are associated with the role of orexin, comprising one or more compounds of any one of claims 1 to 7, or a pharmaceutically acceptable salt thereof, and usual carrier materials and adjuvants.

9. Pharmaceutical compositions for the treatment of eating and sleep disorders, comprising one or more compounds of any one of claims 1 to 7, or a pharmaceutically acceptable salt thereof, and usual carrier materials and adjuvants.

10. The compounds of any one of claims 1 to 7, or a pharmaceutically acceptable salt thereof, for use as medicaments for the treatment of disorders which are associated with a role of orexins.

11. Use of a 1,2,3,4-tetrahydroisoquinoline derivative according to any of claims 1 to 7, or a pharmaceutically acceptable salt thereof, in the preparation of a medicament for the prevention or treatment of diseases selected from the group consisting of depression; anxiety; addictions; obsessive compulsive disorder; affective neurosis; depressive neurosis; anxiety neurosis; dysthymic disorder; mood disorder; sexual dysfunction; psychosexual dysfunction; schizophrenia; manic depression; delirium; dementia; severe mental retardation and dyskinesias such as Huntington's disease and Tourette syndrome; diabetes; appetite/taste disorders; vomiting/nausea; asthma; Parkinson's disease; Cushing's syndrome/disease; basophil adenoma; prolactinoma; hyperprolactinemia; hypopituitarism; hypophysis tumour/adenoma; hypothalamic diseases; inflammatory bowel disease; gastric dyskinesia; gastric ulcers; Froehlich's syndrome; hypophysis diseases, hypothalamic hypogonadism; Kallman's syndrome (anosmia, hyposmia); functional or psychogenic amenorrhea; hypothalamic hypothyroidism; hypothalamic-adrenal dysfunction; idiopathic hyperprolactinemia; hypothalamic disorders of growth hormone deficiency; idiopathic growth deficiency; dwarfism; gigantism; acromegaly; disturbed biological and circadian rhythms; sleep disturbances associated with diseases such as neurological disorders, neuropathic pain and restless leg syndrome; heart and lung diseases, acute and congestive heart failure; hypotension; hypertension; urinary retention; osteoporosis; angina pectoris; myocardial infarction; ischemic or haemorrhagic stroke; subarachnoid haemorrhage; ulcers; allergies; benign prostatic hypertrophy; chronic renal failure; renal disease; impaired glucose tolerance; migraine; pain; enhanced or exaggerated sensitivity to pain such as hyperalgesia, causalgia, and allodynia; acute pain; burn pain; atypical facial pain; neuropathic pain; back pain; complex regional pain syndrome I and II; arthritic pain; sports injury pain; pain related to infection, HIV; post-chemotherapy pain; post-stroke pain; post-operative pain; neuralgia; conditions associated with visceral pain such as irritable bowel syndrome, migraine and angina; urinary bladder incontinence e.g. urge incontinence; tolerance to narcotics or withdrawal from narcotics; sleep disorders; eating disorders; cardiovascular disorders; neurodegenerative disorders; sleep apnea; narcolepsy; insomnia; parasomnia; and neurodegenerative disorders including nosological entities such as disinhibition-dementia-parkinsonism-amyotrophy complex; pallido-ponto-nigral degeneration epilepsy, seizure disorders and other diseases related to general orexin system dysfunctions.

12. Use according to claim 11 wherein said diseases are selected from the group consisting of eating disorders or sleep disorders.

13. Use according to claim 12 wherein said eating disorders comprise metabolic dysfunction, dysregulated appetite control, compulsive obesities, emeto-bulimia or anorexia nervosa.

14. Use according to claim 12 wherein said sleep disorders comprise insomnias, narcolepsy and other disorders of excessive sleepiness, sleep-related dystonias, restless leg syndrome, sleep apneas, jet-lag syndrome, shift-work syndrome, delayed or advanced sleep phase syndrome.

15. Use of one or more compounds of any one of claims 1 to 7 for the preparation of a medicament, for use in combination with other pharmacologically active compounds comprising other orexin receptor antagonists, lipid lowering agents, anorectic agents, sleep inducing agents, antidepressants or other drugs beneficial for the prevention or treatment of disorders given in any one of claims 8 to 14.

## Patentansprüche

1. Verbindungen der Formel (I) 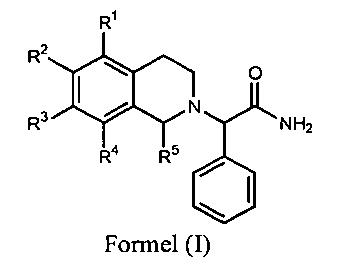 wobei
R¹, R², R³, R⁴ unabhängig Folgendes repräsentieren: Wasserstoff, Cyano, Halogen, Hydroxyl, C1-C4-Alkyl, C2-C4-Alkenyl, C1-C4-Alkoxy, Vinyloxy, Allyloxy, Trifluormethoxy, C3-C6-Cycloalkyloxy, oder R¹ und R² zusammen sowie R² und R³ zusammen oder R³ und R⁴ zusammen mit dem Phenylring, an den sie gebunden sind, einen fünf-, sechs- oder siebengliedrigen Ring bilden können, der ein oder zwei Sauerstoffatome enthält, und
R⁵ Folgendes repräsentiert: eine mono-, di- oder trisubstituierte Phenylethylgruppe, unabhängig substituiert durch C1-C4-Alkyl, C1-C4-Alkoxy, C2-C4-Alkenyl, Trifluormethyl, Trifluormethoxy, Difluormethoxy oder Halogen,
und pharmazeutisch akzeptable Salze davon.

2. Verbindungen der Formel (I) nach Anspruch 1, wobei
R¹ und R⁴ Wasserstoff repräsentieren,
R² und R³ C1-C4-Alkoxy repräsentieren und
R⁵ eine 2-Phenylethylgruppe repräsentiert, wobei die Phenylgruppe einen, zwei oder drei Substituenten trägt, die jeweils unabhängig ausgewählt sind aus C1-C4-Alkyl oder Halogen, und pharmazeutisch akzeptable Salze davon.

3. Verbindungen der Formel (I) nach Anspruch 1, wobei
R¹ und R⁴ Wasserstoff repräsentieren,
R² und R³ C1-C4-Alkoxy repräsentieren und
R⁵ eine 2-Phenylethylgruppe repräsentiert, wobei die Phenylgruppe einen oder zwei Substituenten trägt, die jeweils unabhängig ausgewählt sind aus C1-C4-Alkoxy oder Halogen,
und pharmazeutisch akzeptable Salze davon.

4. Verbindungen der Formel (I) nach Anspruch 1, wobei
R¹ und R⁴ Wasserstoff repräsentieren,
R² und R³ C1-C4-Alkoxy repräsentieren und
R⁵ eine 2-Phenylethylgruppe repräsentiert, wobei die Phenylgruppe einen oder zwei Substituenten trägt, die jeweils unabhängig ausgewählt sind aus Trifluormethyl oder Halogen,
und pharmazeutisch akzeptable Salze davon.

5. Verbindungen der Formel (I) nach Anspruch 1, wobei
R¹ und R⁴ Wasserstoff repräsentieren,
R² und R³ C1-C4-Alkoxy repräsentieren und
R⁵ eine 2-Phenylethylgruppe repräsentiert, wobei die Phenylgruppe einen oder zwei Substituenten trägt, die jeweils unabhängig ausgewählt sind aus Difluormethoxy oder Halogen,
und pharmazeutisch akzeptable Salze davon.

6. Verbindungen der Formel (I) nach Anspruch 1, wobei
R¹ und R⁴ Wasserstoff repräsentieren,
R² und R³ C1-C4-Alkoxy repräsentieren und
R⁵ eine 2-Phenylethylgruppe repräsentiert, wobei die Phenylgruppe einen oder zwei Substituenten trägt, die jeweils unabhängig ausgewählt sind aus Trifluormethoxy oder Halogen,
und pharmazeutisch akzeptable Salze davon.

7. Verbindungen nach Anspruch 1, ausgewählt aus der Gruppe bestehend aus:
2-{1-[2-(3,4-Difluor-phenyl)-ethyl]-6,7-dimethoxy-3,4-dihydro-1H-isochinolin-2-yl}-2-phenyl-acetamid;
2-{1-[2-(2,3-Difluor-phenyl)-ethyl]-6,7-dimethoxy-3,4-dihydro-1H-isochinolin-2-yl}-2-phenyl-acetamid;
2-{6,7-Dimethoxy-1-[2-(4-trifluormethyl-phenyl)-ethyl]-3,4-dihydro-1H-isochinolin-2-yl}-2-phenyl-acetamid;
2-{6,7-Dimethoxy-1-[2-(3-methoxy-phenyl)-ethyl]-3,4-dihydro-1H-isochinolin-2-yl}-2-phenyl-acetamid;
2-{1-[2-(2,5-Difluor-phenyl)-ethyl]-6,7-dimethoxy-3,4-dihydro-1H-isochinolin-2-yl}-2-phenyl-acetamid;
2-{6,7-Dimethoxy-1-[2-(2-methoxy-phenyl)-ethyl]-3,4-dihydro-1H-isochinolin-2-yl}-2-phenyl-acetamid;
2-{1-[2-(4-Fluor-phenyl)-ethyl]-6,7-dimethoxy-3,4-dihydro-1H-isochinolin-2-yl}-2-phenyl-acetamid;
2-{1-[2-(3,4-Dimethoxy-phenyl)-ethyl]-6,7-dimethoxy-3,4-dihydro-1H-isochinolin-2-yl}-2-phenyl-acetamid;
(R)-2-{(S)-6,7-Dimethoxy-1-[2-(2-methyl-5-trifluormethyl-phenyl)-ethyl]-3,4-dihydro-1H-isochinolin-2-yl}-2-phenyl-acetamid;
(R)-2-{(S)-1-[2-(3-Chlor-2-fluor-phenyl)-ethyl]-6,7-dimethoxy-3,4-dihydro-1H-isochinolin-2-yl}-2-phenyl-acetamid;
(R)-2-{(S)-1-[2-(3-Fluor-4-methyl-phenyl)-ethyl]-6,7-dimethoxy-3,4-dihydro-1H-isochinolin-2-yl}-2-phenyl-acetamid;
(R)-2-{(S)-1-[2-(4-Fluor-2-trifluormethyl-phenyl)-ethyl]-6,7-dimethoxy-3,4-dihydro-1H-isochinolin-2-yl}-2-phenyl-acetamid;
(R)-2-{(S)-1-[2-(5-Fluor-2-trifluormethyl-phenyl)-ethyl]-6,7-dimethoxy-3,4-dihydro-1H-isochinolin-2-yl}-2-phenyl-acetamid;
(R)-2-{(S)-1-[2-(2-Fluor-5-trifluormethyl-phenyl)-ethyl]-6,7-dimethoxy-3,4-dihydro-1H-isochinolin-2-yl}-2-phenyl-acetamid;
(R)-2-{(S)-1-[2-(3-Fluor-4-trifluormethyl-phenyl)-ethyl]-6,7-dimethoxy-3,4-dihydro-1H-isochinolin-2-yl}-2-phenyl-acetamid;
(R)-2-{(S)-1-[2-(4-Fluor-3-methyl-phenyl)-ethyl]-6,7-dimethoxy-3,4-dihydro-1H-isochinolin-2-yl}-2-phenyl-acetamid;
(R)-2-{(S)-6,7-Dimethoxy-1-[2-(2,3,5-trifluor-phenyl)-ethyl]-3,4-dihydro-1H-isochinolin-2-yl}-2-phenyl-acetamid;
(R)-2-{(S)-1-[2-(4-Fluor-3-trifluormethyl-phenyl)-ethyl]-6,7-dimethoxy-3,4-dihydro-1H-isochinolin-2-yl}-2-phenyl-acetamid;
(R)-2-{(S)-1-[2-(3-Fluor-5-trifluormethyl-phenyl)-ethyl]-6,7-dimethoxy-3,4-dihydro-1H-isochinolin-2-yl}-2-phenyl-acetamid;
(R)-2-{(S)-1-[2-(5-Chlor-2-trifluormethyl-phenyl)-ethyl]-6,7-dimethoxy-3,4-dihydro-1H-isochinolin-2-yl}-2-phenyl-acetamid;
(R)-2-{(S)-1-[2-(2-Fluor-3-trifluormethyl-phenyl)-ethyl]-6,7-dimethoxy-3,4-dihydro-1H-isochinolin-2-yl}-2-phenyl-acetamid;
(R)-2-{(S)-1-[2-(2-Fluor-4-trifluormethyl-phenyl)-ethyl]-6,7-dimethoxy-3,4-dihydro-1H-isochinolin-2-yl}-2-phenyl-acetamid;
(R)-2-{(S)-1-[2-(2-Difluormethoxy-phenyl)-ethyl]-6,7-dimethoxy-3,4-dihydro-1H-isochinolin-2-yl}-2-phenyl-acetamid;
(R)-2-{(S)-1-[2-(3-Fluor-2-trifluormethyl-phenyl)-ethyl]-6,7-dimethoxy-3,4-dihydro-1H-isochinolin-2-yl}-2-phenyl-acetamid;
(R)-2-{(S)-1-[2-(2-Chlor-3-trifluormethyl-phenyl)-ethyl]-6,7-dimethoxy-3,4-dihydro-1H-isochinolin-2-yl}-2-phenyl-acetamid;
(R)-2-{(S)-1-[2-(2-Fluor-6-trifluormethyl-phenyl)-ethyl]-6,7-dimethoxy-3,4-dihydro-1H-isochinolin-2-yl}-2-phenyl-acetamid;
(R)-2-{(S)-1-[2-(4-Chlor-3-trifluormethyl-phenyl)-ethyl]-6,7-dimethoxy-3,4-dihydro-1H-isochinolin-2-yl}-2-phenyl-acetamid;
(R)-2-{(S)-1-[2-(2,3-Difluor-4-methyl-phenyl)-ethyl]-6,7-dimethoxy-3,4-dihydro-1H-isochinolin-2-yl}-2-phenyl-acetamid;
(R)-2-{(S)-1-[2-(4-Difluormethoxy-phenyl)-ethyl]-6,7-dimethoxy-3,4-dihydro-1H-isochinolin-2-yl}-2-phenyl-acetamid;
(R)-2-{(S)-1-[2-(3,4-Dimethyl-phenyl)-ethyl]-6,7-dimethoxy-3,4-dihydro-1H-isochinolin-2-yl}-2-phenyl-acetamid;
(R)-2-{(S)-1-[2-(3-Chlor-4-methyl-phenyl)-ethyl]-6,7-dimethoxy-3,4-dihydro-1H-isochinolin-2-yl}-2-phenyl-acetamid;
(R)-2-{(S)-1-[2-(2-Chlor-6-fluor-phenyl)-ethyl]-6,7-dimethoxy-3,4-dihydro-1H-isochinolin-2-yl}-2-phenyl-acetamid;
(R)-2-[(S)-6,7-Dimethoxy-1-(2-o-tolyl-ethyl)-3,4-dihydro-1H-isochinolin-2-yl]-2-phenyl-acetamid;
(R)-2-[(S)-6,7-Dimethoxy-1-(2-m-tolyl-ethyl)-3,4-dihydro-1H-isochinolin-2-yl]-2-phenyl-acetamid;
(R)-2-[(S)-6,7-Dimethoxy-1-(2-p-tolyl-ethyl)-3,4-dihydro-1H-isochinolin-2-yl]-2-phenyl-acetamid;
(R)-2-{(S)-1-[2-(2-Fluor-phenyl)-ethyl]-6,7-dimethoxy-3,4-dihydro-1H-isochinolin-2-yl}-2-phenyl-acetamid;
(R)-2-{(S)-1-[2-(3-Fluor-phenyl)-ethyl]-6,7-dimethoxy-3,4-dihydro-1H-isochinolin-2-yl}-2-phenyl-acetamid;
(R)-2-{(S)-1-[2-(3,4-Dichlor-phenyl)-ethyl]-6,7-dimethoxy-3,4-dihydro-1H-isochinolin-2-yl}-2-phenyl-acetamid;
(R)-2-{(S)-1-[2-(3,5-Dimethyl-phenyl)-ethyl]-6,7-dimethoxy-3,4-dihydro-1H-isochinolin-2-yl}-2-phenyl-acetamid;
(R)-2-{(S)-6,7-Dimethoxy-1-[2-(2-trifluormethyl-phenyl)-ethyl]-3,4-dihydro-1H-isochinolin-2-yl}-2-phenyl-acetamid;
(R)-2-{(S)-1-[2-(2,4-Difluor-phenyl)-ethyl]-6,7-dimethoxy-3,4-dihydro-1H-isochinolin-2-yl}-2-phenyl-acetamid;
(R)-2-{(S)-6,7-Dimethoxy-1-[2-(2,3,6-trifluor-phenyl)-ethyl]-3,4-dihydro-1H-isochinolin-2-yl}-2-phenyl-acetamid;
(R)-2-{(S)-6,7-Dimethoxy-1-[2-(4-trifluormethoxy-phenyl)-ethyl]-3,4-dihydro-1H-isochinolin-2-yl}-2-phenyl-acetamid;
(R)-2-{(S)-1-[2-(2-Fluor-3-methyl-phenyl)-ethyl]-6,7-dimethoxy-3,4-dihydro-1H-isochinolin-2-yl}-2-phenyl-acetamid;
(R)-2-{(S)-1-[2-(4-Chlor-phenyl)-ethyl]-6,7-dimethoxy-3,4-dihydro-1H-isochinolin-2-yl}-2-phenyl-acetamid;
(R)-2-{(S)-1-[2-(3-Chlor-phenyl)-ethyl]-6,7-dimethoxy-3,4-dihydro-1H-isochinolin-2-yl}-2-phenyl-acetamid;
(R)-2-{(S)-6,7-Dimethoxy-1-[2-(3-trifluormethoxy-phenyl)-ethyl]-3,4-dihydro-1H-isochinolin-2-yl}-2-phenyl-acetamid;
(R)-2-{(S)-6,7-Dimethoxy-1-[2-(3,4,5-trifluor-phenyl)-ethyl]-3,4-dihydro-1H-isochinolin-2-yl}-2-phenyl-acetamid;
(R)-2-{(S)-6,7-Dimethoxy-1-[2-(3-trifluormethyl-phenyl)-ethyl]-3,4-dihydro-1H-isochinolin-2-yl}-2-phenyl-acetamid;
(R)-2-{(S)-6,7-Dimethoxy-1-[2-(2,3,4-trifluor-phenyl)-ethyl]-3,4-dihydro-1H-isochinolin-2-yl}-2-phenyl-acetamid;
(R)-2-{(S)-1-[2-(4-Brom-2-fluor-phenyl)-ethyl]-6,7-dimethoxy-3,4-dihydro-1H-isochinolin-2-yl}-2-phenyl-acetamid;
(R)-2-{(S)-1-[2-(2,6-Difluor-phenyl)-ethyl]-6,7-dimethoxy-3,4-dihydro-1H-isochinolin-2-yl}-2-phenyl-acetamid;
(R)-2-{(S)-6,7-Dimethoxy-1-[2-(2-trifluormethoxy-phenyl)-ethyl]-3,4-dihydro-1H-isochinolin-2-yl}-2-phenyl-acetamid;
(R)-2-{(S)-1-[2-(2,4-Dichlor-phenyl)-ethyl]-6,7-dimethoxy-3,4-dihydro-1H-isochinolin-2-yl}-2-phenyl-acetamid;
(R)-2-{(S)-6,7-Dimethoxy-1-[2-(2,4,5-trifluor-phenyl)-ethyl]-3,4-dihydro-1H-isochinolin-2-yl}-2-phenyl-acetamid;
(R)-2-{(S)-1-[2-(3-Brom-phenyl)-ethyl]-6,7-dimethoxy-3,4-dihydro-1H-isochinolin-2-yl}-2-phenyl-acetamid;
(R)-2-{(S)-1-[2-(2-Brom-phenyl)-ethyl]-6,7-dimethoxy-3,4-dihydro-1H-isochinolin-2-yl-2-phenyl-acetamid;
(R)-2-{(S)-1-[2-(4-Brom-phenyl)-ethyl]-6,7-dimethoxy-3,4-dihydro-1H-isochinolin-2-yl}-2-phenyl-acetamid;
(R)-2-{(S)-6,7-Dimethoxy-1-[2-(4-trifluormethyl-phenyl)-ethyl]-3,4-dihydro-1H-isochinolin-2-yl}-2-phenyl-acetamid;
(R)-2-{(S)-1-[2-(2,6-Dichlor-phenyl)-ethyl]-6,7-dimethoxy-3,4-dihydro-1H-isochinolin-2-yl}-2-phenyl-acetamid; und
(R)-2-{(S)-1-[2-(4-tert-Butyl-phenyl)-ethyl]-6,7-dimethoxy-3,4-dihydro-1H-isochinolin-2-yl}-2-phenyl-acetamid.

8. Pharmazeutische Zusammensetzungen zur Behandlung von Störungen, die mit der Rolle von Orexin zusammenhängen, die eine oder mehrere Verbindungen nach einem der Ansprüche 1 bis 7 oder ein pharmazeutisch akzeptables Salz davon und übliche Trägermaterialien und Adjuvanzien umfassen.

9. Pharmazeutische Zusammensetzungen zur Behandlung von Ess- und Schlafstörungen, die eine oder mehrere Verbindungen nach einem der Ansprüche 1 bis 7 oder ein pharmazeutisch akzeptables Salz davon und übliche Trägermaterialien und Adjuvanzien umfassen.

10. Verbindungen nach einem der Ansprüche 1 bis 7 oder ein pharmazeutisch akzeptables Salz davon zur Verwendung als Medikamente zur Behandlung von Störungen, die mit einer Rolle von Orexinen zusammenhängen.

11. Verwendung eines 1,2,3,4-Tetrahydroisochinolinderivats nach einem der Ansprüche 1 bis 7 oder eines pharmazeutisch akzeptablen Salzes davon zur Herstellung eines Medikaments zur Vorbeugung gegen oder Behandlung von Krankheiten, die ausgewählt sind aus der Gruppe bestehend aus Depressionen; Angst; Sucht; obsessive Zwangsstörungen; Affektneurosen; depressive Neurosen; Angstneurosen; Dysthymiestörungen; Gemütszustandsstörungen; Sexualdysfunktion; psychosexueller Dysfunktion; Schizophrenie; manische Depression; Delirium; Demenz; starke geistige Unterentwicklung und Dyskinesien wie die Huntingtonsche Krankheit und das Tourette-Syndrom; Diabetes; Appetit-/Geschmacksstörungen; Erbrechen/Übelkeit; Asthma; die Parkinsonsche Krankheit; das/die Cushingschen Syndrom/Krankheit; basophiles Adenom; Prolaktinom; Hyperprolaktinämie; Hypopituitarismus; Hypophysentumor/-adenom; hypothalamische Krankheiten; entzündliche Darmerkrankung; gastrische Dyskinesie; Magengeschwüre; Fröhlichsches Syndrom; Hypophysenkrankheiten; hypothalamischer Hypogonadismus; Kallman-Syndrom (Anosmie, Hyposmie); funktionelle oder psychogene Amenorrhoe; hypothalamischer Hypothyreoidismus; hypothalamischadrenale Dysfunktion; idiopathische Hyperprolaktinämie; durch hypothalamische Störungen bedingte Wachstumshormondefizienz; idiopathische Wachstumsdefizienz; Zwergwuchs; Riesenwuchs; Akromegalie; gestörte biologische und zirkadische Rhythmen; Schlafstörungen in Verbindung mit Krankheiten wie neurologische Störungen, neuropathische Schmerzen und das Syndrom der unruhigen Beine; Herz- und Lungenkrankheiten, akute und kongestive Herzinsuffizienz; Hypotension; Hypertension; Urinretention; Osteoporose; Angina pectoris; Myokardinfarzierung; ischämischer oder hämorrhagischer Anfall; Subarachnoidalblutung; Geschwüre; Allergien; gutartige Prostatahypertrophie; chronisches Nierenversagen; Nierenerkrankung; beeinträchtigte Glucosetoleranz; Migräne; Schmerzen; verstärkte oder übertriebene Empfindlichkeit gegenüber Schmerzen wie Hyperalgesie, Kausalgie und Allodynie; akute Schmerzen; Verbrennungsschmerzen; atypische Gesichtsschmerzen; neuropathische Schmerzen; Rückenschmerzen; komplexes regionales Schmerzsyndrom I und II; arthritische Schmerzen; Sportverletzungsschmerzen; Schmerzen in Verbindung mit Infektionen, HIV; Schmerzen nach einer Chemotherapie; Schmerzen nach einem Hirnschlag; postoperative Schmerzen; Neuralgie; Leiden in Verbindung mit viszeralen Schmerzen wie Reizdarmsyndrom, Migräne und Angina; Harnblaseninkontinenz, z.B. Dranginkontinenz; Narkotikatoleranz oder Narkotikaentzug; Schlafstörungen; Essstörungen; Herz-Kreislauf-Erkrankungen; neurodegenerative Störungen; Schlafapnoe; Narkolepsie; Insomnie; Parasomnie und neurodegenerative Störungen wie nosologische Entitäten wie Disinhibition-Demenz-Parkinsonismus-Amyothrophie-Komplex; Pallido-Ponto-Nigral-Degenerationsepilepsie; Anfallsstörungen und andere Krankheiten, die mit allgemeinen Orexinsystemdysfunktionen zusammenhängen.

12. Verwendung nach Anspruch 11, wobei die genannten Krankheiten aus der Gruppe bestehend aus Essstörungen oder Schlafstörungen ausgewählt sind.

13. Verwendung nach Anspruch 12, wobei die genannten Essstörungen Stoffwechselfunktionsstörungen, fehlregulierte Appetitkontrolle, kompulsive Fettleibigkeit, Emeto-Bulimie oder Anorexia nervosa umfassen.

14. Verwendung nach Anspruch 12, wobei die genannten Schlafstörungen Insomnien, Narkolepsie und andere Störungen übermäßiger Schläfrigkeit, schlafbedingte Dystonien, das Syndrom der unruhigen Beine, Schlafapnoe, das Jetlag-Syndrom, das Schichtarbeitersyndrom, das verzögerte oder vorverlagerte Schlafphasensyndrom umfassen.

15. Verwendung von einer oder mehreren Verbindungen nach einem der Ansprüche 1 bis 7 zur Herstellung eines Medikaments zur Verwendung in Kombination mit anderen pharmakologisch aktiven Verbindungen, umfassend andere Orexinrezeptor-Antagonisten, Lipidsenker, Anorektika, schlafinduzierende Mittel, Antidepressiva oder andere Arzneimittel zur Vorbeugung gegen oder Behandlung von in einem der Ansprüche 8 bis 14 genannten Störungen.

## Revendications

1. Composés de formule (I) 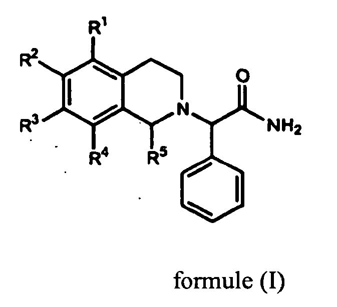 où
R¹, R², R³, R⁴ représentent indépendamment un des suivants : hydrogène ; cyano ; halogène ; hydroxyle ; alkyle en C1-C4 ; alkényle en C2-C4 ; alkoxy en C1-C4 ; vinyloxy ; allyloxy ; trifluorométhoxy ; cycloalkyloxy en C3-C6 ; ou R¹ et R² ensemble ainsi que R² et R³ ensemble ou R³ et R⁴ ensemble peuvent former, avec l'anneau phényle auquel ils sont rattachés, un cycle à cinq, six ou sept chaînons qui contient un ou deux atomes d'oxygène ; et
R⁵ représente un groupement phényl-éthyle mono-, di- ou trisubstitué, chaque substituant étant indépendamment sélectionné parmi un alkyle en C1-C4, alkoxy en C 1-C4, alkényle en C2-C4, trifluorométhyle, trifluorométhoxy, difluorométhoxy ou halogène ;
et sels pharmaceutiquement acceptables de ceux-ci.

2. Composés de formule (I) selon la revendication 1, où
R¹ et R⁴ représentent un hydrogène ;
R² et R³ représentent un alkoxy en C1-C4 ; et
R⁵ représente un groupement 2-phényl-éthyle dans lequel le groupement phényle porte un, deux ou trois substituants qui sont chacun indépendamment sélectionnés parmi un alkyle en C1-C4 ou un halogène ;
et sels pharmaceutiquement acceptables de ceux-ci.

3. Composés de formule (I) selon la revendication 1, où
R¹ et R⁴ représentent un hydrogène ;
R² et R³ représentent un alkoxy en C1-C4 ; et
R⁵ représente un groupement 2-phényl-éthyle dans lequel le groupement phényle porte un ou deux substituants qui sont chacun indépendamment sélectionnés parmi un alkyle en C1-C4 ou un halogène ;
et sels pharmaceutiquement acceptables de ceux-ci.

4. Composés de formule (I) selon la revendication 1, où
R¹ et R⁴ représentent un hydrogène ;
R² et R³ représentent un alkoxy en C1-C4; et
R⁵ représente un groupement 2-phényl-éthyle dans lequel le groupement phényle porte un ou deux substituants qui sont chacun indépendamment sélectionnés parmi un trifluorométhyle ou un halogène ;
et sels pharmaceutiquement acceptables de ceux-ci.

5. Composés de formule (I) selon la revendication 1, où
R¹ et R⁴ représentent un hydrogène ;
R² et R³ représentent un alkoxy en C1-C4 ; et
R⁵ représente un groupement 2-phényl-éthyle dans lequel le groupement phényle porte un ou deux substituants qui sont chacun indépendamment sélectionnés parmi un difluorométhoxy ou un halogène ;
et sels pharmaceutiquement acceptables de ceux-ci.

6. Composés de formule (I) selon la revendication 1, où
R¹ et R⁴ représentent un hydrogène ;
R² et R³ représentent un alkoxy en C1-C4 ; et
R⁵ représente un groupement 2-phényl-éthyle dans lequel le groupement phényle porte un ou deux substituants qui sont chacun indépendamment sélectionnés parmi un trifluorométhoxy ou un halogène ;
et sels pharmaceutiquement acceptables de ceux-ci.

7. Composés selon la revendication 1, qui sont sélectionnés dans le groupe consistant en :
2-{1-[2-(3,4-difluoro-phényl)-éthyl]-6,7-diméthoxy-3,4-dihydro-1H-isoquinolin-2-yl}-2-phényl-acétamide ;
2-{1-[2-(2,3-difluoro-phényl)-éthyl]-6,7-diméthoxy-3,4-dihydro-1H-isoquinolin-2-yl}-2-phényl-acétamide ;
2-{6,7-diméthoxy-1-[2-(4-trifluorométhyl-phényl)-éthyl]-3,4-dihydro-1H-isoquinolin-2-yl}-2-phényl-acétamide ;
2-{6,7-diméthoxy-1-[2-(3-méthoxy-phényl)-éthyl]-3,4-dihydro-1H-isoquinolin-2-yl}-2-phényl-acétamide ;
2-{1-[2-(2,5-difluoro-phényl)-éthyl]-6,7-diméthoxy-3,4-dihydro-1H-isoquinolin-2-yl}-2-phényl-acétamide ;
2-{6,7-diméthoxy-1-[2-(2-méthoxy-phényl)-éthyl]-3,4-dihydro-1H-isoquinolin-2-yl}-2-phényl-acétamide ;
2-{1-[2-(4-fluoro-phényl)-éthyl]-6,7-diméthoxy-3,4-dihydro-1H-isoquinolin-2-yl}-2-phényl-acétamide ;
2-{1-[2-(3,4-diméthoxy-phényl)-éthyl]-6,7-diméthoxy-3,4-dihydro-1H-isoquinolin-2-yl}-2-phényl-acétamide ;
(R)-2-{(S)-6,7-diméthoxy-1-[2-(2-méthyl-5-trifluorométhyl-phényl)-éthyl]-3,4-dihydro-1H-isoquinolin-2-yl}-2-phényl-acétamide ;
(R)-2-{(S)-1-[2-(3-chloro-2-fluoro-phényl)-éthyl]-6,7-diméthoxy-3,4-dihydro-1H-isoquinolin-2-yl}-2-phényl-acétamide ;
(R)-2-{(S)-1-[2-(3-fluoro-4-méthyl-phényl)-éthyl]-6,7-diméthoxy-3,4-dihydro-1H-isoquinolin-2-yl}-2-phényl-acétamide ;
(R)-2-{(S)-1-[2-(4-fluoro-2-trifluorométhyl-phényl)-éthyl]-6,7-diméthoxy-3,4-dihydro-1H-isoquinolin-2-yl}-2-phényl-acétamide ;
(R)-2-{(S)-1-[2-(5-fluoro-2-trifluorométhyl-phényl)-éthyl]-6,7-diméthoxy-3,4-dihydro-1H-isoquinolin-2-yl}-2-phényl-acétamide ;
(R)-2-{(S)-1-[2-(2-fluoro-5-trifluorométhyl-phényl)-éthyl]-6,7-diméthoxy-3,4-dihydro-1H-isoquinolin-2-yl}-2-phényl-acétamide ;
(R)-2-{(S)-1-[2-(3-fluoro-4-trifluorométhyl-phényl)-éthyl]-6,7-diméthoxy-3,4-dihydro-1H-isoquinolin-2-yl}-2-phényl-acétamide ;
(R)-2-{(S)-1-[2-(4-fluoro-3-méthyl-phényl)-éthyl]-6,7-diméthoxy-3,4-dihydro-1H-isoquinolin-2-yl}-2-phényl-acétamide ;
(R)-2-{(S)-6,7-diméthoxy-1-[2-(2,3,5-trifluoro-phényl)-éthyl]-3,4-dihydro-1H-isoquinolin-2-yl}-2-phényl-acétamide ;
(R)-2-{(S)-1-[2-(4-fluoro-3-trifluorométhyl-phényl)-éthyl]-6,7-diméthoxy-3,4-dihydro-1H-isoquinolin-2-yl}-2-phényl-acétamide ;
(R)-2-{(S)-1-[2-(3-fluoro-5-trifluorométhyl-phényl)-éthyl]-6,7-diméthoxy-3,4-dihydro-1H-isoquinolin-2-yl}-2-phényl-acétamide ;
(R)-2-{(S)-1-[2-(5-chloro-2-trifluorométhyl-phényl)-éthyl]-6,7-diméthoxy-3,4-dihydro-1H-isoquinolin-2-yl}-2-phényl-acétamide ;
(R)-2-{(S)-1-[2-(2-fluoro-3-trifluorométhyl-phényl)-éthyl]-6,7-diméthoxy-3,4-dihydro-1H-isoquinolin-2-yl}-2-phényl-acétamide ;
(R)-2-{(S)-1-[2-(2-fluoro-4-trifluorométhyl-phényl)-éthyl]-6,7-diméthoxy-3,4-dihydro-1H-isoquinolin-2-yl}-2-phényl-acétamide ;
(R)-2-{(S)-1-[2-(2-difluorométhoxy-phényl)-éthyl]-6,7-diméthoxy-3,4-dihydro-1H-isoquinolin-2-yl}-2-phényl-acétamide ;
(R)-2-{(S)-1-[2-(3-fluoro-2-trifluorométhyl-phényl)-éthyl]-6,7-diméthoxy-3,4-dihydro-1H-isoquinolin-2-yl}-2-phényl-acétamide ;
(R)-2-{(S)-1-[2-(2-chloro-3-trifluorométhyl-phényl)-éthyl]-6,7-diméthoxy-3,4-dihydro-1H-isoquinolin-2-yl}-2-phényl-acétamide ;
(R)-2-{(S)-1-[2-(2-fluoro-6-trifluorométhyl-phényl)-éthyl]-6,7-diméthoxy-3,4-dihydro-1H-isoquinolin-2-yl}-2-phényl-acétamide ;
(R)-2-{(S)-1-[2-(4-chloro-3-trifluorométhyl-phényl)-éthyl]-6,7-diméthoxy-3,4-dihydro-1H-isoquinolin-2-yl}-2-phényl-acétamide ;
(R)-2-{(S)-1-[2-(2,3-difluoro-4-méthyl-phényl)-éthyl]-6,7-diméthoxy-3,4-dihydro-1H-isoquinolin-2-yl}-2-phényl-acétamide ;
(R)-2-{(S)-1-[2-(4-difluorométhoxy-phényl)-éthyl]-6,7-diméthoxy-3,4-dihydro-1H-isoquinolin-2-yl}-2-phényl-acétamide ;
(R)-2-{(S)-1-[2-(3,4-diméthyl-phényl)-éthyl]-6,7-diméthoxy-3,4-dihydro-1H-isoquinolin-2-yl}-2-phényl-acétamide ;
(R)-2-{(S)-1-[2-(3-chloro-4-méthyl-phényl)-éthyl]-6,7-diméthoxy-3,4-dihydro-1H-isoquinolin-2-yl}-2-phényl-acétamide ;
(R)-2-{(S)-1-[2-(2-chloro-6-fluoro-phényl)-éthyl]-6,7-diméthoxy-3,4-dihydro-1H-isoquinolin-2-yl}-2-phényl-acétamide ;
(R)-2-[(S)-6,7-diméthoxy-1-(2-*o*-tolyl-éthyl)-3,4-dihydro-1H-isoquinolin-2-yl]-2-phényl-acétamide ;
(R)-2-[(S)-6,7-diméthoxy-1-(2-*m*-tolyl-éthyl)-3,4-dihydro-1H-isoquinolin-2-yl]-2-phényl-acétamide ;
(R)-2-[(S)-6,7-diméthoxy-1-(2-*p*-tolyl-éthyl)-3,4-dihydro-1H-isoquinolin-2-yl]-2-phényl-acétamide ;
(R)-2-{(S)-1-[2-(2-fluoro-phényl)-éthyl]-6,7-diméthoxy-3,4-dihydro-1H-isoquinolin-2-yl}-2-phényl-acétamide ;
(R)-2-{(S)-1-[2-(3-fluoro-phényl)-éthyl]-6,7-diméthoxy-3,4-dihydro-1H-isoquinolin-2-yl}-2-phényl-acétamide ;
(R)-2-{(S)-1-[2-(3,4-dichloro-phényl)-éthyl]-6,7-diméthoxy-3,4-dihydro-1H-isoquinolin-2-yl}-2-phényl-acétamide ;
(R)-2-{(S)-1-[2-(3,5-diméthyl-phényl)-éthyl]-6,7-diméthoxy-3,4-dihydro-1H-isoquinolin-2-yl}-2-phényl-acétamide ;
(R)-2-{(S)-6,7-diméthoxy-1-[2-(2-trifluorométhyl-phényl)-éthyl]-3,4-dihydro-1H-isoquinolin-2-yl}-2-phényl-acétamide ;
(R)-2-{(S)-1-[2-(2,4-difluoro-phényl)-éthyl]-6,7-diméthoxy-3,4-dihydro-1H-isoquinolin-2-yl}-2-phényl-acétamide ;
(R)-2-{(S)-6,7-diméthoxy-1-[2-(2,3,6-trifluoro-phényl)-éthyl]-3,4-dihydro-1H-isoquinolin-2-yl}-2-phényl-acétamide ;
(R)-2-{(S)-6,7-diméthoxy-1-[2-(4-trifluorométhoxy-phényl)-éthyl]-3,4-dihydro-1H-isoquinolin-2-yl}-2-phényl-acétamide ;
(R)-2-{(S)-1-[2-(2-fluoro-3-méthyl-phényl)-éthyl]-6,7-diméthoxy-3,4-dihydro-1H-isoquinolin-2-yl}-2-phényl-acétamide ;
(R)-2-{(S)-1-[2-(4-chloro-phényl)-éthyl]-6,7-diméthoxy-3,4-dihydro-1H-isoquinolin-2-yl}-2-phényl-acétamide ;
(R)-2-{(S)-1-[2-(3-chloro-phényl)-éthyl]-6,7-diméthoxy-3,4-dihydro-1H-isoquinolin-2-yl}-2-phényl-acétamide ;
(R)-2-{(S)-6,7-diméthoxy-1-[2-(3-trifluorométhoxy-phényl)-éthyl]-3,4-dihydro-1H-isoquinolin-2-yl}-2-phényl-acétamide ;
(R)-2-{(S)-6,7-diméthoxy-1-[2-(3,4,5-trifluoro-phényl)-éthyl]-3,4-dihydro-1H-isoquinolin-2-yl}-2-phényl-acétamide ;
(R)-2-{(S)-6,7-diméthoxy-1-[2-(3-trifluorométhyl-phényl)-éthyl]-3,4-dihydro-1H-isoquinolin-2-yl}-2-phényl-acétamide ;
(R)-2-{(S)-6,7-diméthoxy-1-[2-(2,3,4-trifluoro-phényl)-éthyl]-3,4-dihydro-1H-isoquinolin-2-yl}-2-phényl-acétamide ;
(R)-2-{(S)-1-[2-(4-bromo-2-fluoro-phényl)-éthyl]-6,7-diméthoxy-3,4-dihydro-1H-isoquinolin-2-yl}-2-phényl-acétamide ;
(R)-2-{(S)-1-[2-(2,6-difluoro-phényl)-éthyl]-6,7-diméthoxy-3,4-dihydro-1H-isoquinolin-2-yl}-2-phényl-acétamide ;
(R)-2-{(S)-6,7-diméthoxy-1-[2-(2-trifluorométhoxy-phényl)-éthyl]-3,4-dihydro-1H-isoquinolin-2-yl}-2-phényl-acétamide ;
(R)-2-{(S)-1-[2-(2,4-dichloro-phényl)-éthyl]-6,7-diméthoxy-3,4-dihydro-1H-isoquinolin-2-yl}-2-phényl-acétamide ;
(R)-2-{(S)-6,7-diméthoxy-1-[2-(2,4,5-trifluoro-phényl)-éthyl]-3,4-dihydro-1H-isoquinolin-2-yl}-2-phényl-acétamide ;
(R)-2-{(S)-1-[2-(3-bromo-phényl)-éthyl]-6,7-diméthoxy-3,4-dihydro-1H-isoquinolin-2-yl}-2-phényl-acétamide ;
(R)-2-{(S)-1-[2-(2-bromo-phényl)-éthyl]-6,7-diméthoxy-3,4-dihydro-1H-isoquinolin-2-yl}-2-phényl-acétamide ;
(R)-2-{(S)-1-[2-(4-bromo-phényl)-éthyl]-6,7-diméthoxy-3,4-dihydro-1H-isoquinolin-2-yl}-2-phényl-acétamide ;
(R)-2-{(S)-6,7-diméthoxy-1-[2-(4-trifluorométhyl-phényl)-éthyl]-3,4-dihydro-1H-isoquinolin-2-yl}-2-phényl-acétamide ;
(R)-2-{(S)-1-[2-(2,6-dichloro-phényl)-éthyl]-6,7-diméthoxy-3,4-dihydro-1H-isoquinolin-2-yl}-2-phényl-acétamide ; et
(R)-2-{(S)-1-[2-(4-*tert*-butyl-phényl)-éthyl]-6,7-diméthoxy-3,4-dihydro-1H-isoquinolin-2-yl}-2-phényl-acétamide.

8. Compositions pharmaceutiques indiquées dans le traitement d'affections associées à un rôle d'une orexine, qui comprennent un ou plusieurs composés de l'une quelconque des revendications 1 à 7 ou un sel pharmaceutiquement acceptable de ceux-ci et des matériaux véhicules et adjuvants conventionnels.

9. Compositions pharmaceutiques indiquées dans le traitement de troubles du comportement alimentaire et du sommeil, qui comprennent un ou plusieurs composés de l'une quelconque des revendications 1 à 7 ou un sel pharmaceutiquement acceptable de ceux-ci et des matériaux véhicules et adjuvants conventionnels.

10. Composés de l'une quelconque des revendications 1 à 7 ou un sel pharmaceutiquement acceptable de ceux-ci, conçu pour être utilisé comme médicament dans le traitement d'affections qui sont associées à un rôle des orexines.

11. Utilisation d'un dérivé à motif 1,2,3,4-tétrahydroisoquinoline selon l'une quelconque des revendications 1 à 7 ou d'un sel pharmaceutiquement acceptable de celui-ci dans la préparation d'un médicament indiqué dans la prévention ou le traitement d'affections sélectionnées dans le groupe consistant en les suivantes : dépression ; anxiété ; accoutumances ; trouble obsessionnel compulsif ; névrose affective ; névrose dépressive ; névrose d'angoisse ; trouble dysthymique ; dérèglement de l'humeur ; dysfonction sexuelle ; dysfonction psychosexuelle ; schizophrénie ; dépression maniaque ; délire ; démence ; arriération mentale sévère et dyskinésies telles que la chorée de Huntington et le syndrome de la Tourette ; diabète ; perturbations de l'appétit/du goût ; vomissements/nausées ; asthme ; maladie de Parkinson ; syndrome/maladie de Cushing ; adénome basophile ; prolactinome ; hyperprolactinémie ; hypopituitarisme ; tumeur/adénome hypophysaire ; maladies hypothalamiques ; affection intestinale inflammatoire ; dyskinésie gastrique ; ulcères gastriques ; syndrome de Froehlich ; maladies hypophysaires, hypogonadisme hypothalamique ; syndrome de Kallman (anosmie, hyposmie) ; aménorrhée fonctionnelle ou psychogène ; hypothyroïdie hypothalamique ; dysfonction hypothalamo-surrénalienne ; hyperprolactinémie idiopathique ; maladies hypothalamiques liées à un déficit en hormone de croissance ; retard statural idiopathique ; nanisme ; gigantisme ; acromégalie ; perturbations des rythmes biologiques et circadiens ; perturbations du sommeil associées à des maladies telles que des affections neurologiques, la douleur neurogène et le syndrome des jambes sans repos ; affections cardiaques et pulmonaires, insuffisance cardiaque aiguë et congestive ; hypotension ; hypertension ; rétention urinaire ; ostéoporose ; angor ; infarctus du myocarde ; accident vasculaire cérébral ischémique ou hémorragique ; hémorragie sous-arachnoïdienne ; ulcères ; allergies ; hypertrophie prostatique bénigne ; insuffisance rénale chronique ; affection rénale ; altération de la tolérance au glucose ; migraine ; douleur ; sensibilité accrue ou exagérée à la douleur, par exemple hyperalgésie, causalgie et allodynie ; douleur aiguë ; sensation de brûlure douloureuse ; douleur faciale atypique ; douleur neurogène ; douleur dorsale ; syndrome douloureux régional complexe de type I et II ; douleur arthritique ; douleur due à un traumatisme sportif ; douleur liée à une infection, au VIH ; douleur post-chimiothérapeutique, douleurs post-accident vasculaire cérébral ; douleur post-opératoire ; névralgie ; affections associées à une douleur viscérale comme le syndrome du côlon irritable, la migraine et l'angor ; incontinence urinaire, par exemple incontinence d'urgence mictionnelle ; tolérance aux narcotiques ou sevrage narcotique ; troubles du sommeil ; troubles du comportement alimentaire ; maladies cardio-vasculaires ; maladies neurodégénératives ; apnées du sommeil ; narcolepsie ; insomnie ; parasomnie ; et maladies neurodégénératives, y compris des entités nosologiques telles que le complexe désinhibition-démence-parkinsonisme-amyotrophie ; épilepsie associée à une dégénérescence pallido-ponto-nigrale, troubles convulsifs et autres maladies liées à des dysfonctionnements généraux du système des orexines.

12. Utilisation selon la revendication 11, où lesdites affections sont sélectionnées dans le groupe consistant en des troubles du comportement alimentaire et des troubles du sommeil.

13. Utilisation selon la revendication 12, où lesdits troubles du comportement alimentaire comprennent un dysfonctionnement métabolique, un dérèglement du contrôle de l'appétit, l'obésité compulsive, la boulimie avec vomissements ou l'anorexie mentale.

14. Utilisation selon la revendication 12, où lesdits troubles du sommeil comprennent l'insomnie, la narcolepsie et d'autres affections associées à une torpeur excessive, les dystonies paroxystiques nocturnes, le syndrome des jambes sans repos, les apnées du sommeil, le syndrome du décalage horaire, la fatigue et divers autres malaises attribués au travail en équipes et le syndrome de retard ou d'avance de phase du sommeil.

15. Utilisation de un ou plusieurs composés de l'une quelconque des revendications 1 à 7 dans la préparation d'un médicament indiqué, en combinaison avec d'autres composés pharmacologiquement actifs, y compris d'autres antagonistes des récepteurs aux orexines, des agents hypolipémiants, des agents anorexiants, des agents somnifères, des antidépresseurs ou d'autres médicaments, dans la prévention ou le traitement des affections répertoriées à l'une quelconque des revendications 8 à 14.
